(19) Europäisches Patentamt
European Patent Office
Office européen des brevets

(11) EP 1 934 375 B1

(12) EUROPEAN PATENT SPECIFICATION

(45) Date of publication and mention of the grant of the patent:
**15.04.2015 Bulletin 2015/16**

(21) Application number: **06806238.9**

(22) Date of filing: **12.10.2006**

(51) Int Cl.:
***C12Q 1/68*** *(2006.01)*

(86) International application number:
**PCT/EP2006/009884**

(87) International publication number:
**WO 2007/042306 (19.04.2007 Gazette 2007/16)**

(54) **MEANS AND METHODS FOR THE PREDICTION OF JOINT DESTRUCTION**

MITTEL UND VERFAHREN ZUR VORHERSAGE VON GELENKZERSTÖRUNG

MOYENS ET METHODES DE PREDICTION DE DESTRUCTION D'ARTICULATION

(84) Designated Contracting States:
**AT BE BG CH CY CZ DE DK EE ES FI FR GB GR HU IE IS IT LI LT LU LV MC NL PL PT RO SE SI SK TR**

(30) Priority: **13.10.2005 EP 05022344**

(43) Date of publication of application:
**25.06.2008 Bulletin 2008/26**

(60) Divisional application:
**11172855.6 / 2 380 994**

(73) Proprietors:
- **Schulze-Koops, Hendrik 91056 Erlangen (DE)**
- **Skapenko, Alla 91056 Erlangen (DE)**

(72) Inventors:
- **SCHULZE-KOOPS, Hendrik 91056 Erlangen (DE)**
- **SKAPENKO, Alla 91056 Erlangen (DE)**
- **LIPSKY, Peter E. Rockville, Maryland 20852 (US)**

(74) Representative: **Vossius & Partner Siebertstrasse 3 81675 München (DE)**

(56) References cited:

| | |
|---|---|
| **WO-A-94/14975** | **WO-A-2005/014861** |
| **WO-A2-00/34789** | **WO-A2-02/04009** |
| **WO-A2-89/09621** | **WO-A2-03/010335** |

- **PROTS IRYNA ET AL: "Association of the IL4R single-nucleotide polymorphism I50V with rapidly erosive rheumatoid arthritis" ARTHRITIS & RHEUMATISM, vol. 54, no. 5, May 2006 (2006-05), pages 1491-1500, XP002417320 ISSN: 0004-3591**
- **KOTAKE SHIGERU ET AL: "Association of IL-4 receptor polymorphism with radiologic progression in patients with early rheumatoid arthritis" ARTHRITIS AND RHEUMATISM, vol. 42, no. 9 SUPPL., September 1999 (1999-09), page S247, XP009077988 & 63RD ANNUAL SCIENTIFIC MEETING OF THE AMERICAN COLLEGE OF RHEUMATOLOGY AND THE 34TH ANNUAL SCIENTIFI; BOSTON, MASSACHUSETTS, USA; NOVEMBER 13-17, 1999 ISSN: 0004-3591**
- **BUCHS N ET AL: "IL-4 VNTR gene polymorphism in chronic polyarthritis. The rare allele is associated with protection against destruction" RHEUMATOLOGY (OXFORD), vol. 39, no. 10, October 2000 (2000-10), pages 1126-1131, XP002417322 ISSN: 1462-0324**
- **GORONZY JÖRG J ET AL: "Prognostic markers of radiographic progression in early rheumatoid arthritis." ARTHRITIS AND RHEUMATISM JAN 2004, vol. 50, no. 1, January 2004 (2004-01), pages 43-54, XP002417323 ISSN: 0004-3591**
- **DEICHMANN KLAUS ET AL: "Common polymorphisms in the coding part of the IL4-receptor gene" BIOCHEMICAL AND BIOPHYSICAL RESEARCH COMMUNICATIONS, vol. 231, no. 3, 1997, pages 696-697, XP002417324 ISSN: 0006-291X**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 934 375 B1

- MIREL D B ET AL: "Association of IL4R haplotypes with type 1 diabetes" DIABETES, NEW YORK, NY, US, vol. 51, no. 11, November 2002 (2002-11), pages 3336-3341, XP002243337 ISSN: 0012-1797
- KANEMITSU S ET AL: "Association of interleukin-4 receptor and interleukin-4 promoter gene polymorphisms with systemic lupus erythematosus" ARTHRITIS AND RHEUMATISM, LIPPINCOTT, PHILADELPHIA, US, vol. 42, no. 6, June 1999 (1999-06), pages 1298-99, XP001064873 ISSN: 0004-3591
- MITSUYASU H ET AL: "Ile50Val variant of IL4R alpha upregulates IgE synthesis and associates with atopic asthma." NATURE GENETICS JUN 1998, vol. 19, no. 2, June 1998 (1998-06), pages 119-120, XP002417325 ISSN: 1061-4036
- SKAPENKO ALLA ET AL: "Regulation of T cell differentiation by IL4R alpha-chain single nucleotide polymorphisms." ARTHRITIS & RHEUMATISM, vol. 52, no. 9, Suppl. S, September 2005 (2005-09), pages S492-S493, XP009077989 & 69TH ANNUAL SCIENTIFIC MEETING OF THE AMERICAN-COLLEGE-OF-RHEUMATOLOG Y/ 40TH ANNUAL SCIENTIFIC MEETIN; SAN DIEGO, CA, USA; NOVEMBER 12 -17, 2005 ISSN: 0004-3591
- SKAPENKO A ET AL: "Regulation of T cell differentiation by IL-4R alpha-chain single nucleotide polymorphisms" ANNALS OF THE RHEUMATIC DISEASES, vol. 64, no. Suppl. 3, July 2005 (2005-07), page 79, XP009077987 & ANNUAL EUROPEAN CONGRESS OF RHEUMATOLOGY; VIENNA, AUSTRIA; JUNE 08 11, 2005 ISSN: 0003-4967

## Description

The present invention relates to a method of diagnosing and/or predicting early joint destruction and/or accelerated joint destruction, in rheumatoid arthritis,comprising determining in a sample obtained from an individual independent of HLA-DR shared epitope (SE) the presence of at least one nucleic acid sequence encoding an IL-4 receptor (IL-4R) which contains a mutation in position 465 of the nucleotide sequence of the wild-type IL4R as shown in SEQ ID NO: 1, whereby at said position the nucleotide A is replaced or a nucleic acid sequence encoding an IL-4 receptor (IL-4R), said IL-4R comprising at position 75 as shown in SEQ ID NO: 2 a valine instead of an isoleucine. Furthermore, the invention provides for a method of diagnosing and/or predicting early joint destruction and/or accelerated joint destruction in rheumatoid arthritis comprising determining in a sample obtained from an individual the presence of an encoded

IL-4 receptor (IL-4R) which comprises at the homologous position 75 of IL-4 receptor as depicted in SEQ ID NO: 2 a mutation, said mutation comprising the exchange from an isoleucine to a valine. In addition, the present invention relates to a use of specific probes and/or primers for the preparation of a diagnostic composition for diagnosing and/or predicting early joint destruction and/or accelerated joint destruction.

Rheumatoid arthritis (RA) is one of the most common human systemic autoimmune diseases characterized by synovial infiltrates and a progressive cell-mediated destruction of the joints, which results in substantial disability of the patients (Firestein (2003) Nature, 423(6937):356-361). Evidence from twin (Aho (1986) J Rheumatol, 13(5):899-902; Silman (1993) Br J Rheumatol, 32(10):903-907) and family (Deighton (1991) Ann Rheum Dis, 50(1):62-65) studies suggests that both genetic and environmental factors contribute to susceptibility to RA, and disease heritability has been estimated to be about 60% (MacGregor (2000) Arthritis Rheum, 43(1):30-37) ). To date, only the *human leukocyte antigen* (*HLA*) locus on chromosome 6 has been consistently linked to and associated with RA susceptibility (Seldin (1999) Arthritis Rheum, 42(6):1071-1079). This association, however, accounts for only one-third of the genetic susceptibility (Deighton (1989) Clin Genet, 36(3):178-182). In an attempt to identify the non-*HLA* loci involved in disease susceptibility, genome-wide scans have been performed by different groups in studies involving RA multiplex families with affected siblings pairs (Comelis (1998) Proc Natl Acad Sci U S A, 95(18):10746-10750; Jawaheer (2001) Am J Hum Genet, 68(4):927-936; MacKay (2002) Arthritis Rheum, 46(3):632-639; Shiozawa (1998) Int Immunol, 10(12):1891-1895). Although no common loci apart from the *HLA* region were identified by all the studies, several non-*HLA* loci that demonstrate modest linkage with RA were suggested by multiple studies. One such locus is the chromosome 16p12, for which a modest association of 16p12 markers (*D16S3103*, *D16S403*, *D16S420*, and *D16S401*) with RA was observed in three out of the four studies.

Previously, it has been described that in patients with RA-related DRB1 shared epitope (i.e. SE positive patients) the Larsen-score was significantly higher in patients that are homozygous (V50/V50) in respect of the IL-4R polymorphism; .Kotake et al., Arthritis and Rheumatism; 42(9 SuppL) page S247, 1999).

The region located only about 2.5 Mb centromeric from *D16S401* at 16p11.2-12.1 contains genes coding for specific receptor subunits of T helper type 2 (Th2) cytokines, interleukin 4 (IL4) and IL21. IL4 and IL4R play an important role in the pathogenesis of RA, as diminished production of IL4 was previously believed to contribute to the characteristic Th1-mediated autoimmune rheumatoid inflammation (Skapenko (1999) J Immunol, 163:491-499; Simon (1994) Proc Natl Acad Sci USA, 91:8562-8566). Within the coding region of the *IL4R* gene seven single nucleotide polymorphisms (SNPs) that result in amino acid substitutions have been identified (Deichmann (1997) Biochem Biophys Res Commun, 231(3):696-697; Kruse (1999) Immunology, 96(3):365-371; Hershey (1997) N Engl J Med 1997;337(24):1720-1725). Two of them, the I50V (in the following also designated as "I75V", in particular in relation to a polypeptide which also comprises a signal sequence) and the Q551R reside within sequences coding for functionally important regions of the IL4R molecule and were reported to be associated with functional alterations in cellular assays (Mitsuyasu (1998) Nat Genet, 19(2):119-120; Mitsuyasu (1999) J Immunol, 162(3):1227-1231; Hershey (1997) N Engl J Med, 337(24):1720-1725; Risma (2002) J Immunol, 169(3):1604-1610; Stephenson (2004) J Immunol, 173(7):4523-4528; Wang (1999) J Immunol, 162(8):4385-4389; Kruse (1999) Immunology, 96(3):365-371). In addition, the association of the *IL4R* gene with susceptibility to immune disorders mediated by an imbalance in the Th1/Th2 ratio has already been demonstrated, for example in asthma and atopy (Mitsuyasu (1998), loc. cit.; Kruse (1999) loc. cit.; Hershey (1997), loc. cit.), type 1 diabetes (Mirel (2002) Diabetes, 51(11):3336-3341; Bugawan (2003) Am J Hum Genet, 72(6):1505-1514) or lupus (Kanemitsu (1999) Arthritis Rheum, 42(6):1298-1300). However, even if *IL4R* was considered as a potential candidate for RA susceptibility, a concrete link to RA could not be provided and published data were contradicting.

The RA-characteristic joint destruction occurs in the majority of patients within the first two years of the disease (Lee (2001) Lancet, 358(9285):903-911). The demonstration that early suppression of inflammation may impede the progression of joint destruction even years after treatment (O'Dell (2002) Arthritis Rheum, 46(2):283-285) highlights the importance of identifying at-risk patients as early as possible. However, attempts to identify prognostic factors for a destructive *vs*. a non-destructive outcome yielded disappointing results. The presence of rheumatoid factor (RF) and, more recently, of antibodies to cyclic citrullinated peptides has been associated with more severe disease (De Rycke (2004) Ann Rheum Dis, 63(12):1587-1593), but, unfortunately, these factors are not reliable enough to allow therapeutic decision-making. The only genetic locus with a predictive value for the development of more severe disease identified to date is, also here, the *HLA* locus (Thomson (1999) Arthritis Rheum, 42(4):757-762; Gorman (2004) Arthritis Rheum 50(2):400-412). It was speculated that *IL4R* might be an alternative potential candidate for the prediction of disease progression, because the extent of the Thl dominance during early disease, which is controlled at least in part by inhibitory signaling through IL4R, correlates with disease progression (Kanik (1998) J Rheumatol, 25(1):16-22; van der Graaff (1998) Lancet 1998;351(9120):1931).

Thus, the technical problem underling the present invention was to provide means and methods for an early determination of the potentially erosive and/or fast erosive character of bone and/or joint destruction, for example in rheumatoid arthritis. The solution to said technical problem is achieved by providing the embodiments as characterized in the claims and in the description.

In a first aspect, the present invention provides for a method of diagnosing and/or predicting early joint destruction and/or accelerated joint destruction in rheumatoid arthritis, said method comprising determining in a sample obtained from an individual independent of HLA-DR shared epitope (SE) the presence of at least one nucleic acid sequence selected from the group consisting of

(a) a nucleic acid sequence encoding an IL-4 receptor (IL-4R) which contains a mutation in position 465 of the nucleotide sequence of the wild-type IL4R as shown in SEQ ID NO: 1, whereby at said position the nucleotide "A" (adenosine) is replaced by the nucleotide "G" (guanine);
(b) a nucleic acid sequence encoding an IL-4 receptor (IL-4R), said IL-4R comprising at position 75 as shown in SEQ ID NO: 2 a valine;
(c) a nucleic acid sequence encoding an IL-4 receptor (IL-4R), said IL-4R comprising at position 75 as shown in SEQ ID NO: 2 a valine instead of an isoleucine;
(d) a nucleic acid sequence comprising at least 15 nucleotides of the nucleic acid sequence of any one of (a) to (c) and comprising the mutation as defined in any one of (a) to (c);
(e) a nucleic aid sequence comprising a nucleotide sequence as shown in any one of SEQ ID NOS.3 or a part thereof, said part comprising the nucleotide sequence "gtc" (or "guc" on the RNA level) as depicted in nucleotides numbers 5758 to 5760 of SEQ ID NO: 3;
(f) a nucleic acid sequence encoding a polypeptide comprising the amino acid sequence as shown in SEQ ID NO(s): 4; and
(g) a nucleic acid sequence which hybridizes to a nucleotide sequence defined in any one of (a) to (f) and having a mutation as defined in any one of (a) to (c).

The present invention relates, accordingly to an in vitro method for determining the susceptibility, predisposition, presence and/or potential risk of developing an erosive joint destruction, in particular early after the onset of the disease (i.e. rheumatoid arthritis) in a subject/patient independent of HLA-DR shared epitope (SE), said method comprising the steps of:

biological sample obtained from said subject/patient;
assaying for the IL4R allel as defined herein, namely the herein defined IL4R single nucleotide polymorphism "I50V"/"'I75V"; and
comparing said allelic situation with the situation in a normal control ("I50I/I75I") or a control showing the herein defined mutant single nucleotide polymorphism leading to an exchange of isoleucine ("I") to valine ("V") on position 50 (or, when a signal peptide of 25 amino acids is concerned, position 75) of the expressed IL4R.

The herein defined point mutation/SNP or allelic situation of IL4R can be.assessed on the level of the nucleic acid molecules (e.g. DNA or RNA) as well as on the protein level (i.e. expressed IL4R protein)

As detailed herein below and as also summarized in the experimental part of this invention the present invention provides for a method of predicting/diagnosing at an early point of time a modification in the joints and to predict in particular early joint destruction and/or accelerated joint destruction in rheumatoid arthritis. This "early point of time" relates to a diagnosis and or prognosis even before signs of a joint and/or bone disorder, in particular joint or bone destruction and/or ersosion, can be detected clinically, radiographically, by MRI or by ultrasound. The gist of the present invention lays in the fact that a reliable marker is provided which allows the diagnosis of the further path or prognosis of joint destruction in particular in patients suffering (or being prone to suffer from) rheumatoid arthritis. The means and methods provided herein provide for the first time a reliable diagnostic marker with which it is possible to deduce whether a given individual/human patient is prone to erosive or non-erosive development and/or course of joint destruction, in rheumatoid arthritis. The diagnostic marker provided herein is indicative for in particular, but not-limited to, "early (joint) destruction". In context of this invention the term "early destruction" or "early joint destruction" does not only relate to the specific destruction of the joints but also to corresponding bone modifications.

Rheumatoid arthritis is diagnosed according to the 1987 revised criteria for the classification of rheumatoid arthritis (1. morning stiffness in an around joints lasting for at least one hour before maximal improvement present for at least six weeks; 2. arthritis of three or more joint areas observed by a physician present for at least six weeks; 3. arthritis of the proximal interphalangeal (PIP), metacarpophalangeal (MCP), or wrist joints present for at least six weeks; 4. symmetric arthritis present for at least six weeks; 5. subcutaneous nodules; 6. positive test for rheumatoid factor; 7. radiographic erosions or periarticular osteopenia in hand or wrist joints) if at least four of the following seven criteria are present: 1. morning stiffness in an around joints lasting for at least one hour before maximal improvement present for at least six weeks; 2. arthritis of three or more joint areas observed by a physician present for at least six weeks; 3. arthritis of the proximal interphalangeal (PIP), metacarpophalangeal (MCP), or wrist joints present for at least six weeks; 4. symmetric arthritis present for at least six weeks; 5. subcutaneous nodules; 6. positive test for rheumatoid factor; and 7. radiographic erosions or periarticular osteopenia in hand or wrist joints; see also Arnett (1988) Arthritis Rheum 31:315-324. Therefore, the person skilled in the art has means to diagnose the onset of RA by the above recited criteria.

An "early destruction" can be defined in context of this invention showing signs of joint and/or bone erosion before 5 years after first conventional diagnosis of RA, before 4 years after first conventional diagnosis of RA, before 3 years after first conventional diagnosis of RA and in particular before 2 years after first conventional diagnosis of RA. In this context, the "first conventional diagnosis of RA" relates to the determiantion of the onset of the disease. As documented in the appended examples, bone erosions occurred in more than 65% of the (mutant) V50 allele homozygous patients compared to about 37.0% of the patients homozygous for the (non-mutated) 150 allele at two years after disease onset (OR 3.86, p<0.0001). This association was independent of individual factors previously associated with severe disease, such as rheumatoid factor or the HLA-DR shared epitope. Also Machold (2006), Rheumatology (Advance Access published August 9, 2006) teaches that erosive disease ("early erosion and/or early destruction", developed in 63,6% of patients over 3 years with the majority (74.3%) appearing in the first and 97.2% by the end of the second year. Accordingly, the person skilled in the art consideres "early destruction" as appear in less than 5, less than 4, less than 3 and in particular less than 2 years after the onset of rheumatoid arthritis. As shown in the appended examples, the herein identified mutant of IL4R has an impact on the rapidity of disease progression and the investigation was carried out on RA patients who have either erosive or non-erosive disease based on radiographic analysis on the hands and feet two years after disease onset. Therefore, the point mutation disclosed herein is a genetic marker with high predictive value for early erosive RA.

The methods, means and uses of this invention allow also the early determination of the potential risk of arthritis-related erosions which comprise, for example joint space narrowing and general erosion of articular bones or joint structures, normally within the first two years of disease. Accordingly, the methods and means provided herein allow for the early detection of rapid/rapidly erosive rheumatoid arthritis versus less or even non-erosive rheumatoid arthritis. However, it is to be understood that the means and methods provided herein in addition allow for the detection of the future fate, course or progress of erosive events in a patient suffering from rheumatoid arthritis, as will be detailed herein below. These evidences and erosions may, inter alia, be assessed by standardized radiographs, typically of hand and feet, ultrasound and/or by magnetic resonance imaging. In accordance with the teachings of the present invention, the attending physician may, at any point of time, choose a different treatment option, depending whether the patient, preferably the human patient to be treated, shows early signs of joint or bone destruction and is either homozygous for the here described "I50V"/"I75V" IL4R SNP (both alleles comprise the point mutation "V" on position 50/75 as defined herein), is heterozygous (only one allele comprises the "I50V"/ "I75V" SNP) or comprises two "wild-type" alleles of IL4R in relation to the "I50"/"I75" position, i.e. is (wild-type) "I50I"/"I75I". It is understood by the person skilled in the art that the present invention provides for a specific "point mutation" in the IL4R gene which is diagnostic for (erosive) joint destruction in a human patient. Accordingly, a human individual assessed for said point mutation is either "homozygote" for the mutant allele "V" (both genes comprise "V" on position 50/75 of the amino acid sequence of IL4R as defined herein), homozygote for the allele "I" (both genes comprise "I" on position 50/75 of the amino acid sequence of IL4R as defined herein) or "heterozygote" (i.e. one gene comprises "V" on position 50/75 of the amino acid sequence of IL4R, whereas the other gene comprises "I" on position 50/75 of the amino acid sequence of IL4R as defined herein).

In context of this invention it was surprisingly found that the predictive value of the herein detailed and explained "I50V" ("I75V") SNP or point mutation of IL-4R (CD 124) for erosive disease was not only much higher than that of rheumatoid factor (RF) or the shared epitope (SE) but that this specific point mutation in the allele has also (in contrast to the evaluation in the prior art) a very informative character for predicting the progressive erosive disease, in particular in patients who suffer from rheumatoid arthritis (RA). The present invention also provides for the surprising finding that no association between I50V (I75V) and Q551R IL-4R SNPs and disease (RA) susceptibility was identified. No differences in the genotype and allele frequencies of the I50V or Q55IR SNPs were determined between RA patients (471 individuals) and control individuals (371 healthy control persons). Nevertheless and surprisingly, the I50V (I75V) SNP is strongly associated with the mostly fast development of joint destruction, i.e. there is a surprising difference between the erosive and the non-erosive group of patients. Therefore, the I50V/I75V SNP has, in contrast to the previously described Q551R SNP of IL-4R, a highly predictive value in particular for the early onset or potential early onset of erosive disease as well as for the erosive character of the disease. Furthermore, as documented in the appended examples it was found that the combination of the valine 50 (V50) allele with either other predictive markers resulted in an increase of the predictive power whereas the combination of for example rheumatoid factor (RF) and shared epitope (SE) alleles did never reach corresponding values. Accordingly, the predictive value of the RF as well as the SE alleles in combination is much lower than the predictive value of for example the (mutant)V50 homozygocity in IL-4 receptor. Therefore, with the teachings of the present invention, at risk rheumatoid arthritis patients can be identified at a very early phase of the disease and corresponding early medical intervention/therapy can be applied. It is immediately evident for the skilled artisan that the means and methods provided herein are not limited to the predictive value of the detection of the herein identified "V" point mutation ("V50" or "V75"). In contrast, also the determination of the wild-type allel (I on position 50 or 75 of the herein defined IL4R amino acid sequence or corresponding natural occurring isoforms and variants) is indicative for the erosive or potentially erosive character of the disease. Accordingly, when a human patient is characterized as comprising the wild-type alleles ("I50" or "I75") in the herein defined position of IL4R, a less aggressive or moderate therapy/medical intervention is indicated since in this patient the joint destruction is less erosive or even non-erosive.

As mentioned above and illustrated herein, an association analysis of *IL4R* with RA susceptibility was performed in a well-characterized case-control cohort of 471 RA patients and 371 healthy controls. Two single-nucleotide polymorphisms (SNPs) (I50V and Q551R) located in the coding region of *IL4R* (CD124) were examined by allele-specific polymerase chain reaction using FAM-labeled allele-detecting probes. Patients, from whom radiographs of feet and/or hands were avilable at two years of disease duration (DD), were further stratified according to the radiological outcome into an erosive and a non-erosive group to evaluate the association of the *IL4R* SNPs with disease progression. In an *in vitro* cell culture system the response of CD4 T cells from healthy individuals with known *IL4R* genotypes to IL4 was assessed with regard to the inhibitory effect of IL4 on Th1 cell differentiation to provide a possible mechanism underlying the association.

In accordance with this invention no differences in the genotype and allele frequencies of the investigated SNPs were determined between RA patients and healthy controls. In contrast, significant differences between the two groups of patients stratified according to the presence of radiographic joint destruction at two years DD were observed in the distribution of 150V (processed or mature protein) *IL4R* SNP genotypes ($\chi^2$ 15.68, p = 0.0004). 68.1% of the V50 compared to only 37.0% of the I50 homozygous patients showed radiographic erosion at two years DD (OR 3.86, 95% CI 1.95-7.64, p < 0.0001). This association was independent of individual factors previously associated with severe disease, such as rheumatoid factor (RF) or shared epitope (SE). The predictive value of the V50 homozygosity for the development of joint erosion had a specificity of 0.85, and in combination with either other investigated predictive marker, SE or RF, the specificity further increased. V50 homozygosity had a higher predictive power for early erosive disease than RF and SE. Moreover, homozygosity for V50 SNP lead to a diminished inhibition by IL4 of Th1 cell differentiation and of IL12R$\beta$2 expression on CD4 T cells. Nevertheless, it can also be demonstrated that in a heterozygous V50 situation the V50 SNP/V50 allele as described herein has a high predictive value.

This present finding is surprising since it was recently stipulated that in particular the herein defined isoleucine to valine mutation at position 50 of the mature, wild-type IL-4 receptor protein (corresponding to position 75 in the herein shown protein sequence derived from the wild type cDNA nucleotide sequence and comprising a "signal peptide" of 25 amino acids; see SEQ ID NO: 2) has no predictive value for rheumatoid arthritis, in particular early rheumatoid arthritis (RA). In particular, Goronzy (2004) Arthritis & Rheumatism 50, 43 stressed that only a single SNP in the uteroglobin gene has predictive value and concluded that further SNPs, also comprising the herein defined "I50V" (or "I75V") mutation or variant are not informative.

In contrast to Goronzy (2004, loc. cit.), the data provided herein clearly identify the I50V SNP of IL4R (CD 124) as a reliable genetic marker for early joint destruction, in RA. The herein defined point mutation or "SNP" has a high predictive value for the erosive character of the disease and, i.e., for early joint destruction. This is of particular significance in the clinic and in a medical setting to identify patients with accelerated joint destruction early before the development of rheumatoid damage.

The term "IL4R" as used herein relates to the interleukin-4 receptor and may be abbreviated herein as "IL-4R" and "IL4R", as well as "IL-4 receptor". The term is well known in the art and in the present invention it is of note that the position of nucleotides and/or amino acid residue position relate to the known $\alpha$-chain of the IL-4R/IL4R. The IL4R is also known in the art as "cluster of differentiation 124" or "CD 124" and, accordingly, also in this invention the term "CD 124" is employed as synonym for IL4R, in particular for the IL4R $\alpha$-chain.

The term "mutation in position 465 of the cDNA sequence encoding IL4R/IL-4R/CD124" relates to position 465 of the SEQ ID NO: 1 as provided herein below.

1 ccccgcgcgg cgcgggccag ggaagggcca cccaggggtc ccccacttcc cgcttgggcg
61 cccggacggc gaatggagca ggggcgcgca gataattaaa gatttacaca cagctggaag
121 aaatcataga gaagccgggc gtggtggctc atgcctataa tcccagcact tttggaggct
181 gaggcgggca gatcacttga gatcaggagt tcgagaccag cctggtgcct tggcatctcc
241 caatggggtg gctttgctct gggctcctgt tccctgtgag ctgcctggtc ctgctgcagg
301 tggcaagctc tgggaacatg aaggtcttgc aggagcccac ctgcgtctcc gactacatga
361 gcatctctac ttgcgagtgg aagatgaatg gtcccaccaa ttgcagcacc gagctccgcc
421 tgttgtacca gctggttttt ctgctctccg aagcccacac gtgtatccct gagaacaacg
481 gaggcgcggg gtgcgtgtgc cacctgctca tggatgacgt ggtcagtgcg gataactata
541 cactggacct gtgggctggg cagcagctgc tgtggaaggg ctccttcaag cccagcgagc
601 atgtgaaacc cagggcccca ggaaacctga cagttcacac caatgtctcc gacactctgc
661 tgctgacctg gagcaacccg tatcccctg acaattacct gtataatcat ctcacctatg
721 cagtcaacat ttggagtgaa aacgacccgg cagatttcag aatctataac gtgacctacc
781 tagaaccctc cctccgcatc gcagccagca ccctgaagtc tgggatttcc tacagggcac
841 gggtgagggc ctgggctcag tgctataaca ccacctggag tgagtggagc cccagcacca
901 agtggcacaa ctcctacagg gagcccttcg agcagcacct cctgctgggc gtcagcgttt
961 cctgcattgt catcctggcc gtctgcctgt tgtgctatgt cagcatcacc aagattaaga
1021 aagaatggtg ggatcagatt cccaacccag cccgcagccg cctcgtggct ataataatcc
1081 aggatgctca ggggtcacag tgggagaagc ggtcccgagg ccaggaacca gccaagtgcc
1141 cacactggaa gaattgtctt accaagctct tgccctgttt tctggagcac aacatgaaaa
1201 gggatgaaga tcctcacaag gctgccaaag agatgccttt ccagggctct ggaaaatcag
1261 catggtgccc agtggagatc agcaagacag tcctctggcc agagagcatc agcgtggtgc
1321 gatgtgtgga gttgtttgag gccccggtgg agtgtgagga ggaggaggag gtagaggaag
1381 aaaaagggag cttctgtgca tcgcctgaga gcagcaggga tgacttccag gagggaaggg
1441 agggcattgt ggcccggcta acagagagcc tgttcctgga cctgctcgga gaggagaatg
1501 ggggcttttg ccagcaggac atgggggagt catgccttct tccaccttcg ggaagtacga
1561 gtgctcacat gccctgggat gagttcccaa gtgcagggcc caaggaggca cctccctggg
1621 gcaaggagca gcctctccac ctggagccaa gtcctcctgc cagcccgacc cagagtccag
1681 acaacctgac ttgcacagag acgcccctcg tcatcgcagg caaccctgct taccgcagct
1741 tcagcaactc cctgagccag tcaccgtgtc ccagagagct gggtccagac ccactgctgg
1801 ccagacacct ggaggaagta gaacccgaga tgccctgtgt cccccagctc tctgagccaa
1861 ccactgtgcc ccaacctgag ccagaaacct gggagcagat cctccgccga aatgtcctcc
1921 agcatggggc agctgcagcc cccgtctcgg cccccaccag tggctatcag gagtttgtac
1981 atgcggtgga gcagggtggc acccaggcca gtgcggtggt gggcttgggt cccccaggag
2041 aggctggtta caaggccttc tcaagcctgc ttgccagcag tgctgtgtcc ccagagaaat
2101 gtgggtttgg ggctagcagt ggggaagagg ggtataagcc tttccaagac ctcattcctg

```
2161 gctgccctgg ggaccctgcc ccagtccctg tccccttgtt cacctttgga ctggacaggg
2221 agccacctcg cagtccgcag agctcacatc tcccaagcag ctccccagag cacctgggtc
2281 tggagccggg ggaaaaggta gaggacatgc caaagccccc acttccccag gagcaggcca
2341 cagaccccct tgtggacagc ctgggcagtg gcattgtcta ctcagccctt acctgccacc
2401 tgtgcggcca cctgaaacag tgtcatggcc aggaggatgg tggccagacc cctgtcatgg
2461 ccagtccttg ctgtggctgc tgctgtggag acaggtcctc gccccctaca accccccctga
2521 gggccccaga cccctctcca ggtggggttc cactggaggc cagtctgtgt ccggcctccc
2581 tggcaccctc gggcatctca gagaagagta aatcctcatc atccttccat cctgcccctg
2641 gcaatgctca gagctcaagc cagaccccca aaatcgtgaa ctttgtctcc gtgggaccca
2701 catacatgag ggtctcttag gtgcatgtcc tcttgttgct gagtctgcag atgaggacta
2761 gggcttatcc atgcctggga aatgccacct cctggaaggc agccaggctg gcagatttcc
2821 aaaagacttg aagaaccatg gtatgaaggt gattggcccc actgacgttg gcctaacact
2881 gggctgcaga gactggaccc cgcccagcat tggctgggc tcgccacatc ccatgagagt
2941 agagggcact gggtcgccgt gccccacggc aggcccctgc aggaaaactg aggcccttgg
3001 gcacctcgac ttgtgaacga gttgttggct gctccctcca cagcttctgc agcagactgt
3061 ccctgttgta actgcccaag gcatgttttg cccaccagat catggcccac gtggaggccc
3121 acctgcctct gtctcactga actagaagcc gagcctagaa actaacacag ccatcaaggg
3181 aatgacttgg gcggccttgg gaaatcgatg agaaattgaa cttcagggag ggtggtcatt
3241 gcctagaggt gctcattcat ttaacagagc ttccttaggt tgatgctgga ggcagaatcc
3301 cggctgtcaa ggggtgttca gttaagggga gcaacagagg acatgaaaaa ttgctatgac
3361 taaagcaggg acaatttgct gccaaacacc catgcccagc tgtatggctg gggctcctc
3421 gtatgcatgg aacccccaga ataaatatgc tcagccaccc tgtgggccgg gcaatccaga
3481 cagcaggcat aaggcaccag ttaccctgca tgttggccca gacctcaggt gctagggaag
3541 gcgggaacct tgggttgagt aatgctcgtc tgtgtgtttt agtttcatca cctgttatct
3601 gtgtttgctg aggagagtgg aacagaaggg gtggagtttt gtataaataa agtttctttg
3661 tctctttaaa aaaaaaa (SEQ ID NO: 1)
```

This sequence for the "transcript variant 1" is also accessible under NM_000418 (gi: 56788409) provided by the NCBI database. SEQ ID NO: 1 corresponds to the wild-type cDNA sequence encoding IL-4R, i.e. the IL-4R $\alpha$-chain. The variant/SNP described herein and to be employed in the diagnostic (ex vivo or in vitro) methods and the corresponding inventive uses and means relates to the exchange of isoleucine (encoded in the wild-type IL4R by the codon/triplett "auc"/"atc") to valine (encoded by het codon/triplett "guc"/"gtc"). However, as laid down herein, also the determination of homozygous or heterozygous wild-type sequence is of diagnostic value, in particular for the attending physician. The diagnostic value of the wild-type IL4R in the present case is the fact that a patient who suffers from rheumatoid arthritis and has, however, a wild-type IL4R (on the position 150/175 as described herein or in the corresponding encoding nucleotide sequence) requires a different medical treatment regime than a patient who has the herein described point mutation. A patient suffering from rheumatoid arthritis (RA) but being homozygous for the wild-type allele 150/175 can be treated less aggressively than a patient who has either homozygously or even heterozygously the mutant allele (V50/V75) described herein. Cases with at least one mutant (150 $\rightarrow$V/I75 $\rightarrow$V) allele merit aggressive treatment in order to prevent joint destruction or accelerated joint destruction, whereas (conversely) cases with the wild-type allele, in particular with homozygous wild-type alleles require and allow for a more conservative, less aggressive medical intervention/medical management. Accordingly, it is also part of this invention that in the herein described means and methods the wild-type situation in the herein described "SNP" (I50 I/I75 I) is evaluated. The wild-type ILR4 referred to herein encodes for a polypeptide as shown in the following SEQ ID NO: 2

MGWLCSGLLFPVSCLVLLQVASSGNMKVLQEPTCVSDYMSISTCEWKMNGPTNCSTELRLLY
QLVFLLSEAHTCIPENNGGAGCVCHLLMDDVVSADNYTLDLWAGQQLLWKGSFKPSEHVKPR
APGNLTVHTNVSDTLLLTWSNPYPPDNYLYNHLTYAVNIWSENDPADFRIYNVTYLEPSLRI
AASTLKSGISYRARVRAWAQCYNTTWSEWSPSTKWHNSYREPFEQHLLLGVSVSCIVILAVC
LLCYVSITKIKKEWWDQIPNPARSRLVAIIIQDAQGSQWEKRSRGQEPAKCPHWKNCLTKLL
PCFLEHNMKRDEDPHKAAKEMPFQGSGKSAWCPVEISKTVLWPESISVVRCVELFEAPVECE
EEEVEEEKGSFCASPESSRDDFQEGREGIVARLTESLFLDLLGEENGGFCQQDMGESCLLP
PSGSTSAHMPWDEFPSAGPKEAPPWGKEQPLHLEPSPPASPTQSPDNLTCTETPLVIAGNPA
YRSFSNSLSQSPCPRELGPDPLLARHLEEVEPEMPCVPQLSEPTTVPQPEPETWEQILRRNV
LQHGAAAAPVSAPTSGYQEFVHAVEQGGTQASAVVGLGPPGEAGYKAFSSLLASSAVSPEKC
GFGASSGEEGYKPFQDLIPGCPGDPAPVPVPLFTFGLDREPPRSPQSSHLPSSSPEHLGLEP
GEKVEDMPKPPLPQEQATDPLVDSLGSGIVYSALTCHLCGHLKQCHGQEDGGQTPVMASPCC
GCCCGDRSSPPTTPLRAPDPSPGGVPLEASLCPASLAPSGISEKSKSSSSFHPAPGNAQSSS
QTPKIVNFVSVGPTYMRVS (SEQ ID NO: 2)

This sequence depicted herein above comprises in position 75 an "isoleucine" (I); wild-type sequence with 25 amino acid signal sequence. Said "I" is in the mutant SNP described herein replaced by a "valine" (V). Said point mutation is employed in the inventive methods, means and uses described herein and is a diagnostic marker for joint destruction, i.e. the prognosis or the progression of joint destruction. In particular, in case when such a point mutation is present in a human individual/human patient, it is indicated that this patient is treated in a more aggressive fashion in order to slow down and/or ameliorate joint destruction(s), in particular early joint destruction(s). The present invention is of high importance since an early intervention with medicaments improves the outcome in joint destruction/joint erosions in particular in rheumatoid arthritis. However, in accordance with the present invention, the attending physician can deduce whether an aggressive or less aggressive/moderate medical intervention is indicated in a given patient. The present invention is particularly useful in the deduction of patients who will suffer from a more prominent and/or early joint destruction/joint erosion. If a patient has the herein described point mutation, the likelihood to suffer from early joint destruction is much higher and said patient requires more aggressive and early treatment.

The sequence provided herein above as SEQ ID NO: 2 comprises the encoded IL4R α-chain and an additional original signalpeptide of 25 amino acids. Accordingly, the SNP or variant described herein relates to an amino acid exchange from isoleucine to valine, which can be depicted and illustrated as "I75V" (relating to the polypeptide comprising the additional 25 amino acid signal sequence) and as "I50V" (relating to the encoded polypeptide without signal sequence).

The person skilled in the art also finds IL4R coding regions (located on human chromosome 16) under the gene accession number X52425 (gi: 33833) or under AF421855 (gi:15987825)

In the following SEQ ID NO: 3, the complete genomic DNA of the human IL4R gene is shown and in positions 5758 to 5760 the variant nucleotide sequence "gtc"/"guc" is shown which leads to the encoded I50V/I75V variant.

```
   1 tacaggtgtg agctactgtg tctggcctga ataataaaat ttaaaacaat ttttcaaaaa
  61 ttcaccatga ggtctcacta tattccctag gctggtctca aacccctgga ctccaagtga
 121 tccaccccac cttcccgagt agctgggact agagatgcac accattgcac ccaatagagc
 181 aatacgtttc tgttctttgt aaattacctg ctctaaggta tttttgttat agcagcctgt
 241 atggactaag ctgacttgta acgttacttg agactttaaa gtgttccggt cactgttgga
 301 gggctctgtc tgtgttagct catttaatcc ccacaacacc tcaatcagat ggggctattc
 361 ttagtcccac tttatagata aggaaactga ggcatggaag cacagcttgc tcaaggttca
 421 catctagtca gtgacagagc aggtatttaa acctcaggaa ataatcagag aaacatgtgt
 481 agagggttgt ccaaggaagg ccacatccag aagcatctcc caggacagtt gttgtgtagc
 541 tcaccctctg gactttgtgg gtctgggtgt tgtttcatga ttatagagag agctctgtga
 601 acgtggagga cctgttgtcg gcagagacac aaatggccag ggcatggctg ggcagccgca
 661 gtggctcagg cctgtaatcc cagcacttcg agaagaccag aggggcagat catgaggtca
 721 gaagttcaag accagcctgg ccaacatggt gaaaccccgt ctctactaaa aatacaaaaa
 781 ttagccaggt gtggtggtgg gcacctgtaa tcccagctac tcgggaggct gaggcagaag
 841 aatcgcttga acccgggagg tggaggttgc agtgagctga gattgcaccg ctgcactcca
 901 gccttggaga cagagcgaga ctctgtctcg gaaaaacaaa caaacaagca aacaaacaaa
 961 caaataaatg gccagggcag gggagggttg catattgaat aagatgagct ctgctggaag
1021 cacaggtcag cactaacctg cttcctctct ctctgcaggt gccttggcat ctcccaatgg
1081 ggtggctttg ctctgggctc ctgttccctg tgagctgcct ggtcctgctg caggtggcaa
1141 gctctggtaa gtcaccactt ctcaatcatt catttgttgg ctattaatgg cgtgccaggg
1201 tcctgcagta tgtcacctgg ccttatggag attacactgc agtgggaggg gacagccaat
1261 gacaagtggc cctgattatc agtaaattct aaagattgtt agaaagtgat gggagccggg
1321 tgcagtggct cacacctgta atcccagcac ttcaggaggc cgaggcagga ggatcgcttg
1381 agcccaggag ttcgaggtca gcttgggcaa catagggaga ccttgtctct acaaataata
1441 aaatattagc caggtgtggc agtgcacgcc tgtagcccca gctactcagg aggccgaggt
1501 gggaggatcc cttgaactca ggaggtcaag gctgcagtga actgtgatcg cgccactcca
1561 ctccagcctg cgtgagaaag tgagaccctg tcaaaaaaaa agagaaggtg atggggaaag
1621 aacacagaac agcataagag gggttggggg aagctgggtg gagtgggggg gattgcagtt
1681 gaaagtaggg aagtcaggga aggcctcatt gagctgactt ggaggaagcg ggaaccgtgc
1741 agatgtctgg ggaaggctca ttcttggcag agaggccctg cactgagcct ggcgggaggg
1801 ttgagcacag gagggaatgt ggtggaggag agtgagcagc aggagggagc agtgaaggtc
1861 agcaaggtga cagagtggct gaatcaaaaa agaccttgca gtgtttgagc agaggatcca
1921 tatcatccat tatgttccaa aggactcttc aggatgccgt gtggagaaag gaagagggtg
1981 gaagccagga ggtctggagg gaggtctgga gtggaggaga tgagaggctc cggatccctc
```

```
2041 tgggaggtag atttgaggac agattggaat tgaggtgaaa gacagagaaa gagaagtggc
2101 caggatgact ccaagatttc tgacctaaac tactgggaag gacgcggttg tcatttctga
2161 aatgcagaag gatgccagaa gagaaggtac tttggggagg ggcgggaatc aggagttagt
2221 tttggacatg agataagctt ggaatattta tttgctatct aagacagctc cttaacatgg
2281 taagccctta tgcaagttgt tgtcagctga gatgggcgtg gcactgagca tgggagcatg
2341 gaggcgcctg agtggtctca tgctcaggtg gtttagcaaa ctcagtgtac atcctgccaa
2401 ttccagtcct gccatggcca ctgacaagct aggagggcgc tgaaaggaga aggaccccga
2461 tgtctcctcc agcccatcca tctcctctct cccattggcc aaacccaacc ggaaactaaa
2521 ggccaagggt acccggtgat gaagactgtg gtatcagcct cctgagcaca gagagggcag
2581 aaaggggtgg agacaaagag gggcgcagat agtgggcaaa tggggaagtg gcacttcccc
2641 tagctcgagg gcagaggctt ggtgtgatgg aatggcactc cttaaactgc tacatatttt
2701 ccctttaatt tggccaagaa caagttgtca agtttgtgtg agataaaggt gcacttggtt
2761 cgttcttgtc taatggcccc cgcacccatg ggtatttctt cagcttccac agtcatcccg
2821 acactagctg ggaagctcca gcagccctgg tcctggcccc agctctgtgg gcgctggccc
2881 tcaactttgc ctgcactgtg cttttgtgct attccccttg gtcctgtttg ggtgcaagtc
2941 cccctcacgc attgagttcc tgggccgctc aggctgctcc tgtgtctccc cagggaacat
3001 gaaggtcttg caggagccca cctgcgtctc cgactacatg agcatctcta cttgcgagtg
3061 gaagatgaat ggtcccacca attgcagcac cgagctccgc ctgttgtacc agctggtttt
3121 tctgctctcc gagtaagcct gcgctggagc tggaggtttg gggaggttgt gcccaaaggg
3181 tttgccccaa gagtgagctg ggtccaggtg gtgcgctgga gtgcaggatg ctgagtatgg
3241 tttgctgctg tttatatggt gttagagggg aggtcccatc tccagggaca tgttatgtaa
3301 gatacagtgg agcgcatggt gggagtgttg gtccacgtgg cacatggata cggctggaat
3361 actggactag accagcagtt ctcacacttt ttggtctcag gaccctttttt cacacttaaa
3421 aatgagtgag gacccaaagg gctttggtgt aggtaacaca tcattctatg tttacctaat
3481 tagaacttgc aatgaagaaa tggtgtaatt tttaaaaaat taaaacaatt aaaaattttt
3541 tttcttactg aaatggaggt ctcactgtgt tgcccaggct gctctcaaac tcctgggctc
3601 cagtgatcct cctgcctccg cctcccaaag tgctgggatt acaagcgtga gccgctgtat
3661 ccggcccaaa atggagaaat tttaagtccc aacaacatgc aagcccgcat tcaacaaatc
3721 ttcagatcaa ttacatgatc acaggtcatg tagcctctag aaaattccac tgtacgccag
3781 tgagagagag tgaaaaggca aataacgtcc ctgtattatg atgaaaagag ttttacctgg
3841 tgggcccaga ccacactttg agaaccactg gactagaccc ttgattgagg agtacggtgt
3901 tgagagtgga gtcctctgtg atggtggatg gaccaggaca catggcatag gagtcaggtg
3961 gttccctggg ctactccatg gtgcacagga tgcttcgtta cactggtgcc caggacataa
4021 tcacgtacac aagacacaca gttacggggc agactgggga tatacggcac accagcatgc
4081 agggttcacc agtaaaggtg gtattccatg attattctaa ggtagatggg ctgtgctttg
4141 tttccattgg cttagtccag ggattggcaa actatggccc gtgagccaaa tccggcccac
4201 tgcttgtttt tgtaaataaa gttttattgg aacacactgg ctgctgtagt tgtaacagaa
4261 actgcatggc cctcctttat gtttttttgtt tgtttgtttg tttgtttgtt tgttttcttt
4321 gagacagagt ttcgctcttg ttgcccaggc tggagtgcag tggcacaatc tcggctcact
4381 gcaacctctg cctcccgggt tcaagcgatt ctcctgtctc agcctcccga gtagttggga
4441 ttaatggtgc ctgccaccac acccggctaa tttttcgtat ttttagtaga gaccggtttt
4501 catcatgttg gccaagctgg tctcgaactc ctgaactcag gtgatccacc cgcctcagcc
4561 tcccaaagtg ctgggattac aggcatgagc cactgagccc ggcctcctcc tttatcttaa
4621 ttgaaataat tcagaaatgg aaagtcaaat actgcatgtt ctcacttata agtaagagtt
4681 aaataatgtg tacacatggg cattattcca tgtaccatgg aataacagac attgaagact
4741 tgggagggtg ggagaggggt gaaggaagag aagttactta atgggcatag tgtacaccat
4801 ttgggtgacg gacccaccag aaccccagac ttcaccacta ggcagcatat ccagtgagaa
4861 cagatctgag gcttgccatc aaaattgcac ttgtaaggcc gggcactgtg gtggctcgcg
4921 gctgtaatcc cagccctttg ggaggccgag gtgggcagat cacttgaggt caggagttcg
4981 agaccggcct ggccaacatg gtgaagctcc atctctacta aaaatacaac aattaactgg
5041 gtgtagtggc gcacacctgt aatcccagct actagggagg ctgaggcggg agaattgctt
5101 gagcccagga ggtggaggtt gcagtgagcc gagatcacat cactgtactc tagcctgggt
5161 gacagtgaga ctttgtctca ggaaaaaaaa acaaaaacaa aaaacaaaaa actcgtaccc
5221 cctaaattta tacaaataac caaaaaaaaa aaaaaaaaag gaaattgtgt ggctttgaag
5281 tccaaaatat taactatctg gcctgttaca gaaaaagttt gcagacccct ggcctagccc
5341 gtgagatgtg ggttggctgt taaggtggaa cattggaatt atcttacgat ggccaaactg
5401 tgcgatgcag agcttatgtt gttctaaatt aattagtgcc accggttctt ccctttcatg
5461 ggctttcagg aacaagctaa gtcccaggac cagggccggc agctaggcag gtgtgaggag
5521 catccttggt gcatgtggta agaggctgtg gccagcaaga gaggcaaccc tagtcggctg
5581 ccccagcaca ccctggccgc tcccaagccc ccagatctgt cctcacatcc gtgatcggga
```

5641 agctggaaga gtctgatgcg gttcctggag gcatgtcccg gacacagctg tggggcccag
5701 ccagcctaca ggtgaccagc ctaacccagc ccctgtgtct gcagagccca cacgtgtgtc
5761 cctgagaaca acggaggcgc ggggtgcgtg tgccacctgc tcatggatga cgtggtcagt
5821 gcggataact atacactgga cctgtgggct gggcagcagc tgctgtggaa gggctccttc
5881 aagcccagcg agcatggtga gcagggcgga gtgcggcagg ggtggctggg tgtgttccca
5941 cagctgcctg ggctgagggt ggggtgggca ggggaggagg tggggtcata gcaacagcag
6001 gaggaagccg cctgtatttt cccaaatctg atgggattcc tgcccctgcc tgggcctcag
6061 tcctcccacc tttgaaacgg agctggtcgc agtagaccac caagccccct tcagcccagc
6121 tgtttccacc cctgaactta agtgcccagg aaggcgtatt gagatgaggt gtgcttgctg
6181 gaaggcatgc ctgctgctga ttgaaaaccg aactgggaac attccttcca ttctgtgtcc
6241 actggtcagc tgctgcggct ttggatggtc ttgaccgtgg aaggctgacc ttcttctggt
6301 acccggagtc cctgcaggaa tccccccttga gcttgctggg ctgtggtgac aggagtttaa
6361 aacatgcgtt gtattccagt gatgcatgat atgacatgca tcacaggaat aaaaacctga
6421 ggtctcatgg atatgattgc ttcaaaggag accaagtttt aaaacagatg aatcaaaata
6481 aagaaaaata ctcagtaaat catcataaag tacagagatg tggccaaagg tgtgaaggat
6541 gcagctgtaa aagctgaagt ttgaggccgg gtgtggtggt tcatgcctat aatcccagca
6601 ctttgggagg ccgagcccag cggatcaccg gaggtcagga gttcgagacc agcctggaca
6661 acatggtaaa accccgtctc tactaaaaat acaaaaaatt agtctggcat ggtggcaggc
6721 gcctgtaatc ccagctactt gggaggctga ggtaggagaa tggcttgaac ccaggagaag
6781 gaggttgcag tgagcttaga tcatgctact gccctccagc ctgggcgaca gagtgagatt
6841 acgtctcaaa aaaataaaaa taaataaaaa taaaaagatt ttttaaaagg ctgaagtttg
6901 ggttactttg gctcatacac tttgccttca ctgtagaaag gtggttagta aagaccaggc
6961 gcggtggctc atgcctggaa tcccagcact ttgggagccc agcgcaggca gatcacttga
7021 gccctgggct attgaggctg cagtgagctg ggattgtgcc actgcactcc agcctgggca
7081 acagagtggg accctgtctc aaaaaagaag aaaaaaaggg taattaataa acactaaagt
7141 tctatgtaga attttagcaa cattattgtt attataatct tctttgctat ggctctgaat
7201 ctgtgtggtg ctccagaagt atgctatgga ggttttgtcg accaaaaatc tgggtggtgg
7261 ctgtggtttg taggccgggg ctgggctggg tgatgggggga gtcactgcat agatcctcac
7321 atagaggccg cttctcccgc agtgaaaccc agggccccag gaaacctgac agttcacacc
7381 aatgtctccg acactctgct gctgacctgg agcaacccgt atccccctga caattacctg
7441 tataatcatc tcacctatgc agtcaacatt tggagtgaaa acgacccggc agatgtgagt
7501 gggcatgctt tgacgttttt ctgtgacctc tggggaacag ggtgggtgac cagcagaggc
7561 ccagtccctg gagccaggag cctgggaggc aagccctggg gctggatagc aaatcccagg
7621 agctagagac ctggcttctc acctggctct gccctaggca agtccctttg cttcctggcc
7681 ccccacccct cacatcagag aaggggagtt atctctgcat gccgctcctc ctctgtaaag
7741 gtagggctgt gggccacatc tgtgtttccc agtttggggg acacaagtga tcgtaggtgg
7801 cacattgaca gctcacttga ataaccctat tattgaagag aataatactg actcaagaga
7861 cagtgacccg tgtcagttcc cttttgaggc caacgggtta aggaggaagt ccccatacag
7921 ctgactcgtt tactaattcc tcttaatgaa gagagcagag gccacacccc aggcttagac
7981 tttcccaaga aaacaagatc agtttgttgg ttgttcccca tggaagctgg tcctgacatt
8041 cccttcacag tagtgttggt ggagtttttg ttgttgtttg ttttgagaca gagtctcact
8101 ctgtcaccca gggtggaaca cagtggcgtg atcttggctc actgcaacct ccgcctcctg
8161 ggttctagcg attctcctgc ctcagcctcc tgagcagccg ggactacagg cacctgccac
8221 cgtgcccagc taatttttgt atatttagta gagatggggt ttcactgcgt tggccaggct
8281 ggtctcaaac tcctgacctc agatgatcca ctcgcctcgg cctcccaaag tgctgggatt
8341 acaggtgtga gccaccgcac ctggccagtg gagttccttc ttaagtacat gtattgacat
8401 ctttaaaaag ggcgagagga tttacaggaa actatcaggt cagtaatggc aggggccgtc
8461 cacagtgggt ggctgagtcc ccctattttt ctgctggtgt gcggggaggt catttcctgc
8521 cacccatgtt tccccaccct gaatccacct tcctcacatt cccattggag ggacactctc
8581 tggacatatg ggacctgggg tcccacaggg ctgcattcca atgcctgctg tgccactcgc
8641 cagctgtgtg atgttgggca tatcccataa cctctttgtg cctcagtttc ctcatctgta
8701 acacaggagt gacaagagca cccgcccaca gggctatgac agtacaaggt gtgtgataca
8761 gatgagctcc cctgtttggc ccacatgtgt cctaaaagcc atgtgccctt tctcttgagt
8821 gccccaggcc acagagatcc ccatctgccc gctgtcccac acactggtct gtcatttgtt
8881 ccttgaggtt tgtgagggcc ggctctgtgc atcccagggg cccaggctgg gcctggttgg
8941 ctctcaggga gcaggcaccc gccaccttaa gctcccatgc tggtgtctgt cactgcttcc
9001 tctcaatctg gccaagccag gggtgtcgat ttatatctct caggtctggt ttcccctttg
9061 gcactgggcc aggtatgggg aaagagcagg aatggggcag ttggctcaca cagcagaggc
9121 tcagaaagcg gggggcatgg ggggaaggag tgcacagatg ctagagagtg gggcaagttt
9181 tgtttggtca ataaatctcc ttctcatgcc ccaggcctgt gcaagaccta cagagagtcc

9241 caaggatggg ctggggggaa gagaaaggta ccaccttcag agtccaaaga tatgttattt
9301 aatattttca tatttctaga tctgccttca ggcatggctg gatccagctt ctaggaacct
9361 gtccagctct gcgccctgct ttattctgta ctggcttcgt ttttaggcag gctcttccct
9421 catgtagtgg cagatatgcc tactagttgc tccaggccta catcccaaag ccacagtggg
9481 aaaagggttt tttttcttga cggttctaat aagagtccta aggctgctgc tcagtggcct
9541 ggcttcgatg ctgtgccagc ctctgaacca atcactggct gtgggtggag agagggtgct
9601 ggtggagggc cctgcttgtc cagggaggag tcacatacct gcctctaggg ctgcaggtgg
9661 gctcagctcc atccaaacca gatgaactga aaataaggca ggagtggctt ccccagggga
9721 aactggggaa gaggaagcag gactgtgctg gctaaaatgc cagccaggtt taagacgtgg
9781 caccagatgc cagtcatggg attggattgg tcagcatgcc tgggctatgg cttaggggta
9841 tgttggtgct cagggatgcc acaggcctcc agataccagg tctgaggcag aagaatgaag
9901 tccagcttct cttgtgggtg gaacagtggc aactgagata ccccatctct cccttcccaa
9961 gaacagagct gaacataaag aatttagtga ttggccagag cttggccaca tgctcccctc
10021 tgatgaatga taggccaggt gatgggattg gcacaattgg cttagactaa tgagggttgg
10081 ccctggagtt gcaggcagtg gagttctgtc ctaagcagtg ggcacctaaa cccgatggca
10141 taaaagctgg gcgggtgtcc acctgcatct gccacagcac tgcaggcacc aactgtggct
10201 catactgagt gggataaatt ccagaaagaa acattaggaa cttactatag aattttgggg
10261 ctagagctac tcattcattc ccctagataa tttctaggca aggttccata gtggaggggg
10321 agttttggct tgggcattga aggatgcata ggagttttct agatggggaa agaagggaac
10381 ggtagaccag gcagagggaa ctgcatgata aaaggtttat gggtgtgaaa attcatggaa
10441 tgtttgagga ttatggggtt gggggatgtg ggaatatgtg tagcgataaa gcaccaaaca
10501 aagccaaaag tttagttaga gccctgaatg cctgcctcat aatggtttcc atattttata
10561 tgcctactat gtgccaggca cattgctcag ggtcacacag ctggaaatgg cagggctgag
10621 tttttgttgt tgttgttgtt gttgttgaga cagagtctca ctctatcacc caggctggaa
10681 tgcaggggcg tgatcatggc tcactgcatc cttgacttcc tgggatcagg tgattctccc
10741 acctctgcct cccaggtagc tgggactaca ggcacaggcc accacgccag gctaattttt
10801 tgtattttta gtagcgacag ggtctcgcca tgttgtccgg gctggtctgg atctcctggc
10861 ttcaagtgat ccccctggct cagcctccca aggtgctggg attacaggct tgagccaccg
10921 catccagccc agatctgaga tttgcaccca gtatttgaac tcccaagcct gtgctctttt
10981 tcctcccatg gacatttctc tcagagatgg tctcccaaac acctgtcctt cttgttaaaa
11041 aacagacaaa ccgcaagtag ttctttggaa gctcagattt ctcttttgtt tcttagtaaa
11101 acatttccca gttcccagct cccttccagg gtgtaagatt tcttcggtaa cttacatcta
11161 gctgttgctt cttgtttgct catgtttaga aagaaagaca aaagagagtg agaattttct
11221 ctccccttccc cagtctcccc acaactcaca ccccaccctc agctccctct gtaataggaa
11281 aatctctgaa ctctctgtag ttgctccagc aatcttttgg aactttgctt ctttcttgtg
11341 aaaaaacctc cccttggctc actttgcacc aggtttcccc aaatgtgctt ccaaccacaa
11401 gcagaaatgg agctgccagt aaccaggaag aaactgccgg gggctgagga agaggagagg
11461 gaggtgcata gccctggatc tcgcagggag aggggtgaca ggatgagaac tcaggttgct
11521 cacttgccat cagggtcagt catgaatata gcgttcatgt atcacttttt aaagcttttt
11581 tggagggtaa aagtaatagt tacacaaaat aaaaatacaa atggtacaaa aggacttaga
11641 atggaaacat gtttctctcc cgactccagc ctcctgtttt tcttcccaga gactgaccac
11701 tgctgtctgt ctcttgccag aagggaaagg gaggcaaggt tagggcaggc agagggcatg
11761 tgcatccttt agagagagct tatgtctata caagcaaatg tgtgtgttca gtcatcgctg
11821 tcttagtttt ctattgctgc ataataatgg tactaccagc ttcacagctt taaacaacac
11881 ccatttatta tctcatagtt tctgtggttg ggagtctgga catagcttag ccaggttctc
11941 tgctttagag tctcgtgagg ctataatcaa ggtgtgggat ggggctgcag tttcatctga
12001 ggctcaattg gggaagggtc acttctaagc tcatacaata ttggtgacat tcagtccctg
12061 gcaggctgtt gaactgagag cctcagtttc gtgctggctg ttggttgtag ttaaccctga
12121 attccttccc atgtgccctt tgcaaagcca tcaaggcaga gagacttgcc tagcaagtag
12181 gatattacag tcttctgtaa tataatcaca tccatgaaat cctctatata tcccatcacc
12241 tttaccatat tctgtgggtt agaaacaagt agcaggtcct gcccacactc gagaagacca
12301 gatgacacaa agatgtgatt caaagtgggg atcatcgggg ccatcttagg tttgtctgca
12361 gtgatcactg tgccatctct ctctctctct ctcttttttt tttttttccg agacgaagtc
12421 gtcactctgt cacccaggct ggagtgcagt ggcatgatct cagcttacca caatctctgc
12481 ctcccaggtt caaatgattc ttctgcctca gcctcctgag tagctgggat tacaggtgcc
12541 cgccaccaca cccagctaat ttttgtattt ttagtagaga cagagtttca ccatgttggc
12601 caggctggtc ttgaactcct cacctcaagt gatccaccca cttcggcctc ccaaagtgct
12661 gggattacag gcatgagcca ccatgcccag ccccatctct ctttaaaaaa caaacaaaca
12721 aacaaaaaac ataaaaagaa gcagagaaca catacacatc tgcatcttcc cttgtttact
12781 taacaataga tcttggaagt cacttctcag tagaggctag gttgggcaga gcattggatt

12841 ctaggccagt gagtttggac ttgaccatgg agacactagg aagcccatga aggacagaga
12901 gagatgcctc gaccctgcca gtcctttaga aagatcaccc agtgcttttt gtataccaaa
12961 ccctatttga aatacttacg tatattaacc catttcctta tcaccacaac cctgcgggaa
13021 gggagatagg cacttttatt atcttcattt tgcagatgag gacattgagg tccagagagg
13081 ttatgtcact tacttaaggt cacacagcca ggaagtggta gtagggactc ttacccttgt
13141 tttacagatg agattgaatt atctcacgaa aactcagaaa ggttaaacaa cttgcctaag
13201 taacatacag ctaattagtc gaggagcctg acgcatgttg ctctagcctg gtcacagtta
13261 cagaggtggc aagcaatggc ctgaacagga cgaacaacca aatacccagg ctggtggctc
13321 ttaaacatgg tggggtcagc taacgacagc aaccagggtg ggcactggtg cccctcgccc
13381 ccggctggtg ccctaacatc tccctttcct ctaccagttc agaatctata acgtgaccta
13441 cctagaaccc tccctccgca tcgcagccag caccctgaag tctgggattt cctacagggc
13501 acgggtgagg gcctgggctc agtgctataa caccacctgg agtgagtgga gccccagcac
13561 caagtggcac aactgtgagt atcaagaggc ctaagcaatg gtaatctcca ctctccattc
13621 ttcccctgtg gccagacact tcccctggct gagtctctgg gcttttatat cataggatgc
13681 ctctaatggc aatcctgcca ttagatacac ctgccgtggt gtatctgcca ggtaggcagg
13741 ctaggctgca gtaacacaca agcccacaat ttccatggct taacactata ggaatatatt
13801 tcttgctcac gtaacaagct aacgtgaatg ttgctggttt gtaggtggtt tccctccctg
13861 tagaaatctg ggagtgaggg ttctttccat cttgtggtgc catcattctc caggacaaag
13921 attcttacct acttttgtgt cctggtttcc tttggcagcc tggtgaagcc tatggacctc
13981 atttcagaat atttttaaat acataaaatc ccagcctggg caatatagtg aaacccccat
14041 ctgtacaaaa attagccagg catggtggca tgcacctgta gtcccaggta ctgggaaggc
14101 tgaggtggga ggatcacttg agcccaggag tttgaggctg cagtgagccg tgatcgtacc
14161 actttactcc cacctgggtg acagagcaag agcccatctc taaaaataaa taaatacaat
14221 gaaataaaat aaaataaaat aaatagaact acagaggaaa ctaattgtat tgaaatgcag
14281 ttataaaaca tttaaacaca tttttaatct agagatatat gtgcttcttt attaagatct
14341 ataaataata agttctaggg gtagctcgca taaatactgt aatttcaaag tagataagca
14401 taaataatac tttatgatac tgaaattgtg atgtgatatg agaatagctg tgagttttgt
14461 tttgctgggg acaggatcac tgatgctgtc attactgggg tctcttccct ccattctttt
14521 tttaaaattg tattttattt tatttttaaa attttaaaat aaatagagac agggtatcac
14581 tatgttgccc aggctgcttt tgacctcctg ggctccagtg atcttcccat cttggcttcc
14641 caaagtgctg ggattacaag tgggagccag tgttcctggc cccttcctcc attcttaatg
14701 gaaggagatg ctaggtgtga gaggttaggg aaagtaaaga tgtaatttct ttcccatcca
14761 agttctcaga cccctgaatt ctacctgcag ccatgttggt ccatcaaccc caagtgaaga
14821 atccctgctc tagggcccca ccattgtctg tatccagcca gcagaagagg cgtgattatg
14881 gagatcacat ctgcttcttg aaagcagaca gcccggaagt gggccgcatc acttcctctc
14941 aaattctatt ggtgaaaatg gtcacatgac tacacatagc cacaaaggag gctgggaact
15001 ttctcacttg gaacctacat cccagaaaca actcttttca gtgaggtatc ccacaggtct
15061 ttcgcagtag aaatattgat tatctcacat aaaatgaagt cttacaaatg gacctactgg
15121 gttttgtaca gcagccaagt gatatctctt cccttctgct gtcttccctt ctgccatcct
15181 tcacatggtg gcattgtatc cttagacttg ccacccatgc cctcaggttg gccgttgcac
15241 actgtcttac ataaagcagg aaggaaagga aaggctgcta cgagagagtg taccttgtgc
15301 atctcttttt taatcaggaa gcaaacatct ttctagaagc ttccctagca aaattcccct
15361 tacatctcat tggccaagac tgttacatgt tacatggtta ctgttattac ttgctcattg
15421 caaggaagac tgggaactca aatgcctgga aaaaggaaca ggataatcgt gattggctca
15481 agccttaggg tgggcatggc tccctgacaa gggagagagg aaaaagctgt tgagtgaaga
15541 agactgcttc agtttcccca tctgtataat gggaggagta agggctgtcg tgaaaactca
15601 atgaaagaag attcttcaac gtggtaggtg cagtggcagc tggcagtacc ctgaccctgc
15661 caccgcacag ccctctcagc attgctcatc ctgcactgtg gatatcagtt gagccacgtg
15721 tctcctgccc tggctgtga gctccatagg cagggtctcc atggctgtat ctccagaacc
15781 cagcacagaa ccaggtgctt gggaaagttt tgaattgatt ctcatctgcc attggcatgg
15841 ggaagggaac tagcttgtat gaaacagata acaatgtatg ggaccctcat tcattatttc
15901 agcaaatatt tgctgagttc ctcctacatg gctagccctg tgctagacac tggggaatcg
15961 gcgatgaaca aagcagatag aaatccccac tcttgtggag ctgacattct ggagggagag
16021 acaaaaagca aacatataaa gaaagaaaga aatcacatgg atctggatga cagtgagtgc
16081 tgggaagaaa ataaaagcag aggaagggga tggagcgatg ggcagggggc aacggtaggg
16141 agggtgtcgg ggaaaacttt ttggagaatg tgacgatgaa agtgaacaag gagaagtcaa
16201 ccgtgttgag atgatggcag ctaatgatgt ggacaggcca ctctgttctg agtgcattat
16261 ctattgattc atcatgtcat cctcgcaaca gccctgcacg atcaattctg tcattaaccc
16321 catagtacag atgaggatgc ggaggcacag agaagataag ggacttgtcc tgtgtcacac
16381 agcaaggagc catccggctc ctaagttggt gcatttgact tctgtgcttc cggaaagaaa

16441 gagcagcaag tttaagatct ggaggtggca ctgagctttg gaggagcagg gggcaatgag
16501 gtggccggtg tgacgaggac tcaatgtgca agagggagag tggtggggag atgaggtgga
16561 ggggtggtcg gcggtcagat cgtggagggt ctcggacgag ggtcctgacc ctgggtctcc
16621 agtcctggga agtggagccc aggctgtacc atggctgacc tcagctcatg gcttcccctc
16681 ccacttccag cctacaggga gcccttcgag cagcacctcc tgctgggcgt cagcgtttcc
16741 tgcattgtca tcctggccgt ctgcctgttg tgctatgtca gcatcaccaa gtgagtcctg
16801 ggcccagtgc tgccgagcag tccctctgga gtgcagggtg gcagggactt gcccctctag
16861 tctgcccctt tgcagtcctc tcagtcaata atacgtattt actgagcagc tactacacac
16921 cttgagagta gagctgagaa catatcgaca aggaccccac tttttctttt tttttttttt
16981 tttttttttt tgagacggag tctcactctg tcacccaggc tggagtatag tggcacaatc
17041 ttgcctaaca gtaacctccg cctcccgggt tcaagcaatt cttctgcctc agcctccaga
17101 gtagctggga ttacaggcgc atgccactat gcccggctaa tttttgtat ttttggtaga
17161 gatggggttt caccatgttg gtcaggctgg tctcgaactc ctgacctcat gatctgcctg
17221 cctcagcctc ccaaagtgct gggattacag gtgtgagcca ctgcacccaa ccaggactcc
17281 acatttctaa aaccggcatc ctactgggga gactgaaaat acatatcaat cacaaacagg
17341 tggttttcca tagtgaccca ctctctgaat gcactagacc agggtggagg ccagagatct
17401 tctggggtgc tttttgcaag gggaccagg ataaggctct ccaaggaggg aaaatttgag
17461 gggggccctg actggggaga atgagctggc cagggataag caagatggag tcatcccaca
17521 tccccttaca acactgggtg cctgggcaac tgggggcatt tgggggcatg tggtaggagc
17581 cagaggaatt tgcgacgatt gccctgatgg agtcaggaga cctgggtttg aatcctggcc
17641 ttggagcttg gtagctggcg gccgacaagt tgctgaaacc cctgagcctg gggttcctgc
17701 tttgcagagt gacagtgatg gtgagaacat atttcatcag ccagaagagg ccaaatcaca
17761 gtaaaggctg agggaggaga tgagtggcga gtggctggga ggtggtggaa ggagcctcgt
17821 ttccagagag ctcttgccag cccttggaat catggtgtct cagagcctca gtcctcccat
17881 ctctgaaatg ggactagcaa gctcaacctc actaagtcag gattagaggt ggctaaggat
17941 tattaacatg attgatgaaa gtgcccactc ttggcccagc acacactagg taggcaggga
18001 atgcaaattc ccctccatat cttgtcactg atgcctccga gcaaccttgg actgatcgcc
18061 ttgctctgag cctcagtttc cccatcacct gtacctcttc ccactcccca tcactatatc
18121 ccagcatgcc agcctctttg ctgttctttg tctttggttt cttgttttgt tctgtttttt
18181 agacagggtc tcactctgtt agccaggctg aagtgcagtg gcgcggttac ggctcactgc
18241 agcctccaat tcctgggcta aagagatcct cccatttcaa cttccagagc agctgggaca
18301 acaggcgctt gccaccacac ctggctaatt ttcttatttt aatttaattt tattttattt
18361 tttgggacag agtggagtct caaaaaccaa gctagagtgc agtggtgcga tctcgactca
18421 ctgcaatctc tgcctcccgg gttcaagcga ttctcctgcc ttagcctccc gactagctgg
18481 gattacaggc gtgtgccacg acacccagct aatttttgta tttttagtag agatggggtt
18541 tcaccatgtt ggccaggatg gtcttgaact cctgacctca agtgatccac ccacctcgtt
18601 ctcccaaggt gctgggtaca ggcatgagcc actgtgcctg gccaattttc ttacattttg
18661 tagagactgg ctgtcactta tgtagcccag gctgatcttg aacttctacc cctttatctt
18721 tattcatggc acttattacc atgaatgaat gacctcatat aagcatttct ttcgtttttt
18781 tttttttttc tttgagatgg agtctcatgt tgtcccccag gctggagtgc agtggcgcga
18841 tctcagctca ctgcaacctc cgccttccgg gttcaagcga ttctcctgcc tcagcctcct
18901 gagtagctgg gattgcaggc gcctgccacc atgcctggct aagttttgca tttttagtag
18961 agacggtgtt tcaccatatt ggccaggctg gtctcgaact tctgacctca ggtgatacac
19021 ctgccttggc ctcccaaagt gctgggatta caggcgtgag ccgccatgcc tggcctcata
19081 taagcatttc tgtctccatt tatcatccat ctttccctct tgaaggtcag tttcaccaag
19141 gcaggcatct ttgtctcgtt cactgttgtg gcctcagggc caggcacagt gagtcaaaca
19201 tagaaggtgc tcaataaata tgtgtttatt tattgaaacc atgggcagag gctaattcag
19261 aagcggtctg aggaccttac ctcccagtga tgatgcacca tggccccagg caggccagga
19321 agagagaagg gttgtgtttc tccgtaggtc ccccagcttc ccaggccatc ccaggccatt
19381 ccctggtcat ttgccctcag ctgctctgaa aaagggattg ttgaggggaa cctagaatcc
19441 tctctctgca gtttgagtct ttcctaatcc cctggggtct cattcccact gaggacatag
19501 gtggcctcct caggaactct gtgctgggta acagaatgcg ggagtgtgaa cctggctctg
19561 ccacctacca gctgtcactc cacctccttg ggcctcactc tcctcatctg tagaataggg
19621 ttagcaatag aatccatgtc accaggttag aatgatgagt cagtggtttg acctccagaa
19681 actaatcagc ctgatctctg atgccaaata agtattggtg ataacgacca cttttatggg
19741 aggagcgttc acctgtcaat aattcagaga tcaacacctt ttccttttgt ttttcaggat
19801 taagaaagaa tggtgggatc agattcccaa cccagcccgc agccgcctcg tggctataat
19861 aatccaggat gctcaggtag gagtaggcgt ggatgaggac atgtgggact gtgtacatga
19921 agaagtgtgg ttcagaacac ctgggctgtt aaggaccttc actggcttct ggaatggcaa
19981 atagacagtc aggagggttg caggggagac agaggcagaa gccgaatgag gtcattagca

```
20041 gaccagaggc tttcccgccc ttccccttgg caatcccagc ctggggtggg cttctctggg
20101 gttggtttcc tgtttttttc cctcccccttg ggagaatgac ccttgggtca tcatcactgt
20161 gtcattccct ggggaggtgc cagtaccagg gctagaggcc agaaggagtg gaggaaggag
20221 agggtgacag gctttctgtg tcttcttctt aagcatagga aactgccccc gaagcactag
20281 caaatccctt ccgggttctc attggcctga aatgtatccc acccctaagc caggggtgga
20341 gtcagcttcc ccaaggcgat ggtcctgtgg gtgagtgggt ggggtttgcc tgagcaagat
20401 gagagttctc taggtaggag aaagggggat tataggtcct gtctagaaga gaaggtctga
20461 gggtccttgc ttttccaggg actctggaat ctagtggtgt tggctttgaa tcctgactct
20521 gccactcact ggcagtgtgg acttgagcaa gttgcttaat tctctgagcc tcagtttcct
20581 cttgtgggtt ataacagtgt ttacctggta ggacagatat tggaatttat tgagacaata
20641 catataaagt gcatattcca gcctcttgca aataccaagt gccatttatg tatcagttag
20701 tgtttgctgt gtaacaaatg accccgaaat gtagagggtt acaacaactt tatttagctt
20761 atgcttctgc aggctggcat ttggggctgg gctcagcagt gagggtggcg ggggaggctg
20821 ggctgggctg ggctgggcag atctgaattg agctgacccg tccccgtagc ctccctccgt
20881 gtctgacagt tggctttttt tttttttttc tttttctgag acggagtttt gctcttattg
20941 cccaggagtg caatggcgtg atcttggctc actgcaacct ctgcctcctg ggttcaagca
21001 attttcttgc ctcagcctcc caagtagctg ggattacagg catgtgccac cacgccaggc
21061 taattttgta tttttaatag agatggggtt tcttcatgtt ggtcaggctg gtctggaact
21121 cctaatatca gatgatccac ccacctcagc ctcccaaagt gctgggatta caggcgtgag
21181 ccactgcacc cagcctagtt ggctgacttt tacctgggac agtgcaggtg cctgagccat
21241 gtgcctctca ctctccagca ggccggccca ggcttgttta cagagtggct cagttttcaa
21301 gggtgggaag tcccaaggct tcttgaggcc taggcgcagc actggcatga tatcacttcc
21361 atcacattct atgggcccaa gcaagtccca gggccagtgt agattcaagg gatgggagga
21421 gattcagagc actcctctgt ggccactttt gccatcgacc acagtccctg taaatattag
21481 gacaatgtaa ttaattccca ggaatctgag gctcagaaag cgtaagtgac ctgttggact
21541 tctgatctgt gtgatgtcga ggcttgtacc ccttcctgag cattgccgta ctccaggccg
21601 ggctgcaagg ccactctgct ctttcattgg ctgtctctgt attttagggg tcacagtggg
21661 agaagcggtc ccgaggccag gaaccagcca agtgcccgta tgtatctgaa cttaggtcac
21721 agcctgcatg cattgggaag gtgatagaat tggagaggca agcccctagc tccatgtctg
21781 ccttctcttc cctgcattcg gtaattgccc tgtgacatta gccttcaagg gacggcagga
21841 ggaggggtgt tctggaaacg tggactgctg gccaagcccc ctgagtttca ctggtgtgtc
21901 aggtacatgg tgatacccct tgggagtgct gttatagtta acaaccagag cagccgtgcc
21961 tgttgttaaa atcttgacct aattgtatac ttgtcggcaa atagccacta tcctgaacac
22021 tcccctcctt ttttttaata tacaggatct cactctgtgg cccaggctgg tgtgcagtgg
22081 tgcgatcata gctcactgca ccttcaaact cctgagctca agtgatcctc ccatcttagc
22141 ctcccgagta gctgatacta cagatgtgca ttaccacgcc tggctatttt aaaaggtttt
22201 tgcctgtaat tccagctact caggaggctg aggcatgaga atcacttgaa cccgggaggc
22261 agaggttgca gtgagcgcag attgtgccac tgcactccag cctgggcgac agagtgagac
22321 tcttgtctca aaaaaaataa taccaaaaaa agtttttgta aagacaagct ctcgctgtgt
22381 tgccccgcca ctgtggcctc cttagcttct tccctggggc ctgctggacc tttccatact
22441 ccagaaacta aagggggtcc aggaccctgc ttcaacccta ggatcccgca tctttttttt
22501 tttttttttt ttggacgcag ggtcttgctg tgtccctcag gctggagtgc agtgattcac
22561 tgcagcctca aactcgtggg ctcaagtgat tctctagcct cagccttcta agtagctggg
22621 actacagtca tacaccaaca tgcccagcta attttccttt tttttaattc ttgtagagat
22681 gtttgagacg gcttgggctc tgttgcccag gctgttctca aactcctgag ctcaagcgat
22741 cctccctcct cagcctccta aagtgctggg attacaggcg tgagccaccg cacccggctt
22801 ccatatcctt tctaattggt catggcttgg gataatggtg ttgcttttaa ttatcatcat
22861 ccataaagac tttttcttac tcaacagatc tgagcttgta tttggtgccc aggacatgtg
22921 ctgggttccc gaaatcccaa agacacagac cctaccctca gggatttctc attctagcaa
22981 catagactga tcaattactg attataacgt tagaaggcat gtctgaagta gacagccatc
23041 aggacatggt gatttcaggc tgggctttga agaatgaata ggagtttttc aagtgtcgaa
23101 actgaaccct gaccaacctt tgcttttgca gacactggaa gaattgtctt accaagctct
23161 tgccctgttt tctggagcac aacatgaaaa gggatgaaga tcctcacaag gctgccaaag
23221 agatgccttt ccagggctct ggaaaatcag catggtgccc agtggagatc agcaagacag
23281 tcctctggcc agagagcatc agcgtggtgc gatgtgtgga gttgtttgag gccccggtgg
23341 agtgtgagga ggaggaggag gtagaggaag aaaaagggag cttctgtgca tcgcctgaga
23401 gcagcaggga tgacttccag gagggaaggg agggcattgt ggcccggcta acagagagcc
23461 tgttcctgga cctgctcgga gaggagaatg ggggcttttg ccagcaggac atgggggagt
23521 catgccttct tccaccttcg ggaagtacga gtgctcacat gccctgggat gagttcccaa
23581 gtgcagggcc caaggaggca cctccctggg gcaaggagca gcctctccac ctggagccaa
```

```
23641 gtcctcctgc cagcccgacc cagagtccag acaacctgac ttgcacagag acgcccctcg
23701 tcatcgcagg caaccctgct taccgcagct tcagcaactc cctgagccag tcaccgtgtc
23761 ccagagagct gggtccagac ccactgctgg ccagacacct ggaggaagta gaacccgaga
23821 tgccctgtgt cccccagctc tctgagccaa ccactgtgcc ccaacctgag ccagaaacct
23881 gggagcagat cctccgccga aatgtcctcc agcatggggc agctgcagcc cccgtctcgg
23941 cccccaccag tggctatcag gagtttgtac atgcggtgga gcagggtggc acccaggcca
24001 gtgcggtggt gggcttgggt cccccaggag aggctggtta caaggccttc tcaagcctgc
24061 ttgccagcag tgctgtgtcc ccagagaaat gtgggtttgg ggctagcagt ggggaagagg
24121 ggtataagcc tttccaagac ctcattcctg gctgccctgg ggaccctgcc ccagtccctg
24181 tccccttgtt cacctttgga ctggacaggg agccacctcg cagtccgcag agctcacatc
24241 tcccaagcag ctccccagag cacctgggtc tggagccggg ggaaaaggta gaggacatgc
24301 caaagccccc acttccccag gagcaggcca cagacccccct tgtggacagc ctgggcagtg
24361 gcattgtcta ctcagccctt acctgccacc tgtgcggcca cctgaaacag tgtcatggcc
24421 aggaggatgg tggccagacc cctgtcatgg ccagtccttg ctgtggctgc tgctgtggag
24481 acaggtcctc gccccctaca accccccctga gggccccaga cccctctcca ggtggggttc
24541 cactggaggc cagtctgtgt ccggcctccc tggcaccctc gggcatctca gagaagagta
24601 aatcctcatc atccttccat cctgcccctg gcaatgctca gagctcaagc cagaccccca
24661 aaatcgtgaa ctttgtctcc gtgggaccca catacatgag ggtctcttag gtgcatgtcc
24721 tcttgttgct gagtctgcag atgaggacta gggcttatcc atgcctggga aatgccacct
24781 cctggaaggc agccaggctg gcagatttcc aaaagacttg aagaaccatg gtatgaaggt
24841 gattggcccc actgacgttg gcctaacact gggctgcaga gactggaccc cgcccagcat
24901 tgggctgggc tcgccacatc ccatgagagt agagggcact gggtcgccgt gccccacggc
24961 aggcccctgc aggaaaactg aggcccttgg gcacctcgac ttgtgaacga gttgttggct
25021 gctccctcca cagcttctgc agcagactgt ccctgttgta actgcccaag gcatgttttg
25081 cccaccagat catggcccac gtggaggccc acctgcctct gtctcactga actagaagcc
25141 gagcctagaa actaacacag ccatcaaggg aatgacttgg gcggccttgg gaaatcgatg
25201 agaaattgaa cttcagggag ggtggtcatt gcctagaggt gctcattcat ttaacagagc
25261 ttccttaggt tgatgctgga ggcagaatcc cggctgtcaa ggggtgttca gttaagggga
25321 gcaacagagg acatgaaaaa ttgctatgac taaagcaggg acaatttgct gccaaacacc
25381 catgcccagc tgtatggctg gggctcctc gtatgcatgg aacccccaga ataaatatgc
25441 tcagccaccc tgtgggccgg gcaatccaga cagcaggcat aaggcaccag ttaccctgca
25501 tgttggccca gacctcaggt gctagggaag gcgggaacct tgggttgagt aatgctcgtc
25561 tgtgtgtttt agtttcatca cctgttatct gtgtttgctg aggagagtgg aacagaaggg
25621 gtggagtttt gtataaataa agtttctttg tctctttatt ttttatgtat taaccaaaca
25681 tacctccaga cactgctgtg agtgctgtgt ctctgttaac tcctggaatt cacccatcca
25741 gaggaaccag gatgcaagag gttaagaaac ttgccatctg ggtttgggtt ccccatacaa
25801 ggattcaaat agttgattta ggaagtaatc ccgggaaacc ctgctaaggt agtggggaac
25861 tgaggcaggg aaggacacaa accaagaaag tgttacctga aaggggtcca gatgcagacc
25921 ccaaaagagg gttcttgaat ctcatgcaag aaagaattca gagcgagtcc atagagtcag
25981 tgaaagcaag ttaatgagga aagtaaagga ataaaagaat ggctactccg tagacagagc
26041 agccctgagg gttgctggct gcctattttt atggttattg attaattata ttccaaacaa
26101 ggggtggatt attatgcctc cctttttagac catatagggt aacttcctga tgttgccatg
26161 gcatttgtaa actgtcatgg cgctgttggg agtgtagcag tgaggacaac cagaggtcac
26221 tcttgttgcc atcttggttt tggtgggtta gagccatctt ctttactgca acctgtttta
26281 tcagcaaggt ctttatgact tgtatcggtg acgacctcct gtctcattct atgactaaga
26341 atgccctaac ctcccaggaa tgcagcccag taagtctcag cctcatttta cccagcccct
26401 cttcaaagct ccagtttaaa taaacctctg acaaaagggt gagttattca acagattacc
26461 agcatgagca actgatgctt acctgccggg gatctctgga agaccatgca tggcacatgc
26521 ccagttatgc ctgcaaagga gagggagctg (SEQ ID NO: 3)
```

SEQ ID NO: 3 is also illustrative for an IL4R coding sequence which is homologous to the wild-type IL4R sequence shown in SEQ ID NO: 1 and which comprises additional identified variations to the wild-type sequence. Said SEQ ID NO: 3 is accessible under Accession number AF421855 (gi: 15987825) of the NCBI database.

Variations to the wild-type sequence of IL4R are deducible from this NCBI entry and are also illustrated by the following information. These variations may also be comprised (and in addition) be detected in the methods provided herein. Accordingly, the exemplified variations provided below represent some naturally occurring variants or isofoms of human IL4R. The means, methods and uses provided in this invention are also related to the determination of the I50V/I75V variant of IL4R in these isoforms/variants.

Such isoforms/variants/variations comprise the following (in respect to SEQ ID No. 3 provided above):

| | |
|---|---|
| repeat region | 1216..1313 /rpt_family="L2" /rpt_type=dispersed |
| repeat region | 1314..1606 /rpt_family="AluJo" /rpt_type=dispersed |
| variation | 1454 /gene="IL4R" /frequency="0.01" /replace="a" |
| variation | 1596 /gene="IL4R" /frequency="0.01" /replace="c" |
| repeat region | 1607..2263 /rpt_family="L2" /rpt_type=dispersed |
| variation | 1673 /gene="IL4R" /frequency="0.02" /replace="c" |
| variation | 2265 /gene="IL4R" /frequency="0.12" /replace="t" |
| variation | 2371 /gene="IL4R" /frequency="0.14" /replace="a" |
| repeat region | 2404..2593 /rpt_family="LTR33" /rpt_type=dispersed |
| variation | 2438 /gene="IL4R" /frequency="0.02" |
| variation | /replace="t" 2487 /gene="IL4R" /frequency="0.02" /replace="g" |
| variation | 2490 /gene="IL4R" /frequency="0.02" /replace="a" |
| variation | 2517 /gene="IL4R" /frequency="0.02" /replace="c" |
| variation | 2740 /gene="IL4R" /frequency="0.01" /replace="a" |
| variation | 2782 /gene="IL4R" /frequency="0.17" /replace="a" |
| variation | 2820 /gene="IL4R" /frequency="0.02" /replace="a" |
| variation | 2909 /gene="IL4R" /frequency="0.13" /replace="t" |
| variation | 3337 /gene="IL4R" /frequency="0.03" /replace="a" |
| repeat region | 3373..3536 /rpt_family="Charliel" /rpt_type=dispersed |
| repeat region | 3537..3666 /rpt_family="FLAM_C" /rpt_type=dispersed |
| variation | 3619 /gene="IL4R" /frequency="0.15" /replace="a" |
| repeat region | 3677..3847 /rpt_family="Charliel" /rpt_type=dispersed |
| repeat region | 3849..3870 /rpt_family="MER5A" /rpt_type=dispersed |
| variation | 3913 /gene="IL4R" /frequency="0.17" /replace="t" |
| variation | 4083 /gene="IL4R" /frequency="0.44" /replace="c" |
| repeat region | 4158..4236 /rpt_family="MER58A" /rpt_type=dispersed |
| repeat region | 4280..4604 /rpt_family="AluSq" /rpt_type=dispersed |
| variation | 4395 /gene="IL4R" /frequency="0.04" /replace="g" |
| variation | 4503 /gene="IL4R" /frequency="0.16" /replace="c" |
| variation | 4557 /gene="IL4R" /frequency="0.01" /replace="g" |
| variation | 4560 /gene="IL4R" /frequency="0.47" /replace="g" |
| variation | 4606 /gene="IL4R" /frequency="0.01" /replace="t" |
| repeat region | 4609..4896 /rpt_family="L1PB4" /rpt_type=dispersed |
| variation | 4841 /gene="IL4R" /frequency="0.16" /replace="t" |
| repeat region | 4897..5209 /rpt_family="AluSx" /rpt_type=dispersed |
| variation | 4905 /gene="IL4R" /frequency="0.19" /replace="g" |
| variation | 4918 /gene="IL4R" /frequency="0.34" |
| variation | /replace="a" 4986 /gene="IL4R" /frequency="0.02" /replace="a" |
| misc feature | 5165..5307 /gene="IL4R" /note="Region not scanned for variation" |
| repeat region | 5210..5232 /rpt_family="L1PB4" /rpt_type=dispersed |
| repeat region | 5234..5331 /rpt_family="MER58B" /rpt_type=dispersed |
| **variation** | **5758 /gene="IL4R" /frequency="0.48" /replace="a"** |
| variation | 5914 /gene="IL4R" /frequency="0.35" /replace="t" |
| variation | 5953 /gene="IL4R" /frequency="0.38" /replace="t" |
| variation | 6162 /gene="IL4R" /frequency="0.02" /replace="g" |
| **variation** | **6222 /gene="IL4R" /frequency="0.42" /replace="g"** |
| variation | 6235 /gene="IL4R" /frequency="0.01" /replace="t" |
| variation | 6235 /gene="IL4R" /frequency="0.02" /replace="a" |
| variation | 6244 /gene="IL4R" /frequency="0.02" /replace="c" |
| variation | 6296 /gene="IL4R" /frequency="0.01" /replace="t" |

(continued)

| | |
|---|---|
| variation | 6447 /gene="IL4R" /frequency="0.08" /replace="a" |
| repeat region | 6565..6876 /rpt_family="AluSx" /rpt_type=dispersed |
| variation | 6568 /gene="IL4R" /frequency="0.02" /replace="t" |
| variation | 6619 /gene="IL4R" /frequency="0.02" /replace="g" |
| variation | 6663 /gene="IL4R" /frequency="0.02" /replace="g" |
| variation | 6834 /gene="IL4R" /frequency="0.02" /replace="c" |
| repeat region | 6955..7117 /rpt_family="AluJb" /rpt_type=dispersed |
| variation | 7086 /gene="IL4R" /frequency="0.01" /replace="a" |
| variation | 7339 /gene="IL4R" /frequency="0.05" /replace="a" |
| variation | 7482 /gene="IL4R" /frequency="0.06" /replace="t" |
| variation | 7595 /gene="IL4R" /frequency="0.01" /replace="t" |
| variation | 7613 /gene="IL4R" /frequency="0.01" /replace="t" |
| variation | 7653 /gene="IL4R" /frequency="0.04" /replace="a" |
| variation | 7685 /gene="IL4R" /frequency="0.01" /replace="g" |
| variation | 7687 /gene="IL4R" /frequency="0.37" /replace="t" |
| variation | 7745 /gene="IL4R" /frequency="0.08" /replace="a" |
| variation | 7758 /gene="IL4R" /frequency="0.50" /replace="g" |
| variation | 7906 /gene="IL4R" /frequency="0.05" /replace="a" |
| repeat region | 8065..8366 /rpt_family="AluSx" /rpt_type=dispersed |
| variation | 8199 /gene="IL4R" /frequency="0.02" /replace="t" |
| variation | 8293 /gene="IL4R" /frequency="0.01" /replace="t" |
| variation | 8318 /gene="IL4R" /frequency="0.42" /replace="t" |
| variation | 8414 /gene="IL4R" /frequency="0.01" /replace="a" |
| variation | 8443 /gene="IL4R" /frequency="0.14" /replace="a" |
| variation | 8503 /gene="IL4R" /frequency="0.35" /replace="a" |
| variation | 8576 /gene="IL4R" /frequency="0.48" |
| repeat region | /replace="a" 8595..8737 rpt_family="MIR" /rpt_type=dispersed |
| variation | 8616 /gene="IL4R" /frequency="0.49" /replace="a" |
| variation | 8749 /gene="IL4R" /frequency="0.07" /replace="t" |
| variation | 8851 /gene="IL4R" /frequency="0.08" /replace="a" |
| variation | 9130 /gene="IL4R" /frequency="0.01" /replace="a" |
| repeat region | 9322..9751 /rpt_family="MLT1K" /rpt_type=dispersed |
| variation | 9399 /gene="IL4R" /frequency="0.49" /replace="a" |
| variation | 9596 /gene="IL4R" /frequency="0.10" /replace="a" |
| variation | 9640 /gene="IL4R" /frequency="0.01" /replace="c" |
| variation | 9787 /gene="IL4R" /frequency="0.05" /replace="" |
| repeat region | 9990..10091 /rpt_family="MLT1L" /rpt_type=dispersed |
| variation | 10182 /gene="IL4R" /frequency="0.47" /replace="a" |
| variation | 10183 /gene="IL4R" /frequency="0.47" /replace="t" |
| repeat region | 10296..10450 /rpt_family="L2" /rpt_type=dispersed |
| variation | 10411 /gene="IL4R" /frequency="0.04" /replace="t" |
| variation | 10487 /gene="IL4R" /frequency="0.05" /replace="a" |
| repeat region | 10583..10619 rpt_family="MIR" /rpt_type=dispersed |
| variation | 10607 /gene="IL4R" /frequency="0.04" /replace="g" |
| repeat region | 10620..10929 /rpt_family="AluJb" /rpt_type=dispersed |
| variation | 10644..10646 /gene="IL4R" /frequency="0.96" /replace="" |
| variation | 10725 /gene="IL4R" /frequency="0.43" /replace="c" |
| variation | 10871 /gene="IL4R" /frequency="0.05" /replace="a" |
| variation | 10895 /gene="IL4R" /frequency="0.02" /replace="a" |
| variation | 10920 /gene="IL4R" /frequency="0.09" /replace="a" |
| repeat region | 10930..10978 rpt_family="MIR" /rpt_type=dispersed |

(continued)

| | |
|---|---|
| variation | 11135 /gene="IL4R" /frequency="0.02" /replace="g" |
| variation | 11351 /gene="IL4R" /frequency="0.08" /replace="t" |
| repeat region | 11819..12359 /rpt_family="MLT1E" /rpt_type=dispersed |
| variation | 12007 /gene="IL4R" /frequency="0.02" /replace="g" |
| variation | 12091 /gene="IL4R" /frequency="0.06" /replace="c" |
| repeat region | 12380..12692 /rpt_family="AluSx" /rpt_type=dispersed |
| variation | 12408 /gene="IL4R" /frequency="0.43" /replace="t" |
| variation | 12598 /gene="IL4R" /frequency="0.06" /replace="a" |
| variation | 12604 /gene="IL4R" /frequency="0.06" /replace="a" |
| repeat region | 12729..12811 /rpt_family="L1MC4a" /rpt_type=dispersed |
| variation | 12787 /gene="IL4R" /frequency="0.04" /replace="c" |
| variation | 12822 /gene="IL4R" /frequency="0.02" /replace="c" |
| repeat region | 12941..13228 /rpt_family="MIR" /rpt_type=dispersed |
| variation | 12951 /gene="IL4R" /frequency="0.01" /replace="a" |
| variation | 13060 /gene="IL4R" /frequency="0.01" /replace="c" |
| variation | 13168 /gene="IL4R" /frequency="0.06" /replace="a" |
| variation | 13243 /gene="IL4R" /frequency="0.13" /replace="g" |
| variation | 13291 /gene="IL4R" /frequency="0.02" /replace="t" |
| variation | 13715 /gene="IL4R" /frequency="0.33" /replace="t" |
| repeat region | 13720..13915 /rpt_family="MLT1J2" /rpt_type=dispersed |
| variation | 13757 /gene="IL4R" /frequency="0.29" /replace="a" |
| variation | 13790 /gene="IL4R" /frequency="0.24" /replace="g" |
| variation | 13810 /gene="IL4R" /frequency="0.33" /replace="t" |
| variation | 13902 /gene="IL4R" /frequency="0.23" /replace="g" |
| variation | 13912 /gene="IL4R" /frequency="0.02" /replace="c" |
| repeat region | 13916..14009 /rpt_family="Charlie4a" /rpt_type=dispersed |
| variation | 13995 /gene="IL4R" /frequency="0.01" /replace="a" |
| repeat region | 14011..14243 /rpt_family="AluJb" /rpt_type=dispersed |
| variation | 14029 /gene="IL4R" /frequency="0.03" /replace="a" |
| variation | 14129 /gene="IL4R" /frequency="0.03" /replace="g" |
| variation | 14239..14243 /gene="IL4R" /frequency="0.95" /replace="" |
| repeat region | 14244..14516 /rpt_family="Charlie4a" /rpt_type=dispersed |
| variation | 14368 /gene="IL4R" /frequency="0.36" /replace="a" |
| variation | 14480 /gene="IL4R" /frequency="0.13" /replace="g" |
| repeat region | 14564. .14680 /rpt_family="AluJb" /rpt_type=dispersed |
| variation | 14639 /gene="IL4R" /frequency="0.03" /replace="t" |
| variation | 14680 /gene="IL4R" /frequency="0.04" /replace="t" |
| repeat region | 14686..14827 /rpt_family="Charlie4a" /rpt_type=dispersed |
| repeat region | 14828..14998 /rpt_family="MLT1J" /rpt_type=dispersed |
| variation | 15015 /gene="IL4R" /frequency="0.36" /replace="g" |
| variation | 15030 /gene="IL4R" /frequency="0.05" /replace="g" |
| repeat region | 15069..15435 /rpt_family="MLT1L" /rpt_type=dispersed |
| variation | 15176 /gene="IL4R" /frequency="0.32" /replace="g" |
| variation | 15234 /gene="IL4R" /frequency="0.01" /replace="a" |
| variation | 15242 /gene="IL4R" /frequency="0.01" /replace="t" |
| variation | 15244 /gene="IL4R" /frequency="0.01" /replace="a" |
| variation | 15389 /gene="IL4R" /frequency="0.01" /replace="a" |
| repeat region | 15545..15604 /rpt_family="MIR" /rpt_type=dispersed |
| variation | 15592 /gene="IL4R" /frequency="0.12" /replace="a" |

(continued)

| | |
|---|---|
| variation | 15688 /gene="IL4R" /frequency="0.05" /replace="g" |
| variation | 15708 /gene="IL4R" /frequency="0.01" /replace="a" |
| repeat region | 15719..15819 /rpt_family="L2" /rpt_type=dispersed |
| variation | 15858 /gene="IL4R" /frequency="0.08" /replace="c" |
| repeat region | 15889..16212 /rpt_family=’L2" /rpt_type=dispersed |
| variation | 15960 /gene="IL4R" /frequency="0.01" /replace="a" |
| variation | 16061 /gene="IL4R" /frequency="0.05" /replace="g" |
| variation | 16124 /gene="IL4R" /frequency="0.26" /replace="g" |
| repeat region | 16220..16386 /rpt_family="MIR" /rpt_type=dispersed |
| repeat region | 16428..16594 /rpt_family="L2" /rpt_type=dispersed |
| variation | 16431 /gene="IL4R" /frequency="0.10" /replace="t" |
| variation | 16544 /gene="IL4R" /frequency="0.05" /replace="c" |
| variation | 16656 /gene="IL4R" /frequency="0.01" /replace="g" |
| variation | 16788 /gene="IL4R" /frequency="0.03" /replace="g" |
| misc feature | 16817..16969 /gene="IL4R" /note="Region not scanned for variation" |
| repeat region | 16962..17272 /rpt_family="AluSg" /rpt_type=dispersed |
| variation | 17088 /gene="IL4R" /frequency="0.02" /replace="t" |
| variation | 17134 /gene="IL4R" /frequency="0.01" /replace="t" |
| variation | 17135 /gene="IL4R" /frequency="0.01" /replace="a" |
| variation | 17296 /gene="IL4R" /frequency="0.07" /replace="c" |
| variation | 17298 /gene="IL4R" /frequency="0.08" /replace="" |
| variation | 17387 /gene="IL4R" /frequency="0.26" /replace="c" |
| variation | 17399 /gene="IL4R" /frequency="0.01" /replace="g" |
| repeat region | 17404..17492 /rpt_family="L2" /rpt_type=dispersed |
| variation | 17424 /gene="IL4R" /frequency="0.02" /replace="c" |
| variation | 17468 /gene="IL4R" /frequency="0.01" /replace="t" |
| variation | 17533 /gene="IL4R" /frequency="0.26" /replace="g" |
| variation | 17560 /gene="IL4R" /frequency="0.17" /replace="c" |
| repeat region | 17608..17737 /rpt_family="MIR" /rpt_type=dispersed |
| variation | 17659 /gene="IL4R" /frequency="0.02" /replace="t" |
| repeat region | 17978..18086 /rpt_family="MIR" /rpt_type=dispersed |
| repeat region | 18158..18329 /rpt_family="AluJo" /rpt_type=dispersed |
| variation | 18306 /gene="IL4R" /frequency="0.02" /replace="t" |
| repeat region | 18330..18643 /rpt_family="AluSx" /rpt_type=dispersed |
| variation | 18351 /gene="IL4R" /frequency="0.35" /replace="a" |
| variation | 18394 /gene="IL4R" /frequency="0.36" /replace="g" |
| repeat region | 18644..18707 /rpt_family="AluJo" /rpt_type=dispersed |
| variation | 18665..18666 /gene="IL4R" /frequency="0.11" /replace="" |
| repeat region | 18709..18765 /rpt_family="L2" /rpt_type=dispersed |
| repeat region | 18766..19075 /rpt_family="AluSx" /rpt_type=dispersed |
| variation | 19053 /gene="IL4R" /frequency="0.19" /replace="a" |
| variation | 19056 /gene="IL4R" /frequency="0.01" /replace="a" |
| variation | 19063 /gene="IL4R" /frequency="0.31" /replace="a" |
| repeat region | 19076..19237 /rpt_family="L2" /rpt_type=dispersed |
| variation | 19170 /gene="IL4R" /frequency="0.17" /replace="a" |
| variation | 19300 /gene="IL4R" /frequency="0.01" /replace="g" |
| repeat region | 19532..19671 /rpt_family="MIR" /rpt_type=dispersed |
| variation | 20015 |

(continued)

| | |
|---|---|
| | /gene="IL4R" /frequency="0.17" /replace="a" |
| repeat region | 20210..20332 /rpt_family="MLT1L" /rpt_type=dispersed |
| misc feature | 20330..20467 /gene="IL4R" /note="Region not scanned for variation" |
| repeat region | 20476..20682 /rpt_family="MIR" /rpt_type=dispersed |
| variation | 20497. .20499 /gene="IL4R" /frequency="0.63" /replace="" |
| repeat region | 20689..20815 /rpt_family="MLT1H" /rpt_type=dispersed |
| variation | 20719 /gene="IL4R" /frequency="0.36" /replace="c" |
| variation | 20809 /gene="IL4R" /frequency="0.03" /replace="t" |
| repeat region | 20839..20894 /rpt_family="MLT1H" /rpt_type=dispersed |
| repeat region | 20895..21195 /rpt_family="AluSp" /rpt_type=dispersed |
| variation | 20958 /gene="IL4R" /frequency="0.31" /replace="a" |
| variation | 20981 /gene="IL4R" /frequency="0.02" /replace="g" |
| variation | 20985 /gene="IL4R" /frequency="0.18" /replace="t" |
| variation | 21100 /gene="IL4R" /frequency="0.32" /replace="c" |
| variation | 21132 /gene="IL4R" /frequency="0.39" /replace="g" |
| variation | 21144 /gene="IL4R" /frequency="0.01" /replace="t" |
| variation | 21145 /gene="IL4R" /frequency="0.31" /replace="t" |
| repeat region | 21196..21438 /rpt_family="MLT1H" /rpt_type=dispersed |
| variation | 21510 /gene="IL4R" /frequency="0.24" /replace="a" |
| variation | 21877 /gene="IL4R" /frequency="0.02" /replace="a" |
| variation | 21956 /gene="IL4R" /frequency="0.05" /replace="t" |
| repeat region | 22029..22190 /rpt_family="ERAM" /rpt_type=dispersed |
| variation | 22084 /gene="IL4R" /frequency="0.04" /replace="a" |
| repeat region | 22202..22351 /rpt_family="AluSg" /rpt_type=dispersed |
| variation | 22211 /gene="IL4R" /frequency="0.08" /replace="g" |
| repeat region | 22491. .22799 /rpt_family="AluJo" /rpt_type=dispersed |
| variation | 22652 /gene="IL4R" /frequency="0.04" /replace="" |
| variation | 22690 /gene="IL4R" /frequency="0.21" /replace="a" |
| variation | 22738 /gene="IL4R" /frequency="0.01" /replace="a" |
| variation | 22825 /gene="IL4R" /frequency="0.02" /replace="a" |
| repeat region | 22877..22977 /rpt_family="L2" /rpt_type=dispersed |
| variation | 22889 /gene="IL4R" /frequency="0.01" /replace="c" |
| variation | 23026 /gene="IL4R" /frequency="0.08" /replace="c" |
| variation | 23117 /gene="IL4R" /frequency="0.37" /replace="a" |
| variation | 23171 /gene="IL4R" /frequency="0.02" /replace="c" |
| variation | **23431 /gene="IL4R" /frequency="0.30" /replace="c"** |
| variation | 23474 /gene="IL4R" /frequency="0.30" /replace="t" |
| variation | 23523 /gene="IL4R" /frequency="0.11" /replace="c" |
| variation | **23525 /gene="IL4R" /frequency="0.02" /replace="t"** |
| variation | 23531 /gene="IL4R" /frequency="0.31" /replace="c" |
| **variation** | **23539 /gene="IL4R" /frequency="0.04" /replace="t"** |
| **variation** | **23739 /gene="IL4R" /frequency="0.26" /replace="c"** |
| **variation** | **23959 /gene="IL4R" /frequency="0.43" /replace="g"** |
| variation | 23967 /gene="IL4R" /frequency="0.06" /replace="a" |
| variation | 24255 /gene="IL4R" /frequency="0.02" /replace="t" |
| variation | 24486 /gene="IL4R" /frequency="0.14" /replace="g" |
| variation | 24629 /gene="IL4R" /frequency="0.06" /replace="c" |
| variation | 24716 /gene="IL4R" /frequency="0.09" /replace="c" |

(continued)

| | |
|---|---|
| variation | 24718 /gene="IL4R" /frequency="0.08" /replace="c" |
| variation | 24759 /gene="IL4R" /frequency="0.02" /replace="t" |
| variation | 24798 /gene="IL4R" /frequency="0.04" /replace="g" |
| variation | 24982 /gene="IL4R" /frequency="0.01" /replace="a" |
| variation | 24992 /gene="IL4R" /frequency="0.01" /replace="a" |
| variation | 25101 /gene="IL4R" /frequency="0.38" /replace="a" |
| variation | 25346 /gene="IL4R" /frequency="0.39" /replace="g" |
| variation | 25448 /gene="IL4R" /frequency="0.21" /replace="t" |
| variation | 25776 /frequency="0.39" /replace="g" |
| repeat region | 25777..25890 /rpt_family="LTR16C" /rpt_type=dispersed |
| repeat region | 25891..26433 /rpt_family="MER41A" /rpt_type=dispersed |
| variation | 26042 /frequency="0.01" /replace="c" |
| variation | 26196 /frequency="0.30" /replace="g" |
| repeat region | 26434..26550 /rpt_family="LTR16C" /rpt_type=dispersed |
| variation | 26457 /frequency="0.01" /replace="a" |
| variation | 26469 /frequency="0.40" /replace="t" |

Patricularly relevant isoforms/variants/variations are indicated herein above in bold print.

The above depicted SEQ ID NO: 3 encodes for a polypeptide, comprising the herein described "I50V" (or "I75V") SNP/variant. Said encoded polypeptide is shown in SEQ ID NO: 4, comprising the "V" at position 75 of the IL4R/CD124 polypeptide with a signal peptide of 25 amino acids (resulting in the "I75V" denomination).

**MGWLCSGLLFPVSCLVLLQVASSGNMKVLQEPTCVSDYMSISTCEWKMNGPTNCST**
**ELRLLYQLVFLLSEAHTCVPENNGGAGCVCHLLMDDVVSADNYTLDLWAGQQLLW**
**KGSFKPSEHVKPRAPGNLTVHTNVSDTLLLTWSNPYPPDNYLYNHLTYAVNIWSEND**
**PADFRIYNVTYLEPSLRIAASTLKSGISYRARVRAWAQCYNTTWSEWSPSTKWHNSY**
**REPFEQHLLLGVSVSCIVILAVCLLCYVSITKIKKEWWDQIPNPARSRLVAIIIQDAQGS**
**QWEKRSRGQEPAKCPHWKNCLTKLLPCFLEHNMKRDEDPHKAAKEMPFQGSGKSA**
**WCPVEISKTVLWPESISVVRCVELFEAPVECEEEEEVEEEKGSFCASPESSRDDFQEGR**
**EGIVARLTESLFLDLLGEENGGFCQQDMGESCLLPPSGSTSAHMPWDEFPSAGPKEAP**
**PWGKEQPLHLEPSPPASPTQSPDNLTCTETPLVIAGNPAYRSFSNSLSQSPCPRELGPDP**
**LLARHLEEVEPEMPCVPQLSEPTTVPQPEPETWEQILRRNVLQHGAAAAPVSAPTSGY**
**QEFVHAVEQGGTQASAVVGLGPPGEAGYKAFSSLLASSAVSPEKCGFGASSGEEGYK**
**PFQDLIPGCPGDPAPVPVPLFTFGLDREPPRSPQSSHLPSSSPEHLGLEPGEKVEDMPKP**
**PLPQEQATDPLVDSLGSGIVYSALTCHLCGHLKQCHGQEDGGQTPVMASPCCGCCCG**
**DRSSPPTTPLRAPDPSPGGVPLEASLCPASLAPSGISEKSKSSSSFHPAPGNAQSSSQTP**
**KIVNFVSVGPTYMRVS (SEQ ID NO: 4)**

The present invention provides for a method of diagnosing and/or predicting early joint destruction in rheumatoid arthritis, rapidly erosive rheumatoid arthritis and/or accelerated joint destruction in rheumatoid arthritis, said method comprising determining in a sample obtained from an individual independent of HLA-DR shared epitope (SE) the presence of an encoded IL-4 receptor (IL-4) which comprises at the homologous position 75 of IL-4 receptor as depicted in SEQ ID NO: 2 a mutation, said mutation comprising the exchange from an isoleucine to a valine. As mentioned above, it is evident for the skilled artisan that also the determination of the wild-type ("T") allele has predictive and/or diagnostic character, i.e. is of diagnostic relevance. Is, e.g. the wild-type allel (I50/I75) determined in a human patient, i.e. a patient suffering from rheumatoid arthritis (or a related disorder), is determined to have on position 50 or 75 of the IL4R as described herein the wild-type allele(s) I, it can be assumed that the disorder is of less erosive character or even non-erosive character in terms of joint destruction. In such a case, a less aggressive and/or moderate treatment/medical intervention is indicated. In contrast, in cases wherein the human individual/human patient has at least one mutant allele on position 50/75 of IL4R (i.e. at least one "V" allele) a more aggressive therapy is indicated since this mutant allele is the predictor for an erosive development and/or character of joint destruction. Accordingly, the marker provided herein is a predictor for an erosive course of (rheumatoid) arthritis versus a non-ersosive course. The corresponding genetic disposition of an individual can, accordingly, influence the medical intervention, i.e. aggressive versus non- or less aggressive (e.g. DMARDs-treatment versus NSAIDs-treatment).

It will also be understood that further mutations might occur within the IL4R-gene, which as such may correlate with the presence of the mutation described herein. However, the appended examples also document that the herein defined "I50V" / "I75V" variant of IL4R (CD124) is solely indicative for the herein described early joint destruction, accelerated joint destruction and/or rapidly erosive rheumatoid arthritis. Particularly, it is envisaged that e.g. future SNPs or modification are detected within the IL4R-gene which are also correlated with the presence of the "I50V"/ "I75V" variant as described herein.

The methods described herein, i.e. the methods of diagnosing and/or predicting early joint destruction, accelerated joint destruction, rapid/rapidly erosive rheumatoid arthritis etc. may comprise PCR, MALDI-TOF, ligase chain reaction, NASBA, restriction digestion, direct sequencing, nucleic acid amplification techniques, hybridization techniques or immunoassays. Also further techniques are envisaged. In accordance with this embodiment of the present invention, the diagnosis of a rapid erosive rheumatoid disorder can, e.g., be effected by isolating cells from an individual, and isolating the genomic DNA of said cells. Such cells can be collected from body fluids, skin, hair, biopsies and other sources. As documented in the appended examples, in particular peripheral blood is useful. Collection and analysis of cells from bodily fluids such as blood, urine and cerebrospinal fluid is well known to the art; see for example, Rodak, "Haematology: Clinical Principles & Applications" second ed., WB Saunders Co, 2002; Brunzel, "Fundamentals of Urine and Body Fluids Analysis", WB Saunders Co, 1994; Herndon and Brumback (Ed.), "Cerebrospinal Fluid", Kluwer Academic Pub., 1989. In addition, methods for DNA isolation are well described in the art; see, for example, Sambrook et al., "Molecular Cloning: A Laboratory Manual", 3rd edition, Cold Spring Harbor Laboratory, 2001.

Once DNA has been isolated, various oligonucleotide primers spanning the variant (I50V) coding region of IL4R (CD124) as described herein locus may be designed in order to amplify the genetic material by Polymerase Chain Reaction (PCR). Conventional methods for designing, synthesizing, producing said oligonucleotide primers and performing PCR amplification may be found in standard textbooks, see, for example Agrawal (Ed.), "Protocols for Oligonucleotides and Analogs: Synthesis and Properties (Methods in Molecular Biology, 20)", Humana Press, 1993; Innis et al. (Ed.), "PCR Applications: Protocols for Functional Genomics", Academic Press, 1999; Chen and Janes (Ed.), "PCR Cloning Protocols: From Molecular Cloning to Genetic", 2nd edition, Humana Press, 2002. Primers for the detection of IL4R variation(s) is/are also given in the appended examples. Once DNA has been amplified, nucleotide structure can be analysed by sequencing methods and compared to normal IL4R (CD124) DNA, i.e. comprising a codon encoding for "isoleucine" at position 75 of SEQ ID NO: 2. Sequencing may be performed manually by any molecular biologist of ordinary skills or by an automated sequencing apparatus. These procedures are common in the art, see, for example, Adams et al. (Ed.), "Automated DNA Sequencing and Analysis", Academic Press, 1994; Alphey, "DNA Sequencing: From Experimental Methods to Bioinformatics", Springer Verlag Publishing, 1997. Also RNA analysis may be employed in order to detect the presence or absence of the herein described I50V/I75V variant of IL4R (CD124). Also RT-PCR methods and techniques may be employed in accordance with this invention.

Detection and analysis of variations, in particular the variant described herein, in IL4R/CD124 encoding nucleic acid sequences can also be performed using amplification refractory mutation system (ARMSTM), amplification refractory mutation system linear extension (ALEXTM), single-strand conformation polymorphism (SSCP), heteroduplex analysis, PCR-SSCP, fluorescent SSCP in an automated DNA sequencer, denaturing gradient gel electrophoresis, RNase protection assays, detection of mutations by sequence specific oligonucleotide hybridization, chemical cleavage methods, enzyme mismatch cleavage methods, cleavage fragment length methods, allele-specific oligonucleotide hybridization on DNA chips or wafers or arrays, and other such methods known in the art, see, for example Nollau et al, Clin. Chem. 43 (1997), 1114-1128; Burczak and Mardis (Ed.), "Polymorphism Detection & Analysis Techniques", Eaton Pub Co, 2000; Cotton et al. (Ed.), "Mutation Detection: A Practical Approach", Irl Press, 1998; Taylor (Ed.), "Laboratory Methods for the Detection of Mutations and Polymorphisms in DNA", CRC Press, 1997.

Therefore, the person skilled in the art is readily in a postion to work the present invention over the full scope. SNPs and single point mutations, in particular the mutation (I50V/I75V) described herein, can, inter alia, also be detected using various methods, like the analysis of restriction fragment length polymorphism and mobility shift of single-stranded DNAs (Botstein, 1980 Am J Hum Genet 32: 314-31; Orita, 1989 PNAS 86: 2766-2770). Furthermore, PCR related methods or methods used in genotyping studies including automated DNA sequencing may provide important information about the occurrence of SNP's and single point mutations (Saiki, 1989 PNAS 86: 6230-6234; Verpy, 1994 PNAS 91: 1873-1877). Corresponding techniques are also shown in the appended examples.

Alternatively, fluorescently labelled allele- or SNP-specific hybridization probes are methods which allow rapid and reliable detection of DNA point mutations. Typical hybridization probes may comprise a short recognition sequence and two fluorophores whose spatial separation is governed by strand-specific hybridization. One unit serves as the reporting element and is modulated (turned on/off) by the proximity of the second by using Fluorescence resonance energy transfer (FRET) technology. Non-limiting examples include stem-loop molecular beacons and TaqMan-type probes, which are of current interest in real-time SNP screening (Bonnet, 1999 PNAS 96: 6171-6176; Marras, 1999 Genet Anal 14: 151-156; Livak, 1999 Genet Anal 14: 143-149; Gaylord, 2005 PNAS 102: 34-39). High-throughput screening at the nucleotide level for previously identified point mutations may be accomplished by using genotyping chips (Wang, 1998 Science 280: 1077-1082).

On the protein level, point mutations may be detected by using mass spectrometry techniques such as electrospray ionization-time of flight (ESI-TOF) or matrix-assisted laser desorption/ionization-time of flight (MALDI-TOF) analysis (Taranenko,1996 Genet Anal 13: 87-94; Ball, 1998 Protein Sci 7: 758-764). It is also envisaged that antibody-based techniques be employed, like Western-blot analysis, ELISA, RIA, IRMA and the like. These techniques may employ specific antibodies directed against the mutant "V" or the wild-type "I" variant of IL-4R. The gereration of antibodies is well-known in the art, see, inter alia, Harlow & Lane, "Antibodies: A Laboratory Manual" (1988) Cold Spring Harbor Laboratory Press.In accordance with the present invention, the term "nucleic acid sequence" means the sequence of bases comprising purine- and pyrimidine bases which are comprised by nucleic acid molecules, whereby said bases represent the primary structure of a nucleic acid molecule. Nucleic acid sequences include DNA, cDNA (e.g. obtained from spliced and/or unspliced mRNA), genomic DNA, RNA, synthetic forms and mixed polymers, both sense and antisense strands, or may contain non-natural or derivatized nucleotide bases, as will be readily appreciated by those skilled in the art.

In the context of the present invention the term "molecular variant"/"molecular isoform" IL4R/CD124 gene or protein as used herein means that said IL4R (CD124) gene or protein differs from the wild type IL4R (CD124) gene or protein by at least one nucleotide or amino acid substitution, deletion and/or addition. Variations and isoforms of IL4R are also provided above in context of SEQ ID NO. 3.The cDNA sequences of the wildtype ILAR gene as used herein is shown in SEQ ID NO: 1, and the wild-type IL4R amino acid sequence is shown in SEQ ID NO: 2. It will be understood that the methods of the present invention include that instead of determining the presence of a nucleotide sequence having the mutation described herein it is, alternatively, also possible to detect the presence of the encoded variant polypeptide of the described IL4R variant (namely the exchange from an isoleucine to a valine at position 75 of the IL4R as shown in SEQ ID NO : 2 or a similar or homologous substitution in a homologous variant (allelic variant) of the ILR as shown in SEQ ID NO: 2). As pointed out above, also the determination of the wild-type allel (I50/I75 and their corresponding encoding nucleotides in the coding nucleotide sequence) is of high diagnostic relevance since it allows for the determination of a less or even non-erosive character of the disease, i.e. in RA.

A nucleotide sequence of human IL4R/CD124 is disclosed in NCBI nucleotide entry NCBI NM_000418 (gi: 56788409). Under the accession number AF421855 the complete cds of the IL4R gene is provided and specific variations, inter alia, the herein defined I50V/I75V variation is described. However, in the context of the present invention, the sequence as depicted in SEQ ID No:1 is referred to as the "wildtype" sequence.

When used herein, the term "polypeptide" means a peptide, a protein, or a polypeptide which encompasses amino acid chains of a given length, wherein the amino acid residues are linked by covalent peptide bonds. However, peptidomimetics of such proteins/polypeptides wherein amino acid(s) and/or peptide bond(s) have been replaced by functional analogs are also encompassed by the invention as well as other than the 20 gene-encoded amino acids, such as selenocysteine. Peptides, oligopeptides and proteins may be termed polypeptides. The terms polypeptide and protein are often used interchangeably herein. The term polypeptide also refers to, and does not exclude, modifications of the polypeptide, e.g., glycosylation, acetylation, phosphorylation and the like. Such modifications are well described in basic texts and in more detailed monographs, as well as in a voluminous research literature.

When used in the context of the present invention the term "a predisposition for a disease" in particular the early joint destruction and/or rapidly erosive rheumatoid arthrits includes a tendency or susceptibility to develop a certain disease that can be triggered under certain conditions, e.g. influence of the environment, personal circumstances of a subject, such as the nutritional behavior of a subject. The tendency or susceptibility to develop a certain disease is associated with a genotype that increases the risk for developing a disease, if other certain conditions, such as those mentioned above, are present. Genetic testing is able to identify subjects who are genetically predisposed to certain health problems as defined herein. The disease for which a subject has a tendency or susceptibility to develop is preferably a disease related to the presence of a molecular variant of IL4R/CD124 as described herein and comprises early joint destruction, accelerated joint destruction and/or rapidly erosive rheumatoid arthritis.

The term "position" when used in accordance with the present invention means the position of either an amino acid within an amino acid sequence depicted herein or the position of a nucleotide within a nucleic acid sequence depicted herein. The term "corresponding" as used herein also includes that a position is not only determined by the number of the preceding nucleotides/amino acids. The exact position of a given nucleotide in accordance with the present invention which may be substituted may vary due to deletions or additional nucleotides elsewhere in the IL4R/CD124 promoter or gene. Thus, under a "corresponding position" in accordance with the present invention it is to be understood that nucleotides may differ in the indicated number (for example position "75" of the full IL4R (with signal sequence) or position 50 of the mature protein (without signal sequence) or position 465 of the nucleotide sequence representing the cDNA nucleotide sequence of a wildtype IL4R as shown in SEQ ID NO: 1 but may still have similar neighboring nucleotides. Said nucleotides which may be exchanged, deleted or comprise additional nucleotides are also comprised by the term "corresponding position".

The position with respect to nucleotide sequences mentioned herein refers to the sequence shown in SEQ ID NO: 1. This sequence represents the cDNA nucleic acid sequence of the IL4R gene encoding the interleukin 4 receptor (CD124). It is possible for the skilled person to identify the position in the cDNA sequence corresponding to a position in SEQ ID NO: 1 by aligning the sequences.

In order to determine whether an amino acid residue or nucleotide residue in a given IL4R sequence corresponds to a certain position in the amino acid sequence or nucleotide sequence of SEQ ID NO: 1 or 2, the skilled person can use means and methods well-known in the art, e.g., alignments, either manually or by using computer programs such as those mentioned further down below in connection with the definition of the term "hybridization" and degrees of homology.

For example, BLAST2.0, which stands for Basic Local Alignment Search Tool (Altschul, Nucl. Acids Res. 25 (1997), 3389-3402; Altschul, J. Mol. Evol. 36 (1993), 290-300; Altschul, J. Mol. Biol. 215 (1990), 403-410), can be used to search for local sequence alignments. BLAST produces alignments of both nucleotide and amino acid sequences to determine sequence similarity. Because of the local nature of the alignments, BLAST is especially useful in determining exact matches or in identifying similar sequences. The fundamental unit of BLAST algorithm output is the High-scoring Segment Pair (HSP). An HSP consists of two sequence fragments of arbitrary but equal lengths whose alignment is locally maximal and for which the alignment score meets or exceeds a threshold or cutoff score set by the user. The BLAST approach is to look for HSPs between a query sequence and a database sequence, to evaluate the statistical significance of any matches found, and to report only those matches which satisfy the user-selected threshold of significance. The parameter E establishes the statistically significant threshold for reporting database sequence matches. E is interpreted as the upper bound of the expected frequency of chance occurrence of an HSP (or set of HSPs) within the context of the entire database search. Any database sequence whose match satisfies E is reported in the program output.

Analogous computer techniques using BLAST (Altschul (1997), loc. cit.; Altschul (1993), loc. cit.; Altschul (1990), loc. cit.) are used to search for identical or related molecules in nucleotide databases such as GenBank or EMBL. This analysis is much faster than multiple membrane-based hybridizations. In addition, the sensitivity of the computer search can be modified to determine whether any particular match is categorized as exact or similar. The basis of the search is the product score which is defined as:

$$\frac{\%\text{sequence identity} \times \%\text{ maximum BLAST score}}{100}$$

and it takes into account both the degree of similarity between two sequences and the length of the sequence match. For example, with a product score of 40, the match will be exact within a 1-2% error; and at 70, the match will be exact. Similar molecules are usually identified by selecting those which show product scores between 15 and 40, although lower scores may identify related molecules. Another example for a program capable of generating sequence alignments is the CLUSTALW computer program (Thompson, Nucl. Acids Res. 2 (1994), 4673-4680) or FASTDB (Brutlag Comp. App. Biosci. 6 (1990), 237-245), as known in the art.

As described herein, the present invention describes a specific informative variant of one IL4R gene which means that at a certain position (465 of the full cDNA sequence as shown in SEQ ID NO: 1) of the wild-type IL4R/CD124 nucleotide sequence a nucleotide is replaced by a different nucleotide as described herein. The term "IL4R gene" or "CD 124 gene" includes the 5'UTR, exons, introns and the 3'UTR of IL4R/CD124 whose cDNA wild-type nucleotide sequences is shown in SEQ ID NO: 1. The term "is replaced by a different nucleotide" when used in the context of the present invention means that the nucleotide residing at a position of the wild-type IL4R nucleic acid sequences mentioned herein is replaced by a nucleotide which is not the same nucleotide as that residing on the said position of the wild-type IL4R nucleic acid sequences.

The present invention also relates to the use of nucleic acid molecules which hybridize to one of the above described nucleic acid molecules and which shows a mutation as described hereinabove in the methods and means provided herein.

The term "hybridizes" as used in accordance with the present invention may relate to hybridizations under stringent or non-stringent conditions. If not further specified, the conditions are preferably non-stringent. Said hybridization conditions may be established according to conventional protocols described, for example, in Sambrook, Russell "Molecular Cloning, A Laboratory Manual", Cold Spring Harbor Laboratory, N.Y. (2001); Ausubel, "Current Protocols in Molecular Biology", Green Publishing Associates and Wiley Interscience, N.Y. (1989), or Higgins and Hames (Eds.) "Nucleic acid hybridization, a practical approach" IRL Press Oxford, Washington DC, (1985). The setting of conditions is well within the skill of the artisan and can be determined according to protocols described in the art. Thus, the detection of only specifically hybridizing sequences will usually require stringent hybridization and washing conditions such as 0.1xSSC, 0.1% SDS at 65°C. Non-stringent hybridization conditions for the detection of homologous or not exactly complementary sequences may be set at 6xSSC, 1% SDS at 65°C. As is well known, the length of the probe and the composition of the nucleic acid to be determined constitute further parameters of the hybridization conditions. Note that variations in the above conditions may be accomplished through the inclusion and/or substitution of alternate blocking reagents used to suppress background in hybridization experiments. Typical blocking reagents include Denhardt's reagent, BLOTTO, heparin, denatured salmon sperm DNA, and commercially available proprietary formulations. The inclusion of specific blocking reagents may require modification of the hybridization conditions described above, due to problems with compatibility. Hybridizing nucleic acid molecules also comprise fragments of the above described molecules. Such fragments may represent nucleic acid sequences which code for a IL-4 receptor molecule (CD124) fragment thereof, and which have a length of at least 12 nucleotides, preferably at least 15, more preferably at least 18, more preferably of at least 21 nucleotides, more preferably at least 30 nucleotides, even more preferably at least 40 nucleotides and most preferably at least 60 nucleotides and which are capable to hybridize to a region to be amplified by recombinant techniques, said region comprising a mutation in position 465 as defined herein in context of SEQ ID NO: 1 (the cDNA wild-type IL4R sequence) and said position to be amplified or said region whereto these fragments hybridize to comprise the herein defined diagnostic mutation. Also indicative is the case where the herein defined mutation is not detected, i.e. wherein no early joint destruction/rapid (rapidly) erosive rheumatoid arthritis and/or accelerated joint destruction is to be detected, diagnosed and/or predicted.

Hybridizing sequences in accordance with this invention may be specific primers and/or probes useful in the detection of the herein described variant/mutation of the IL4R (CD124) "I50V/"I75V". Furthermore, nucleic acid molecules which hybridize with any of the aforementioned nucleic acid molecules also include complementary fragments, derivatives and allelic variants of these molecules. Additionally, a hybridization complex refers to a complex between two nucleic acid sequences by virtue of the formation of hydrogen bonds between complementary G and C bases and between complementary A and T bases; these hydrogen bonds may be further stabilized by base stacking interactions. The two complementary nucleic acid sequences hydrogen bond in an antiparallel configuration. A hybridization complex may be formed in solution (e.g., Cot or Rot analysis) or between one nucleic acid sequence present in solution and another nucleic acid sequence immobilized on a solid support (e.g., membranes, filters, chips, pins or glass slides to which, e.g., cells have been fixed). The terms complementary or complementarity refer to the natural binding of polynucleotides under permissive salt and temperature conditions by base-pairing. For example, the sequence "A-G-T" binds to the complementary sequence "T-C-A". Complementarity between two single-stranded molecules may be "partial", in which only some of the nucleic acids bind, or it may be complete when total complementarity exists between single-stranded molecules. The degree of complementartity between nucleic acid strands has significant effects on the efficiency and strength of hybridization between nucleic acid strands. This is of particular importance in amplification reactions, which depend upon binding between nucleic acids strands.

The term "hybridizing sequences" preferably refers to sequences which display a sequence identity of at least 40%, preferably at least 50%, more preferably at least 60%, even more preferably at least 70%, particularly preferred at least 80%, more particularly preferred at least 90%, even more particularly preferred at least 95% and most preferably at least 97% identity with a nucleic acid sequence as described above encoding an IL4R (CD124) protein having a described mutation. Moreover, the term "hybridizing sequences" preferably refers to sequences encoding an IL4R (CD124) protein having a sequence identity of at least 40%, preferably at least 50%, more preferably at least 60%, even more preferably at least 70%, particularly preferred at least 80%, more particularly preferred at least 90%, even more particularly preferred at least 95% and most preferably at least 97% identity with an amino acid sequence of an IL4R (CD 124) variant comprising the herein described mutation/variation isoleucine to valine as described herein above.

In accordance with the present invention, the term "identical" or "percent identity" in the context of two or more nucleic acid or amino acid sequences, refers to two or more sequences or subsequences that are the same, or that have a specified percentage of amino acid residues or nucleotides that are the same (e.g., 60% or 65% identity, preferably, 70-95% identity, more preferably at least 95% identity), when compared and aligned for maximum correspondence over a window of comparison, or over a designated region as measured using a sequence comparison algorithm as known in the art, or by manual alignment and visual inspection. Sequences having, for example, 60% to 95% or greater sequence identity are considered to be substantially identical. Such a definition also applies to the complement of a test sequence. Preferably the described identity exists over a region that is at least about 15 to 25 amino acids or nucleotides in length, more preferably, over a region that is about 50 to 100 amino acids or nucleotides in length. Those having skill in the art will know how to determine percent identity between/among sequences using, for example, algorithms such as those based on CLUSTALW computer program (Thompson Nucl. Acids Res. 2 (1994), 4673-4680) or FASTDB (Brutlag Comp. App. Biosci. 6 (1990), 237-245), as known in the art.

Although the FASTDB algorithm typically does not consider internal non-matching deletions or additions in sequences, i.e., gaps, in its calculation, this can be corrected manually to avoid an overestimation of the % identity. CLUSTALW, however, does take sequence gaps into account in its identity calculations. Also available to those having skill in this art are the BLAST and BLAST 2.0 algorithms (Altschul Nucl. Acids Res. 25 (1977), 3389-3402). The BLASTN program for nucleic acid sequences uses as defaults a word length (W) of 11, an expectation (E) of 10, M=5, N=4, and a comparison of both strands. For amino acid sequences, the BLASTP program uses as defaults a wordlength (W) of 3, and an expectation (E) of 10. The BLOSUM62 scoring matrix (Henikoff Proc. Natl. Acad. Sci., USA, 89, (1989), 10915) uses alignments (B) of 50, expectation (E) of 10, M=5, N=4, and a comparison of both strands.

Moreover, the present invention also relates to the use of nucleic acid molecules the sequence of which is degenerate in comparison with the sequence of an above-described hybridizing molecule. When used in accordance with the present invention the term "being degenerate as a result of the genetic code" means that due to the redundancy of the genetic code different nucleotide sequences code for the same amino acid. Also these nucleic acid molecules are useful in the methods and means (like diagnostic kits) provided herein.

In another embodiment of the methods described herein, the "I50V"/"I75V" SNP or point mutation is detected in homozygous alleles/homozygosity, in particular said mutation/SNP is detected in a sample from a homozygous individual. As discussed herein, the detection in a homozygous individual is advantageous, however, also the detection of the I50V/I75V SNP/variant in a heterozygous subject or individual is conclusive and may successfully be employed in the methods and means of the invention. However, as pointed out above, also the determination of the wild-type allel(s) (I50/I75) is/are of high diagnostic value, since it allows for the prediction whether the disases is of less (or even non-) erosive character. Individuals which are diagnosed in accordance with this invention to be bearer of at least one mutant allel as described herein (i.e. V50/V75 of IL4R) will suffer from a more erosive disease and more aggressive medical intervention is indicated in these patients. A more aggressive medical intervention in this erosive joint destruction is e.g. anti-TNF treatment, anti-IL-15 treatment, anti-IL-6 treatment, treatment with CTLA-4Ig, depletion of B-cells (like with antibodies directed against CD20 or CD22; see e.g. Rituximab ®), or the "DMARD-therapy".

Some patients with rheumatoid arthritis (RA) are more likely than others to show joint damage within the first 2 years of disease onset (early onset and early joint destruction). Some studies have shown that patients who have active RA in multiple joints and those who show high levels of certain antibodies based on blood tests have a 70% chance of developing joint damage within this early time period. The present invention provides for a reliable test in particular for these subjects/patients, since it is now possible to scrutinize now at an early point of time whether a given patient will suffer from erosive, early joint destruction or whether the diseases is less or even non-erosive, in case he/she is "wild-type" for the I50/I75 allel of IL4R as described herein.

In the context of the present invention, the term "subject" means an individual in need of a treatment of an affective disorder. Preferably, the subject is a mammalian, particularly preferred a human, a horse, a camel, a dog, a cat, a pig, a cow, a goat or a fowl. The present invention is particularly useful in the determination whether a given or suspected RA of a human patient is erosive or non-erosive or whether a joint destruction is to be expected early on. If this is the case, since the human patient is either homo-or heterozygous for the herin desceibd point mutation (V50/V75 of IL4R), an aggressive medical intervention is indicated.

As discussed herein and shown in the experimental part, the present invention provides for the prediction and/or diagnosis of erosive joint destruction, in particular an early joint destruction. Said joint destruction is in particular associated with early erosion(s) and/or a higher severity in the progression of rheumatoid arthritis (RA). The means and methods provided herein are particularly useful, since a positive result, i.e. the presence of the I50V/I75V IL4R SNP/mutation will ideally prompt an early and more aggressive therapeutic intervention in the positively screened/scored individuals. As pointed out herein, is a given subject patient diagnosed as comprising the herein defined "wild-type" allel (I50/I75 of IL4R), a more moderate or less aggressive therapy is indicated. Early prognosis of an early or an erosive joint destruction may prevent further damage, like bone destruction. "Aggressive treatment" that has been shown to prevent joint damage includes treatment with substances to inhibit the activity of TNF, treatment with substances to inhibit the activity of IL-1, treatment with substances to inhibit T cell costimulation (such as neutralizing antibodies to CD28 or fusion proteins such as CTLA-4Ig or derivatives thereof), treatment with substances to inhibit the activity of IL-6, treatment with substances to inhibit the activity of IL-15, treatment with substances to inhibit the activity of IL-17 or treatment with substances to deplete B cells (such as antibodies antibodies to CD20 or CD22, like Rituximab®, Rituxan® and MabThera®, Epratuzumab® and the like). Aggressive treatment also refers to any combination of two or more so called "disease modifying anti-rheumatic drugs (DMARDs)", such as methotrexate, cyclosporine, azathioprine, sulfasalazine, mycophenolate mofetil, chloroquirie, hydroxychloroquine, gold, D-penicillamine, bucillamine, cyclophosphamide and leflunomide. In particular, when the herein described mutant "I50V"/"I75V" SNP (mutant allel V54/V75 of IL4R) is detected in a homozygous setting, the treatment of choice would preferably be an "aggressive treatment" as described above. Particularly, patients who may show signs of bone and/or joint destruction but who appear to be homozygously "wild-type" at position 50 or 75 of the IL4R α-chain as described herein (i.e. "I50V"/ "I75V") may be treated with commonly used non-steroidal anti-inflammatory drugs/NSAIDs, in particular during an early phase of the disease. However, it is of note that also patients with a "wild-type" genetic setting of IL4R on position I50/I75 of Il4R as described herein, but already suffering from bone erosions should be treated aggressivly.

Yet, such homozygous "wild-type" (I50/I75) subject or patients who are diagnosed early on (for example during their first visit at the attending physician or rheumatologist) as suffering from RA can be treated less aggressivly, for example with non-steroidal anti-inflammatory drugs/NSAIDs such as acetylsalicylic acid, indomethazine, acemathazine, proglumethazine, mefenaminic acid, diclofenac, aceclofenac, lonacolac, ibuprofen, ketoprofen, naproxene, flurbiprofen, tiaprofenic acid, piroxicam, tenoxicam, meloxicam, lomoxicam, celecoxib, etoricoxib, lumiracoxib, phenylbutazone, mofebutazone or solely with (low-dose) corticosteroids. Accordingly, with the teaching of the present invention, different potential treatments may be offered to different patients, in particular potential arthritis patients, depending on the allelic setting in position 50 (75) of the amino acid sequence of IL4R. Wild-type individuals ("1501"/ "I75I" in the IL4R SNP, e.g. "I" on the herein defined position 50 or 75 of alpha-chain IL4R protein sequence;) showing no signs of joint and/or bone destruction may be treated with, inter alia, NSAIDs and/or low dose steroids, heterozygous patients (comprising one "150V"/ "I75V" allele/SNP in IL4R) may be treated with one DMARD, low dose steroids or NSAIDs (and should be frequently controlled by radiography) and homozygous patients (comprising the "I50V"/"I75V" SNP in both IL4R alleles) should be treated with "aggressive therapy" with biologicals as identified above, e.g. TNF neutralizing agents and/or a combination of two or more DMARDs in order to prevent bone erosions.

Without being bound by theory, the following treatment regime is envisaged as a non-limiting example. The determination of the allels at the IL4R I50V locus can be used for a stratification of the therapy in patients with recent onset rheumatoid arthritis. If a patient with recent onset rheumatoid arthritis has not developed bone erosions at the time of presentation, a patient with homozygosity for the wildtype allel (I50I/I75I) could be treated conservatively with low dose corticosteroids (for example and non-limiting :< 10 mg/d) and NSAIDS, but without DMARDs or biologics. The expected mild course of the disease would also merit control visits in intervals of six months durings the first two years of the disease. A patient with a heterozygosity at the IL4R I50V/I75V locus (i.e. one "mutant allel V50/V75) would require more aggresive treatment regimens, such as medium doses of corticosteroids at the beginning (for example and non-limiting: 20 mg/d initially and a reduction to 10 mg over a period of six weeks), NSAIDS and one DMARD, such as methotrexate. The follow-up visits would be scheduled in three to six months intervals. In case of homozygosity for the mutant allele (V50V/V75V) the patients should be started with most aggressive therapy, such as high dose steroids (for example and non-limiting: 50 mg/d with a reduction to 10 mg/d over a period of 6 weeks), a DMARD such as methotrexate or biologics (such as TNF inhibitors). Follow-up visit should be scheduled every six weeks to three months, and any indication of persistently active disease should result in the decision to add a second DMARD or to switch to a biologic, like Rituximab or Abatacept.

As pointed out above, the method of detecting the herein described I50V/I75V SNP of IL4R may comprise detection on the protein as well as the nucleic acid molecule level and common methods comprise PCR-technology, ligase-chain reaction, NASBA, restriction digestion, direct sequencing, nucleic acid amplification techniques, MALDI, MALDI-TOF, hybridization techniques and/or immuno assays. Accordingly, in e.g. immunoassays specific antibodies and/or binding molecules directed against an epitope, comprising the defined "V" in context of the surrounding amino acids may be employed. Therefore, preferably binding molecules and/or antibodies detecting an epitope comprise, e.g. the amino acid stretch SEAHTCVPENN or a part thereof may be employed. The specific antibody or binding molecule may also comprise a binding molecule that specifically detects a conformational and/or split epitope which is present in the I50V variant polypeptide described herein and which is not present in a wild-type IL4R α-chain or a homologous IL4R α-chain which does not comprise the "I50V"/ "I75V" SNP/variant. Such a binding molecule may also be a specific aptamer and the like.

Antibodies to be employed in the present methods of the invention are not limited to polyclonal or monoclonal antibodies or antibodies derived from immunization of e.g. corresponding (laboratory) animals. The term "antibody" also relates to modified antibodies (like humanized antibodies, CDR-grafted antibodies, single chain antibodies) or antibody fragments, like F(ab) fragments and the like.

Therefore, in context of the present invention, the term "antibody molecule" or "antibody" relates to full immunoglobulin molecules, preferably IgMs, IgDs, IgEs, IgAs or IgGs, more preferably IgG1, IgG2, IgG2a, IgG2b, IgG3 or IgG4 as well as to parts of such immunoglobulin molecules, like Fab-fragments or $V_L$-, $V_H$- or CDR-regions. Furthermore, the term relates to modified and/or altered antibody molecules, like chimeric, CDR-grafted and humanized antibodies. The term also relates to modified or altered monoclonal or polyclonal antibodies as well as to recombinantly or synthetically generated/synthesized antibodies. The term also relates to intact antibodies as well as to antibody fragments/parts thereof, like, separated light and heavy chains, Fab, Fab/c, Fv, Fab', $F(ab')_2$. The term "antibody molecule" also comprises antibody derivatives, the bifunctional antibodies and antibody constructs, like single chain Fvs (scFv) or antibody-fusion proteins.

The invention also relates to the use of specific probes and/or primers, specific antibody molecules or specific binding molecules (like aptamers) in the preparation of a diagnostic composition for diagnosing and/or predicting, early joint destruction and/or accelerated joint destruction in rheumatoid arthritis independent of HLA-DR shared epitope (SE) and/or the erosive character of the disease, said joint destruction may be associated with early erosion(s) and/or a higher severity in the progression of rheumatoid arthritis (RA).

In a further aspect, the present invention relates to the use of specific probes, like nucleic acid probes, primers as well as specific binding molecules, like antibodies or aptamers in the preparation of a diagnostic composition. Accordingly, said diagnostic composition may, inter alia, comprise one or more of the nucleic acid sequences as defined herein above as well as other specific reagents, like specific antibodies.

The corresponding probes/primers/antobodies and/or binding meolecules comprised in the diagnostic composition of the present invention can also be packaged in a "kit". Accordingly, the present invention also relates to a kit comprising specific probes and/or primers for diagnosing and/or predicting early joint destruction and/or accelerated joint destruction in rheumatoid arthritis independent of HLA-DR shared epitope (SE), said probes and/or primers being capable of detecting at least one nucleic acid sequence selected encoding for point mutation as desciebd herein, i.e. the exchange from an isoleucine to a valine on postion 50 (or 75) of the herein shown IL4R (and its corresponding isoforms and variants). Also part of this invention is a kit comprising antibodies, binding molecules, aptamers and the like which are capable of selectively binding to an encoded protein (or fragment thereof) which comprises the point mutation as described herein, e.g the I50V/I75V mutation of IL4R.

The diagnostic composition or kit of the present invention optionally comprises suitable means for detection. The nucleic acid molecule(s), or polypeptide(s) described above are, for example, suitable for use in immunoassays in which they can be utilized in liquid phase or bound to a solid phase carrier. Examples of well-known carriers include glass, polystyrene, polyvinyl ion, polypropylene, polyethylene, polycarbonate, dextran, nylon, amyloses, natural and modified celluloses, polyacrylamides, agaroses, and magnetite. The nature of the carrier can be either soluble or insoluble for the purposes of the invention.

Solid phase carriers are known to those in the art and may comprise polystyrene beads, latex beads, magnetic beads, colloid metal particles, glass and/or silicon chips and surfaces, nitrocellulose strips, membranes, sheets, duracytes and the walls of wells of a reaction tray, plastic tubes or other test tubes. Suitable methods of immobilizing nucleic acid molecule(s), vector(s), host(s), antibody(ies), aptamer(s), polypeptide(s), etc. on solid phases include but are not limited to ionic, hydrophobic, covalent interactions or (chemical) crosslinking and the like. Examples of immunoassays which can utilize said compounds of the invention are competitive and non-competitive immunoassays in either a direct or indirect format. Commonly used detection assays can comprise radioisotopic or non-radioisotopic methods. Examples of such immunoassays are the radioimmunoassay (RIA), the sandwich (immunometric assay) and the Northern or Southern blot assay. Furthermore, these detection methods comprise, inter alia, IRMA (Immune Radioimmunometric Assay), EIA (Enzyme Immuno Assay), ELISA (Enzyme Linked Immuno Assay), FIA (Fluorescent Immuno Assay), and CLIA (Chemioluminescent Immune Assay). Furthermore, the diagnostic compounds of the present invention may be employed in techniques like FRET (Fluorescence Resonance Energy Transfer) assays.

Appropriate labels and methods for labeling are known to those of ordinary skill in the art. Examples of the types of labels which can be used in the present invention include inter alia, fluorochromes (like fluorescein, rhodamine, Texas Red, etc.), enzymes (like horse radish peroxidase, β-galactosidase, alkaline phosphatase), radioactive isotopes (like 32P, 33P, 35S or 1251), biotin, digoxygenin, colloidal metals, chemi- or bioluminescent compounds (like dioxetanes, luminol or acridiniums).

A variety of techniques are available for labeling biomolecules, are well known to the person skilled in the art and are considered to be within the scope of the present invention and comprise, inter alia, covalent coupling of enzymes or biotinyl groups, phosphorylations, biotinylations, random priming, nick-translations, tailing (using terminal transferases). Such techniques are, e.g., described in Tijssen, "Practice and theory of enzyme immunoassays", Burden and von Knippenburg (Eds), Volume 15 (1985); "Basic methods in molecular biology", Davis LG, Dibmer MD, Battey Elsevier (1990); Mayer, (Eds) "Immunochemical methods in cell and molecular biology" Academic Press, London (1987); or in the series "Methods in Enzymology", Academic Press, Inc.

Detection methods comprise, but are not limited to, autoradiography, fluorescence microscopy, direct and indirect enzymatic reactions, etc.

Said diagnostic composition may be used for methods for detecting the presence and/or abundance of a nucleic acid molecule described herein, i.e. a nucleic acid molecule comprising a coding sequence which leads to the I50V (I75V) variant of IL4R (CD124) as described herein, in a biological and/or medical sample and/or for detecting expression of such a nucleic acid molecule (e.g. by determining the mRNA or the expressed polypeptide). As mentioned above, the present invention also relates to a kit comprising the diagnostic composition as described herein or the nucleic acid molecule, the polypeptide the primer or pair of primers of the invention (the mentioned primers are also described in the appended examples and herein below) or the molecule as identified or characterized in a method herein below of the present invention.

Advantageously, the kit of the present invention further comprises, optionally (a) reaction buffer(s), storage solutions and/or remaining reagents or materials required for the conduct of scientific or diagnostic assays or the like. Furthermore, parts of the kit of the invention can be packaged individually in vials or bottles or in combination in containers or multicontainer units.

The kit of the present invention may be advantageously used as diagnostic kits, as research tools or therapeutic tools. Additionally, the kit of the invention may contain means for detection suitable for scientific, medical and/or diagnostic purposes. The manufacture of the kits follows preferably standard procedures which are known to the person skilled in the art.

A specific probe to be used may be 5'-ACACGTGTATCCCTG-3' (SEQ ID NO: 5) for the detection of "I50" allele or 5'-CACGTGTGTCCCTG-3' (SEQ ID NO: 6) for the detection of "V50" allele.

As documented in the examples primer or primer pairs to be employed in the inventive method or in the corresponding uses may be determined by methods known in the art. Such primers/primer pairs may comprise 5'-ACCCAGCCCCTGTGTCT-3' (SEQ ID NO: 7) (forward primer), 5'-CGCGCCTCCGTTGTTC-3' (SEQ ID NO: 8) (reverse primer).

Also comprised in the present invention is the use of the herein described probes/primers/antibodies/binding molecules/aptamers in automated diagnostic devices, like diagnostic robotors, automated analysers, automated PCR assays, automated detection technologies (like nucleic acid hybridization to sequencing, automated ELISA tests. Automated assays known to the skilled artisan are discussed and the person skilled in the art can easily adopt the corresponding assay system to the measurement of the herein disclosed marker/SNP/allelic situation relating to the herein described IL4R single nucleotide polymorphism I50V/I75V. Examples of such automated systems comprise but are not limited to Beckman-Coulter Access, Abbott ARCHITECT and AxSYM, Bayer Advia Centaur, DPC IMMULITE 2000, and Roche Modular Analytics E170 Assay systems and methods in clinical chemistry to be employed in context of this invention are also described in Fraser, C G (1986) "Interpretation of Clinical Chemistry Laboratory Data" Blackwell; Mayne, P D (1994) "Clinical Chemistry in Diagnosis and Treatment" 6th edition. Arnold; Marshall, W J (1996) Clinical Chemistry (3rd) Gower d edition; Price, C P (1990) "Principles and Practice of Immunoassay" Stockton Press; Roitt, I M (1993) "Essential Immunology" Blackwell;. Teitz, N W (1996) "Clinical Guide to Laboratory Tests. Saunders"; Walmsley, R N, Watkinson, L R, Koag, E S C (1989). "Cases in Chemical Pathology - a Diagnostic Approach" P. G. Publishing; Whitby, L G, Smith, A F, Beckett, G J (1988) "Cases in Clinical Biochemistry" Blackwell; Burtis and Ashwood, (1999) "Tietz Textbook of Clinical Chemistry" SAUNDERS; or Bishop, (2005) "Clinical Chemistry: Principles, Procedures, Correlations", Lippincott Williams & Wilkins. PCR technology or hybridization techniques in scientific approaches as well as in diagnosis are, inter alia, described in McPherson and Moller (2006), PCR (The Basics)", Innis et al (1989), "PCR Protocols: A Guide to Methods and Applications", Academic Press.

Therefore, the person skilled in the art is readily in a position to work and carry out the present invention, since the determination of a single nucleotide polymorphism is within the normal and routine skill of the skilled artisan. The analysis, as pointed out above, can be carried out on biological samples obtained form the subject, in particular DNA or RNA samples; see also appended examples wherein,for example, DNA isolation and corresponding genotyping is described in an illustrative manner). However, also the determination of the exchanged amino acid "V" versus "I" in IL4R by methods related to protein diagnosis is invisaged and part of this invention.

The figures show:

**Figure 1. Contribution of *IL4R* to the development of erosive RA. (A)** Numbers of RA patients with erosive (white bars) or non-erosive (black bars) disease in different I50V or Q551R SNP *IL4R* groups are shown. **(B)** Frequencies of RA patients with erosive (white circles) or non-erosive (black circles) disease within different I50V *IL4R* SNP groups were calculated. Zero V50 alleles correspond to the 1501 group, one V50 allele corresponds to the I50V group, and two V50 alleles correspond to the V50V group of the patients.

**Figure 2. Diminished IL4 effect on Th1 cell differentiation from V50 homozygous individuals. (A, B)** Representative intracellular staining patterns of CD4 T cells that had been cultured in the presence of anti-IL4 ("αIL4") (A) or IL4 (B) are demonstrated. (C) The left panel shows frequencies of IFNγ-producing **Th1** cells after priming with anti-IL4 (black bars) or IL4 (white bars) in three groups of healthy individuals with the known I50V SNP genotype. For each individual group, **Th1** cell frequencies were later normalized for the **Th1** cell frequency in the respective anti-IL4-containing culture (right panel). Results are indicated as mean ± SD of 25, 28, and 23 independent experiments, respectively, using cells from different healthy donors.

**Figure 3. Diminished IL4 effect on the expression of IL12Rβ2 from V50 homozygous individuals. (A)** Representative IL12Rβ2 staining patterns of CD4 T cells that had been cultured in the presence of anti-IL4 ("αIL4") or IL4 are demonstrated. The open histogram represents the isotype control. **(B)** The left panel shows relative expression of IL12Rβ2 mRNA in cells cultured in the presence of anti-IL4 (white bars) or IL4 (black bars). On the right panel the values were normalized for the relative expression of IL12Rβ2 mRNA in cells cultured in the presence of anti-IL4. Results are indicated as mean ± SD of 6 independent experiments each using cells from different healthy donors. **(C)** The left panel shows surface expression of IL12Rβ2 on cells cultured in the presence of anti-IL4 (white bars) or IL4 (black bars). On the right panel the values were normalized for IL12Rβ2 expression on cells cultured in the presence of anti-IL4. Results are indicated as mean ± SD of 16 independent experiments each using cells from different healthy donors. MFI, mean fluorescence intensity.

The Examples illustrate the invention.

**Examples**

In the present study, the role of two *IL4R* SNPs (I50V and Q551R) in RA susceptibility and severity in an association study with 371 controls and 471 well-characterized RA patients with erosive disease was evaluated. Radiographs of the hands and feet two years after the disease onset were used to assess disease progression in RA since erosions of the hands and feet are most characteristic for RA and joint destruction is directly associated with work disability and poorer functional status (Kirwan (2001) J Rheumatol, 28(4):881-886; Sokka (2001) J Rheumatol, 28(7):1718-1722; Clarke (2001) J Rheumatol, 28(11):2416-2424). To evaluate the association of *IL4R* SNPs with disease severity, RA patients were stratified according to the radiological outcome into an erosive and a non-erosive group. Although no association between I50V and Q551R *IL4R* SNPs and disease susceptibility was identified, the I50V SNP was strongly associated with a fast development of joint destruction. The predictive power of I50V SNP for early erosive disease was higher than that of RF and the shared epitope (SE), identifying the I50V *IL4R* SNP as a novel genetic marker in RA with a high predictive value for early joint destruction. Furthermore, we addressed the functional role of 150V SNP by analyzing the response of CD4 T cells of healthy individuals with known I50V genotypes to IL4. The cells homozygous for the V50 allele demonstrated a significantly lower responsiveness to IL4 as compared to those homozygous for 150 with regard to the inhibitory effect of IL4 on **Th1** cell differentiation, providing thereby a possible mechanism which might underlie the newly identified association of I50V *IL4R* SNP with early erosions in RA.

**Patients, Materials and Methods used in the present study**

*Study population and clinical evaluation.* 471 patients with an established diagnosis of RA according to the 1987 revised criteria of the American College of Rheumatology (Amett (1988) Arthritis Rheum, 31:315-324) for the diagnosis of the disease were enrolled in the study (see following Table 1).

*Table 1. Study population*

| | Controls | RA patients |
|---|---|---|
| No. | 371 | 471 |
| Sex (f/m) | 264/107 | 369/102 |
| Age[a] (yr±SD) | 55.5 ± 16.8 | 56.9 ± 13.4 |
| Rheumatoid factor positive | n.d. | 353 |
| Shared epitope positive (1xSE/2xSE) | n.d. | 223/86 |

[a]Age was calculated at the time of the analysis
n.d., not determined

Patients with other autoimmune connective tissue diseases such as psoriasis/psoriatic arthritis, systemic lupus erytematosis or ankylosing spondylitis were excluded from the study. The patient cohort consisted of 283 patients recruited by three rheumatologists from one of the major private rheumatology practices in Central Frankonia, a region with about 300.000 inhabitants, and of 188 patients recruited at the Department of Rheumatology at the University of Erlangen-Nuremberg and the Rheumatology Clinic Ratingen. Clinical data with potential diagnostic value such as age, sex, disease duration (DD), erythrocyte sedimentation rate (ESR), levels of C-reactive protein (CRP) and levels of IgM RF were.provided by the physicians. Radiographs two years after disease onset were available from 302 patients. Two independent experienced rheumatologists who were unaware of the study questions assessed the presence or absence of erosive damage. Patients were deemed to have an erosive arthropathy if one or more definitive erosions were apparent on any peripheral joint radiograph. Patients were then stratified at two-year terms into two groups: erosive RA and non-erosive RA. A cohort of 371 healthy individuals matched on the basis of age, sex and origin (Table 1) were used as a healthy control group to evaluate the susceptibility potential of *IL4R* SNPs. All protocols and recruitment sites have been approved by the local institutional review board, and all subjects were enrolled with informed written consent.

*DNA isolation and genotyping.* 10 ml of heparinized peripheral blood were obtained from the RA patients and healthy individuals. Genomic DNA was isolated from white blood cells after the lysis of red blood cells by incubating the blood samples three times with 10 ml lysis buffer (10 mM Tris-HCl, pH 7.4, 10 mM NaCl, 3 mM $MgCl_2$) for 10 min on ice. Afterwards, DNA was isolated using the AquaPure Genomic DNA isolation kit (BioRad Laboratories, Munich, Germany), dissolved in water and stored at -80°C until analysis.

Genotypes for I50V and Q551R SNPs of *IL4R* were determined by allele specific PCR using the Assays-by-Design service for SNP genotyping from Applied Biosystems (Darmstadt, Germany). PCR primer and probe sequences for the I50V SNP were: 5'-ACCCAGCCCCTGTGTCT-3' (SEQ ID NO: 7) (forward primer), 5'-CGCGCCTCCGTTGTTC-3' (SEQ ID NO: 8) (reverse primer), 5'-ACACGTGTATCCCTG-3' (SEQ ID NO: 5) (VIC-labeled 150 allele-detecting probe) and 5'-CACGTGTGTCCCTG-3' (SEQ ID NO: 6) (FAM-labeled V50 allele-detecting probe). PCR primer and probe sequences for the Q551R SNP were: 5' GCCGAAATGTCCTCCAGCAT-3' (SEQ ID NO: 9) (forward primer), 5'-TGCTCCACCGCATGTACAA-3' (SEQ ID NO: 10) (reverse primer), 5'-CAGTGGCTATCAGGAGT-3' (SEQ ID NO: 11) (VIC-labeled Q551 allele-detecting probe) and 5'-CAGTGGCTATCGGGAGT-3' (SEQ ID NO: 12) (FAM-labeled R551 allele-detecting probe). PCR was performed using the TaqMan Universal PCR Master Mix (Applied Biosystems) in the ABI PRISM 7000 Sequence Detection System (Applied Biosystems).

To determine shared epitope (SE) homo- or heterozygosity, *HLA-DRB1* typing was performed using the DRB high-resolution kit from Biotest (Dreieich, Germany) as instructed by the manufacturer in the HLA-typing laboratory of the University of Erlangen.

*Statistical analysis.* Odds ratios (ORs), p values, sensitivities and specificities were calculated for each individual analysis, along with 95% confidence intervals (95% CIs) by use of standard contingency tables (or two-tailed Fisher's exact test). Additional analyses were performed to account for individual matching between different RA patient groups using logistic regression (LR) as described elsewhere (Breslow (1980) IARC Sci Publ (32):5-338). To assess the input of *HLA-DRB1* genotypes, samples were binned according to the number of SE alleles: high risk, two SE alleles; intermediate risk, one SE allele; low risk, no SE allele (Gorman (2004) Arthritis Rheum, 50(2):400-412). Hardy-Weinberg equilibrium for the distribution of genotypes was assessed by Fisher's exact test. Haplotype frequencies were estimated using the expectation maximization algorithm as described elsewhere (Excoffier (1995) Mol Biol Evol, 12(5):921-927). Results of experiments addressing the functional consequences of the *IL4R* SNPs were analyzed by one-way analysis of variance followed by the Bonferroni test.

*Antibodies and cytokines.* The following monoclonal antibodies (mAb) and recombinant cytokines were used for cell purification, culture and staining: anti-CD3 (OKT3), anti-CD8 (OKT8), anti-CD45RO (UCHL1), anti-CD16 (3g8FcIII; all from the American Type Culture Collection); anti-CD19 (Dako Diagnostika, Hamburg, Germany); anti-human CD28 (28.2; BD PharMingen, Heidelberg, Germany); neutralizing anti-human IL4 and human recombinant IL4 (both from Endogen, Woburn, MA); human recombinant IL2 (Chiron, Munich, Germany); FITC- or PE-conjugated mAbs to CD3, CD4, CD45RA (all from Dako Diagnostika); PE-labeled anti-IL4 (MP4-25D2), PE-labeled anti-IL2 (MQ1-17H12), FITC-conjugated anti-IFN$\gamma$ (4S.B3), and PE-conjugated anti-IL12R$\beta$2 (BD Pharmingen). The isotype-matched control for IL12R$\beta$2, PE-conjugated rat IgG2a, was purchased from BD Pharmingen.

*Cell purification.* Total CD4 T cells were isolated from heparinized peripheral blood of healthy individuals with identified *IL4R* genotypes by gradient centrifugation over a Ficoll layer (Biotest) followed by resetting of mononuclear cells with sheep erythrocytes and negative selection of CD4 T cells from the rosette positive fraction using mAbs against CD8, CD19, and CD16. For the purification of naive CD4 T cells, an additional panning with mAbs to CD45RO was performed as previously described (Skapenko (2001) J Immunol, 166(7):4283-4292). The purity of the recovered cell population was assessed by flow cytometry. Typically, $\geq$ 90% of the cells were positive for CD3 and CD4, $\geq$ 90% of isolated naive T cells were positive for CD45RA, and $\geq$ 98% of the cells were viable after purification.

*Cell culture.* Cell cultures were carried out in RPMI 1640 medium supplemented with penicillin G/streptomycin (50 U/ml), L-glutamine (2 mM) (all from Gibco-Invitrogen, Karlsruhe, Germany), and 10% normal human serum. T cell differentiation was assessed employing an *in vitro,* multiple-step differentiation system as previously described (Schulze-Koops (1998) Eur J Immunol, 28:2517-2529; Skapenko (2001) loc. cit.). In brief, freshly isolated naive CD4 T cells (0.5 x $10^6$/ml) were primed by plate-bound OKT3 (1 $\mu$g/ml) and soluble anti-CD28 mAbs (1 $\mu$g/ml) in the presence of IL2 (10 U/ml) and in the presence of either anti-IL4 (10 $\mu$g/ml) or IL4 (31.25 ng/ml). After 5 days of priming, the cells were harvested, washed, counted, and rested for 2.5 days at a concentration of 1 x $10^6$ cells/ml in medium supplemented with IL2 (10 U/ml). The differentiation of the T cells was determined by intracellular flow cytometry of cytoplasmic cytokines. To analyze the IL12R$\beta$2 chain expression, freshly isolated CD4 T cells were activated with immobilized OKT3 (1 $\mu$g/ml) and soluble anti-CD28 mAbs (1 $\mu$g/ml) in the presence of IL2 (10 U/ml) and either anti-IL4 (10 $\mu$g/ml) or IL4 (31.25 ng/ml for IL12R$\beta$2 protein or 7.8 ng/ml for IL12R$\beta$2 mRNA expression analysis). IL12R$\beta$2 protein surface expression was assessed after 96 h of culture by flow cytometry. IL12R$\beta$2 mRNA levels were determined by quantitative RT-PCR after 48 h of priming.

*Flow cytometry.* To determine the phenotype of differentiated CD4 T cells, the cells were stimulated with ionomycin (1 $\mu$M, Calbiochem, Schwalbach, Germany) and PMA (20 ng/ml, Sigma-Aldrich, Taufkirchen, Germany) for 5 h at 37°C in the presence of 2 μM monensin, harvested, washed with PBS (Gibco Invitrogen) and fixed with 3% paraformaldehyde (Sigma-Aldrich) in PBS for 10 min at 37°C. Cells were permeabilized with 0.1% saponin (Sigma-Aldrich) in 2% FCS/PBS, and the unspecific binding sites were blocked with 4% rat and mouse serum (both from Sigma-Aldrich). Cells were incubated for 30 min at 4°C with saturating amounts of directly fluorochrome-labeled mAbs against IL2, IL4, and IFNγ. After washing with 0.1% saponin in 2% FCS/PBS, cells were resuspended in 2% FCS/PBS and analyzed by flow cytometry (Skapenko (1999) J Immunol, 163:491-499). For measuring IL12Rβ2 expression on the surface of activated cells, 100 x $10^3$ cells were washed with 2% FCS/PBS, incubated with saturating amounts of PE-labeled Abs to IL12Rβ2 for 15 min at 4°C and analyzed by flow cytometry.

*Total RNA isolation and quantitative RT-PCR.* CD4 T cells were activated as described above, harvested, washed with PBS, and total RNA was isolated using the RNeasy Mini Kit (Qiagen, Hilden, Germany). To exclude genomic DNA contamination, RNA samples were treated with DNase (Qiagen). mRNA was transcribed to cDNA for 1 h at 42°C in a total volume of 20 μl containing 1 x AMV RT buffer (Promega, Mannheim, Germany), ImM dNTPs, 100 ng/ml oligo $dT_{12-18}$ (both from Amersham Pharmacia Biotech, Freiburg, Germany), and 0.25 U/μl AMV RT (Promega). Real-time PCR was performed in duplicates using the Universal PCR Master Mix in the ABI Prism 7000 Sequence Detection System (all from Applied Biosystem, Darmstadt, Germany). Assays-on-Demand Gene Expression Products for cyclophilin from Applied Biosystem was used to determine cyclophilin mRNA expression. To determine IL12Rβ2 mRNA expression, a set of primers consisting of 5'-TCCAGATCCAGCAAATAGCACTTG-3' (SEQ ID NO: 13) (forward primer), 5'-AGTCTATCAGGAGCCTGTCCAA-3' (SEQ ID NO: 14) (reverse primer) and 5'-TTGCAGAGGAGAAGACAC-3' (SEQ ID NO: 15) (FAM-labeled probe) was designed by the Custom TaqMan Gene Expression Assay Service (Applied Biosystems). Relative quantification of IL12Rβ2 cDNA to cyclophilin was determined by calculating the difference in cross-threshold values (ΔCt) according to the formula $2^{-\Delta Ct}$.

**Example 1: Patient characterization; incidence/susceptibility and association with destruction disease**

The 471 patients represent a geographically diverse, ethnically homogeneous cohort of RA patients who are likely to be broadly representative of patients with RA seen by rheumatologists (see Table 1 above). The mean ± SD age of the patients at time of the analysis was 56.9 ± 13.4 years, and 78% were female. 353 patients (75%) were RF positive, and 309 patients (66%) were positive for at least one SE allele. The mean CRP ± SEM value was 19.9 ± 0.2 mg/l, and the mean ± SEM ESR was 27.8 ± 1.2 mm/h. At the time of analysis mean ± SD DD was 7.0 ± 6.1 years. Radiographs of the hands and/or feet two years after disease onset were available from 302 patients.

In order to examine the possible contribution of I50V and Q551 R polymorphisms *of IL4R* to RA susceptibility, a case-control association study was performed. The distribution of the genotypes in RA patient and in control groups is shown in Table 2.

*Table 2. Frequency (%) of IL4R genotypes in individuals with RA and controls*

| Genotype | Controls (n=371) | RA patients (n=471) | p[a] |
|---|---|---|---|
| At nt 148 (150V) | | | |
| AA (1501) | 29.9 (n=111) | 28.5 (n=134) | ... |
| AG (I50V) | 46.9 (n=174) | 51.4 (n=242) | 0.42 |
| GG (V50V) | 23.2 (n=86) | 20.2 (n=95) | 0.69 |
| At nt 1652 (Q551R) | | | |
| AA (Q551Q) | 61.2 (n=227) | 64.5 (n=304) | ... |
| AG (Q551R) | 35.3 (n=131) | 30.8 (n=145) | 0.21 |
| GG (R551R) | 3.5 (n=13) | 4.7 (n=22) | 0.60 |

[a]Values were calculated based on the individual numbers by use of Fisher's exact test

Each polymorphism was in Hardy-Weinberg equilibrium, and the frequencies were consistent with those previously reported by others (Hackstein (1999) Hum Immunol, 60(11):1119-1127, Howard (2002) Am J Hum Genet, 70(1):230-6). There were no significant differences in the genotype and allele frequencies between RA patients and healthy controls. Moreover, no differences were observed between RA patient and control cohorts in the distribution of possible *IL4R* haplotypes calculated based on the frequencies of those two SNPs either (data not shown). Thus, *IL4R* seems not to be associated with RA susceptibility.

To evaluate the impact of *IL4R* on disease development, we classified the RA patients, from whom radiographs of the hands and/or feet were available two years after disease onset into two groups with erosive and non-erosive disease, respectively. 151 patients showed joint erosions in at least one joint typically affected in RA and 151 patients had no signs of bone destruction at two years DD. Whereas a significant difference in the distribution of the I50V SNP genotypes was observed in the patients with erosive compared to those with non-erosive disease ($\chi^2$ 15.68, p = 0.0004; Figure 1A, left panel), the distribution of genotypes for the Q551R SNP was similar in both groups ($\chi^2$ 0.11, p = 0.95; Figure 1A, right panel). Epidemiologic characteristics of the three groups of patients homo- and heterozygous for the I50V SNP are summarized in Table 3 below.

*Table 3. Epidemiologic parameters of the patients with available radiographs two years after RA onset*

| | Total cohort | AA (1501) | AG (I50V) | GG (V50V) |
|---|---|---|---|---|
| No. | 302 | 92 | 138 | 72 |
| Sex (f/m) | 229/73 | 66/26 | 104/34 | 59/12 |
| Age[a] (yr±SD) | 54.4 ± 13.7 | 53.3 ± 13.9 | 55.9 ± 12.9 | 52.8 ± 14.8 |
| Rheumatoid factor positive | 220 | 65 | 98 | 57 |
| Shared epitope positive (1xSE/2xSE) | 144/57 | 41/18 | 64/28 | 39/11 |

[a]Age was calculated at the time point of two years after disease onset

No significant differences were observed between the three groups and between any of the groups and the total patient cohort either. Moreover, the epidemiologic characteristics of all the three groups were representative of those from the total cohort. Nevertheless, patients homozygous for the V50 mutation developed significantly more often erosive disease within the first two years of the disease than those homozygous for 150 (OR 3.63, 95% CI 1.89-6.97, p < 0.0001; Table 4). 68.1% of the V50 compared to only 37.0% of the 150 homozygous patients showed radiographic erosions at two years DD. The association was independent of individual factors previously associated with severe disease, as CLRs that adjusted for either SE or RF revealed no significant changes in the OR (OR adjusted for SE 3.52, 95% CI 1.84-6.75, p < 0.0001; OR adjusted for RF 3.50, 95% CI 1.83-6.69, p < 0.0001). Adjusting for *HLA-DRB1* genotypes, RF, sex and age resulted even in a slight increase of the OR to 3.86 (95% CI 1.95-7.64, p < 0.0001; see following Table 4). Patients heterozygous for I50V SNP had also an almost two times higher risk to develop joint destruction already in the first two years after disease onset (OR 1.69, 95% CI 0.96-2.98; see following Table 4).

*Table 4. Association of IL4R with bone erosion in RA*

| | | | Results for | | | | | |
|---|---|---|---|---|---|---|---|---|
| | Erosive disease[a] | | Crude analysis[b] | | | CLR[d] | | |
| Genotype | Yes | No | OR[c] | 95% CI | p | OR[c] | 95% CI | p |
| AA (1501) | 34 | 58 | ... | ... | ... | ... | ... | ... |
| AG (I50V) | 68 | 70 | 1.66 | 0.97-2.84 | 0.08 | 1.69 | 0.96-2.98 | 0.07 |
| GG (VSOV) | 49 | 23 | 3.63 | 1.89-6.97 | < 0.0001 | 3.86 | 1.95-7.64 | < 0.0001 |

[a]Joint destruction within the first 2 years of the disease was determined by X-ray analysis
[b]Values were calculated by use of Fisher's exact test
[c]ORs were calculated relative to the reference allele homozygote (AA)
[d]CLR was performed to account for individual matching for sex, age, RF, and SE between different RA patient groups

The contribution of 150V *IL4R* SNP to the development of erosive disease demonstrates, therefore, a significant allele-dose effect of V50 ($\chi^2$ for trend 15.37, p < 0.0001) and can be illustrated by the increase of patients with erosive disease with increasing numbers of the V50 allele (Figure 1B). These data indicate that the presence of the V50 allele favors accelerated destruction of the joints during early RA independent of the *HLA-DRB1* genotype and of RF.

**Example 2: The predictive value of the V50 allele**

The increased risk of RA patients carrying a V50 allele to develop early erosive disease implicates a potential predictive value of *IL4R* in RA. Evaluation of the sensitivity and the specificity of the V50 allele for early development of joint erosion revealed that they both were comparable to those of the SE or the RF; see Table 5 below.

*Table 5. Sensitivity and specificity of IL4R as a predictive marker for early bone erosion in RA*[a]

| Predictive parameter | Sensitivity (95% CI) | Specificity (95% CI) |
|---|---|---|
| 1x *IL4R*/V50[b] | 0.78 (0.70 - 0.84) | 0.38 (0.31 - 0.47) |
| 2x *IL4R*/V50 | 0.33 (0.25 - 0.41) | 0.85 (0.78 - 0.90) |
| 1x *IL4R*/V50 + *HLA-DRB1*/SE[c] | 0.58 (0.49 - 0.66) | 0.64 (0.55 - 0.71) |
| 2x *IL4R*/V50 + *HLA-DRB1*/SE | 0.23 (0.17 - 0.31) | 0.90 (0.84 - 0.94) |
| 1x *IL4R*/V50 + RF | 0.56 (0.49 - 0.65) | 0.54 (0.46 - 0.62) |
| 2x *IL4RN50* + RF | 0.27 (0.20 - 0.35) | 0.89 (0.83 - 0.94) |
| 1x *IL4R*/V50 + *HLA-DRB1*/SE + RF | 0.47 (0.39 - 0.55) | 0.70 (0.62 - 0.77) |
| 2x *IL4R*/V50 + *HLA DRB1*/SE + RF | 0.21 (0.15 - 0.28) | 0.92 (0.87 - 0.96) |
| *HLA-DRB1*/SE | 0.72 (0.64 - 0.79) | 0.39 (0.31 - 0.47) |
| RF | 0.73 (0.65 - 0.80) | 0.27 (0.20 - 0.35) |
| *HLA-DRB1*/SE + RF | 0.60 (0.53 - 0.68) | 0.49 (0.41 - 0.57) |

[a] Values were calculated based on the individual numbers by use of Fisher's exact test
[b]*IL4R*/V50, V50 allele *of IL4R*
[c]*HLA-DRB*/SE, *HLA-DRB1* alleles that share the SE sequence

The presence of two V50 alleles increased the specificity of *IL4R* to 0.85. Moreover, in combination with either other investigated predictive marker, SE or RF, the specificity of the presence of two V50 alleles was further increased and, if SE and RF were both present, the sensitivity was as high as 0.92. In marked contrast, the combination of SE and RF without the impact of *IL4R* did not reach even the predictive power of V50 homozygosity alone. Thus, *IL4R* appears to be a novel genetic marker with high predictive value for early erosive RA and might therefore be of significance in the clinic to identify patients with accelerated joint destruction early before the development of rheumatoid damage.

**Example 3: Elucidation of biological mechanisms and interpretation of results**

Based on the observation that an exaggerated Th1 cell differentiation is associated with accelerated disease progression (Kanik (1998) loc. cit.; van der Graaff (1998) loc. cit.) and on the role of IL4 in controlling Th1 differentiation, we carried out experiments to test whether *IL4R* SNPs can modulate the inhibitory effect of IL4 on Th1 differentiation in healthy individuals with a known I50V SNP genotype. Naive CD4 T cells were primed *in vitro* for 5 days with anti-CD3 and anti-CD28 antibodies to induce Th1 cell differentiation. Neutralizing anti-IL4 antibodies or IL4 were added to the cultures to allow the assessment of the inhibitory effect of IL4. After additional 2.5 days of resting, the extent of Th1 cell differentiation was estimated by flow cytometry of cytoplasmic IFN$\gamma$ and IL4 (Figures 2A, B). As shown in Figure 2C (left panel), Th1 cell differentiation that occurred in the presence of neutralizing anti-IL4 antibodies was comparable in all three individual groups. The presence of IL4 in the culture resulted in a significant reduction in the frequencies of IFN$\gamma$-producing Th1 cells in all groups. Strikingly, however, when the extent of Th1 cell differentiation in the presence of IL4 was normalized to the respective value of Th1 differentiation in the presence of anti-IL4, a clear difference in the effect of IL4 between the three groups became apparent (Figure 2C, right panel). In other words, the extent of the inhibition of Th1 cell differentiation by IL4 was significantly less pronounced in the group of individuals homozygous for the variant allele, V50 as compared to the groups of 1501 or I50V individuals (Figure 2C, right panel).

In a similar way, we investigated the inhibitory effect of IL4 on mRNA and protein expression of IL12R$\beta$2, a subunit of the IL12R expressed on developing Th1 cells (Szabo (1997) J Exp Med, 185:817-824) that facilitates the sensitivity of the cells to the Th1-inducing activity of IL12 (Figure 3A). Whereas both mRNA and protein expression of IL12R$\beta$2 were significantly reduced in CD4 cells that were cultured in the presence of IL4 as compared to those cultured with neutralizing anti-IL4 antibodies independent of the genotype group (Figure 3B, 3C, left panels), normalized expression levels demonstrate significant differences between the groups (Figure 3B, 3C, right panels). The extent of IL4 inhibition for both, protein and mRNA levels of IL12$\beta$2 was about two times as pronounced in T cells from 1501 individuals compared to T cells isolated from V50V individuals. Together, V50 homozygosity mediated a markedly reduced response of the T cells to IL4. This might contribute to a more intensive Th1 cell differentiation in patients homozygous for V50 and lead in those patients to accelerated erosive disease.

The results provided alone are clear evidence for an association of the V50 allele *of IL4R* with the fast development of radiographic bone erosions in the joints of patients with RA. This is surprising in the light of previously published evidence that the I50V allele/SNP has no predictive value and is not informative; see Goronzy (2004), loc. cit. While the association of one V50 allele of the IL4R with erosive disease was relatively modest (yet informative), it was strong with an OR as high as 3.86 (95% CI 1.95-7.64) if two V50 alleles were present. The data provided here show the first association of the V50 allele of IL-R4 with erosive disease in RA.

Although a majority of previous studies demonstrate an association of the SE sequence-sharing *HLA-DRB1* alleles with the development of erosive disease, some striking inconsistencies exist (Meyer (1999) J Rheumatol, 26(5):1024-1034; Combe (1995) Br J Rheumatol, 34(6):529-534; Thomson (1999) loc. cit.; Gorman (2004) loc. cit.), indicating that other genetic loci influencing disease progression might exist. Identification of reliable predictors of severity early in the disease is important, on the other hand, because of the correlation of erosions with clinical outcome such as disability (Kirwan (2001) loc. cit.; Sokka (2001) loc. cit.) and the recent demonstration that early treatment may retard bone destruction (O'Dell (2002) loc. cit.). The development of anti-tumor necrosis factor agents such as etanercept (Bathon (2000) N Engl J Med, 343(22):1586-1593) and infliximab (Lipsky (2000) N Engl J Med 2000;343(22):1594-1602) has revolutionized the treatment of RA, although at this time, long-term safety information is missing and TNF-neutralizing treatment is extremely expensive. Therefore, the potential to more precisely predict early in the disease which individual will require more aggressive treatment would be especially valuable.

The association of the I50V SNP (or "I75V" SNP) with bone erosions of RA patients already at two years DD suggests a potential of this polymorphism as a predictive marker. In fact, the predictive value estimate of the V50 allele revealed the powerful capacity of this allele to identify individuals with a high risk of early onset of joint destruction. Although typing of the patients just for the presence of the V50 allele demonstrated relatively low specificity, identification of patients homozygous for the V50 allele allows the prediction of a fast destructive course of the disease with reasonable confidence (specificity at 0.85). The predictive value of one V50 allele alone in our cohort of the RA patients was comparable to that of the RF or of one SE allele. The ORs of RF as well as SE alleles observed in our study, on the other hand, were comparable to those reported by others (Thomson, (1999) loc. cit.; Meyer (2003) Ann Rheum Dis, 62(2):120-126; Gorman (2004) loc. cit.). However, in contrast to RF or to SE alleles, the combination of the V50 allele with either other predictive marker resulted in an increase of the predictive power to over 0.50, whereas the combination of RF and SE alleles even did not reach that value. Moreover, the predictive value of the RF and SE alleles in combination was much lower than the predictive value of V50 homozygosity, emphasizing the powerful capacity of *IL4R* as a newly identified predictive parameter to identify at-risk RA patients early in the disease.

Elucidating the genetic basis of RA is currently one of the major challenges in modern rheumatology. RA is a complex autoimmune disease that is, unlike those Mendelian traits causally related to the highly penetrant rare mutations of single-genes, caused by small individual effects of many low penetrant common alleles. This makes the identification of the autoimmune disease-related genes substantially difficult. As documented herein the V50 allele of *IL4R* is a reliable marker for susceptibility of RA because of several reasons: 1) its chromosomal position, 2) the important role of its ligand, IL4, in the pathogenesis of the disease, 3) previous identification of SNPs leading to functional alterations of IL4R, and 4) associations *of IL4R* with other complex diseases characterized by a Th1/Th2 shift. Therefore, we undertook a case-control association study with 471 RA patients and 371 age- and sex-matched healthy individuals. However, no association between the *IL4R* and RA susceptibility was detected in our cohort of RA patients. In our analysis, though, we focused only on those SNPs, which were previously demonstrated to lead to some functional alterations of the IL4R. An additional analysis of non-synonymous SNPs, or SNPs in the noncoding region of *IL4R,* or synonymous SNPs that, however, did not result in detectable functional alterations in cellular assays might be required to fully exploit the association of *IL4R* with RA susceptibility. Initial investigations of *IL4R* SNPs in case-control studies revealed association of the two individual SNPs, I50V (Mitsuyasu (1998) loc. cit.) and Q551R (Hershey (1997) loc. cit.) with atopic disorders. In contrast, a later investigation analyzing multicase atopic families was able to identify a significant association of atopy with *IL4R* only if seven *IL4R* SNPs were determined simultaneously, indicating an association of atopic disorders with particular haplotypes of *IL4R* (Ober (2000) Am J Hum Genet, 66(2):517-526). Likewise, only a defined haplotype of *IL4R* could be associated with diabetes in a study of diabetes multiplex families with affected siblings pairs (Mirel (2002) loc. cit.). Even more, only a particular combination of SNPs in *IL4R* and in another relevant gene, for example *IL13,* has been demonstrated to be associated with diabetes among Filipinos or with asthma in a Dutch population (Bugawan (2003) loc. cit.; Howard (2002) Am J Hum Genet, 70(1):230-236). All together, these data confirm the difficulties in exploitation of the genetic basis of complex diseases and indicate the necessity to expand the number of investigated SNPs involving even those SNPs that are located in genes others than *IL4R.*

Because of the well-established linkage between *IL4R* and atopic disorders (Mitsuyasu (1998) loc. cit.; Hershey (1997) loc. cit.; Ober (2000) loc. cit.) several investigations have been performed in attempts to identify whether *IL4R* allelic variants may lead to alterations in cell functions. Specific signaling of the IL4R is transmitted mainly via signal transducer and activator of transcription 6 (STAT6) (Nelms (1999) Annu Rev Immunol, 17:701-738). A broad spectrum of genes is transcriptionally regulated via STAT6. STAT6 facilitates the expression of IgE and its receptor, CD23, in B cells and of a number of genes involved in Th2 differentiation in CD4 T cells. On the other hand, STAT6 acts as a repressor of the transcription of genes involved in Th1 cell differentiation, such as IFN$\gamma$, IL12R$\beta$2, and IL18R$\alpha$. Despite of the I50V SNP vicinity to the IL4-binding site of the IL4R no alterations in IL4R affinity to IL4 have been observed (Mitsuyasu (1998) loc. cit.; Mitsuyasu (1999) loc. cit.). Nevertheless, a more pronounced STAT6-dependent transcriptional activity in Jurkat T cells transfected with the 150 allelic variant of IL4R has been detected (Stephenson (2004) J Immunol, 173(7):4523-4528). Accordingly, a more pronounced response to IL4 of B cells bearing the I50 variant compared to those bearing the V50 variant with regard to CD23 expression and IgE synthesis has been reported (Mitsuyasu (1999) loc. cit.).We found that CD4 T cells isolated from individuals homozygous for the 150 allele demonstrated significantly better responses to IL4 compared to those CD4 T cells isolated from V50V individuals. 1501 CD4 T cells reacted more effectively to the inhibitory effect of IL4 on Th1 cell differentiation. Our observation that the inhibitory effect of IL4 on Th1 cell differentiation can be modulated by *IL4R* allelic variants appears, however, is in contrast to a recent publication (Stephenson (2004) loc. cit.). Without being bound by theory, this may be explained by the fact that in that report murine CD4 T cells transfected with different IL4R constructs were used, whereas we worked with primary CD4 T cells freshly isolated from individuals with determined I50V genotype. The less pronounced inhibition of Th1 cell differentiation as well as of IL12Rβ2 expression by IL4 in CD4 T cells from V50V individuals as demonstrated here fits well with the association of the V50 homozygosity with accelerated radiographic joint erosions, since it is conceivable that a less controlled Thl-mediated rheumatic inflammation would drive more aggressively destructive joint processes. Herein a mechanism is proposed which might underlie the association of V50 SNP of the *IL4R* with rapid development of erosions in RA, implicating thereby a functional impact of the *IL4R* in RA pathophysiology. Yet, it is to be understood that the present invention is not limited to the herein described and proposed mechanism.

In this invention we investigated the association *of IL4R* with both, disease susceptibility and disease progression. Although no association was observed with the onset of the RA, a strong linkage between the I50V *IL4R* SNP and rapid rheumatic joint destruction became apparent. The predictive value of the I50V *IL4R* SNP for the development of erosive disease was superior to that of conventional biomarkers of joint destruction, RF and the presence of the SE. As a possible mechanisms underlying this association, it could be shown that the mutant V50 allele confers reduced responsiveness of T cells to the immunomodulatory effects of IL4, resulting in decreased down-modulation of the IL12β2R and reduced inhibition of Th1 cell differentiation. The data provided herein identify a unique genetic marker with high predictive value that is to be used for the benefit of RA patients to identify those with erosive disease early before irreversible joint damage has occurred. According therapeutic strategies can then be developed in accordance with the genetic pre-disposition of the invidual.

**Example 4: Specificity of the *IL4R* Single-Nucleotide Polymorphism I50V as a Predictive Marker in Rheumatoid Arthritis: No association with Erosive Disease in Psoriatic Arthritis**

IL4 receptor polymorphisms have been demonstrated to predispose to an imbalance in the Thl/Th2 ratio of adaptive immune responses in a variety of immune disorders (Romagnani, Immunol Today (1997) 18:263-6). Moreover, association studies as provided in the above examples of two functional single nucleotide polymorphisms in the coding region of the IL-4 R gene, the I50V and the Q551R polymorphisms (which are on different haplotype blocks) has demonstrated that the inheritance of the 50V encoding SNP enhances the genetic risk for joint destruction and an erosive Th1-biased disease course in rheumatoid arthritis considerably. Since the imbalance of Th1/Th2 immune responses has also been suggested to play an important role in psoriasis (Schlaak, J Invest Dermatol. (1994) 102:145-9) and psoriatic arthritis (Ritchlin, J Rheumatol. (1998) 25:1544-52; Partsch, Ann Rheum Dis. (1998) 57: 691-3), we have decided to perform an association study of the I50V and Q551R polymorphism in large cohorts of German patients suffering from psoriasis either with or without arthritic manifestations.

The 375 single patients with psoriasis vulgaris (Huffmeier, J Invest Dermatol. (2005) 125: 906-12) were recruited through dermatology clinics at two psoriasis rehabilitation hospitals. An early onset form of psoriasis was diagnosed in all but three patients with an average age of onset of 23 ± 11 years. The majority of patients suffered from plaque type of psoriasis vulgaris. 62 % of patients were male. The study including recruitment of controls was approved by the ethical committee of the University of Münster.

The 375 patients (Lascorz, Ann Rheum Dis. (2005) 64: 951-4) with psoriatic arthritis were similarly of German descent and recruited through four different rheumatological centres in Germany: one acute clinic, two rehabilitation hospitals and one private practice. The diagnosis of PsA was made by a board certified rheumatologist according to the criteria of Moll and Wright (Semin Arthritis Rheum 1973; 3: 55-78) in addition all patients included into the investigation also fulfilled the recently developed CASPAR criteria (CIASsification of Psoriatic ARthritis study group). Patients exhibiting erosions in at least one joint on plain radiographs of hand and feet or any other joint investigated were regarded as to have erosive arthritis. Patients without any signs of joint erosion were assigned to have a non-erosive disease course. The study was approved by the Ethics Committee of the Friedrich-Alexander-University of Erlangen-Nuremberg.

Average age of onset for psoriasis vulgaris was 30 ± 13 years. 59.9 % of patients were male. All patients were negative for the rheumatoid factor. For 78 % of the patients, the diagnosis of psoriatic arthritis was made ≥ 3 years prior to recruitment. Erosive disease was recorded in 197 patients (53 %). In 178 patients (47.5 %) the erosive disease was associated with a disease duration of more than 3 years. PsA persisting for a a period longer than three years as a non-erosive disease was recorded in 79 patients (20.8 %). 19 patients with erosive and 36 patients with non-erosive arthritis were not included in the association study on erosive outcome due to the limited arthritis duration of less than 3 years, while for 63 patients (17 %) no data about the radiographic status were available.

Controls. The 376 controls had no psoriasis vulgaris at the time of recruitment when average age was 32 ± 10 years. All of them were healthy blood donors and recruited in Northern Germany. 59 % of probands were male.

All patients and controls were of German origin. The individuals gave their written informed consent. The investigations were conducted according to Declaration of Helsinki principles.

Genotyping of the two SNPs in the IL4R gene was performed with either Taqman SNP Genotyping Assays from Applied Biosystems (Foster City, USA) in the case of rs1805010 (I50V) or with self-designed primers and TaqMan mgb-probes for rs1801275 (Q551R). All Taqman reactions were carried out in an ABI Prism 7900HT Sequence Detection System (Applied Biosystems) with 10 ng of genomic DNA as a template in a 5 μl reaction using standard thermal cycling conditions. Sequences of primers and probes as well as assay IDs are available upon request. Taqman genotypes for both polymorphisms were verified by direct sequencing of 24 randomly chosen samples.

The analysis did not reveal significant differences in allele frequencies between controls and the cohorts of PsA or psoriasis vulgaris patients for any of the two IL-4R variants under investigation, see following table.

| Polymorphism | | Psoriasis vulgaris | | | Controls | Psoriatic arthritis | | |
|---|---|---|---|---|---|---|---|---|
| | | n (%) | $\chi^2$ | p-value | n(%) | n(%) | $\chi^2$ | p-value |
| rs1805010 (I150V) | Allele A<br>Allele G | 402 (55)<br>328 (45) | 0.017 | 0.896 | 405 (55)<br>335 (45) | 415 (57)<br>315 (43) | 0.669 | 0.413 |
| rs1801275 (Q551R) | Allele A<br>Allele G | 588 (80)<br>150 (20) | 0.033 | 0.856 | 582 (79)<br>152 (21) | 587 (79)<br>153 (21) | 0.0 | 1.0 |

Allele frequencies of the two polymorphisms in IL4R in patients and controls and results of the $\chi^2$ statistics.

Hardy-Weinberg equilibrium for both SNPs was confirmed in all cohorts. By contrast to recent results on the predictive value of the 50V variant of the IL-4 receptor for a more rapid progression of bony erosions in RA our stratification of the PsA cohort according to erosive or non-erosive outcome after a disease course of at least 3 years did not show association to the analyzed two functional SNP polymorphism, see the following table:

| Polymorphism | | PsA erosive | | | PsA non-erosive |
|---|---|---|---|---|---|
| | | n (%) | $\chi^2$ | p-value | n (%) |
| rs1805010 (I50V) | Allele A<br>Allele G | 200 (57)<br>150 (43) | 0.152 | 0.696 | 84 (55)<br>68 (45) |
| rs1801275 (Q551R) | Allele A<br>Allele G | 283 (80)<br>69 (20) | 0.161 | 0.688 | 123 (79)<br>33 (21) |

Allele frequencies of the two polymorphisms in IL4R in the PsA patients with and without erosive arthritis after a disease course > 3years and results of the $\chi^2$ statistics.

This lack of association did not change upon the consideration of an allele dosage effect in a slightly modified statistical analysis, see the following table:

| Genotype rs1805010 (I50V) | Yes | No | OR§ | p† |
|---|---|---|---|---|
| AA (I) | 54 | 21 | | |
| AG | 92 | 42 | 0.85 | 0.64 |
| GG (V) | 29 | 13 | 0.86 | 0.83 |

Erosive disease was determined by analysis of radiographs.
† Values were calculated with Fisher's exact test.
§ Odds ratios (ORs) were calculated relative to the reference allele homozygote (AA).

Thus also homozygosity for the IL4R 50V allele that is a risk for an erosive RA course did not prove to have the same predictive value for radiographic outcome in PsA. Particularly interesting are two aspects of these results. 1.

The predictive value of homozygosity for the IL4R-50V allele has been suggested to be used as a genetic marker for the decision making on therapeutic treatment strategies in early RA especially on expensive modalities such as anti-TNF-therapy that is also successfully applied to PsA. These studies suggest that this marker would not improve patient stratification according to individual risk profiles in PsA by contrast to RA where it might be beneficial. 2. Despite the data on the functional impact of the IL-4R50V variant on IL-4 cytokine-induced Stat 6 activation and subsequent modulations of cytokine receptor expression and cell differentiation, the consequences of the same polymorphism for joint pathology in RA, as shown in the example above, PsA still seem to differ considerably despite the fact that a Th1 polarized phenotype is dominating the T cell population in the joints in both diseases.

## Claims

**1.** A method of diagnosing and/or predicting erosive joint destruction in rheumatoid arthritis comprising determining in a sample obtained from an individual independent of HLA-DR shared epitope (SE) the presence of at least one nucleic acid sequence selected from the group consisting of

(a) a nucleic acid sequence encoding an IL-4 receptor (IL-4R) which contains a mutation in position 465 of the nucleotide sequence of the wild-type IL4R as shown in SEQ ID NO: 1, whereby at said position the nucleotide A is replaced by the nucleotide G;
(b) a nucleic acid sequence encoding an IL-4 receptor (IL-4R), said IL-4R comprising at position 75 as shown in SEQ ID NO: 2 a valine;
(c) a nucleic acid sequence comprising at least 15 nucleotides of the nucleic acid sequence of (a) or (b) and comprising the mutation as defined in (a) or (b);
(d) a nucleic acid sequence comprising a nucleotide sequence as shown in SEQ ID NO: 3 or a fragment of the sequence as shown in SEQ ID NO: 3, said fragment comprising the nucleotide sequence "gtc"/"guc" as shown in position 5758 to 5760 of SEQ ID NO: 3 and said fragment comprising at least 15 nucleotides;
(e) a nucleic acid sequence encoding a polypeptide comprising the amino acid sequence as shown in SEQ ID NO: 4; and
(f) a nucleic acid sequence comprising at least 12 nucleotides which hybridizes under stringent conditions to a nucleotide sequence defined in any one of (a) to (e) and having a mutation as defined in (a) or (b).

**2.** A method of diagnosing and/or predicting erosive joint destruction in rheumatoid arthritis comprising determining in a sample obtained from an individual independent of HLA-DR shared epitope (SE) the presence of an encoded IL-4 receptor (IL-4R) which comprises at the homologous position 75 of IL-4 receptor as depicted in SEQ ID NO: 2 a mutation, said mutation comprising the exchange from an isoleucine to a valine.

**3.** The method of claim 1, wherein said mutation is detected in homozygous alleles/homozygosity.

**4.** The method of any one of claims 1 to 3, wherein said joint destruction is associated with erosion(s) and/or a higher severity in the progression of rheumatoid arthritis (RA).

**5.** The method of any one of claims 1 to 4, said method comprising PCR-technology, ligase-chain reaction, NASBA, restriction digestion, direct sequencing, nucleic acid amplification techniques, MALDI, MALDI-TOF, hybridization techniques and/or immuno assays.

**6.** Use of specific probes and/or primers in the *in vitro* diagnosis and/or prediction of erosive joint destruction in rheumatoid arthritis independent of HLA-DR shared epitope (SE), wherein said probes and/or primers are capable of detecting at least one nucleic acid sequence selected from the group consisting of

(a) a nucleic acid sequence encoding an IL-4 receptor (IL-4R) which contains a mutation in position 465 of the nucleotide sequence of the wild-type IL4R as shown in SEQ ID NO: 1, whereby at said position the nucleotide A is replaced by the nucleotide G;
(b) a nucleic acid sequence encoding an IL-4 receptor (IL-4R), said IL-4R comprising at position 75 as shown in SEQ ID NO: 2 a valine;
(c) a nucleic acid sequence comprising at least 15 nucleotides of the nucleic acid sequence of (a) or (b) and comprising the mutation as defined in (a) or (b);
(d) a nucleic acid sequence comprising a nucleotide sequence as shown in SEQ ID NO: 3 or a fragment of the sequence as shown in SEQ ID NO: 3, said fragment comprising the nucleotide sequence "gtc"/"guc" as shown in position 5758 to 5760 of SEQ ID NO: 3 and said fragment comprising at least 15 nucleotides;
(e) a nucleic acid sequence encoding a polypeptide comprising the amino acid sequence as shown in SEQ ID NO: 4; and
(f) a nucleic acid sequence comprising at least 12 nucleotides which hybridizes under stringent conditions to a nucleotide sequence defined in any one of (a) to (e) and having a mutation as defined in (a) or (b).

**7.** Use of specific antibody molecules or specific binding molecules in the *in vitro* diagnosis and/or prediction of erosive joint destruction in rheumatoid arthritis independent of HLA-DR shared epitope (SE), wherein said specific antibody molecule or specific binding molecule is capable to specifically detect and/or bind to an epitope which is encoded by nucleic acid sequence selected from the group consisting of

(a) a nucleic acid sequence encoding an IL-4 receptor (IL-4R) which contains a mutation in position 465 of the nucleotide sequence of the wild-type IL4R as shown in SEQ ID NO: 1, whereby at said position the nucleotide A is replaced by the nucleotide G;
(b) a nucleic acid sequence encoding an IL-4 receptor (IL-4R), said IL-4R comprising at position 75 as shown in SEQ ID NO: 2 a valine;
(c) a nucleic acid sequence comprising at least 15 nucleotides of the nucleic acid sequence of (a) or (b) and comprising the mutation as defined in (a) or (b);
(d) a nucleic acid sequence comprising a nucleotide sequence as shown in SEQ ID NO: 3 or a fragment of the sequence as shown in SEQ ID NO: 3, said fragment comprising the nucleotide sequence "gtc"/"guc" as shown in position 5758 to 5760 of SEQ ID NO: 3 and said fragment comprising at least 15 nucleotides;
(e) a nucleic acid sequence encoding a polypeptide comprising the amino acid sequence as shown in SEQ ID NO: 4; and
(f) a nucleic acid sequence comprising at least 12 nucleotides which hybridizes under stringent conditions to a nucleotide sequence defined in any one of (a) to (e) and having a mutation as defined in (a) or (b),

wherein said antibody or binding molecule detects an epitope comprising the amino acid stretch SEAHTCVPENN.

**8.** The use of claim 6 or 7, wherein said joint destruction is associated with early erosion(s) and/or a higher severity in the progression of rheumatoid arthritis (RA).

**9.** The use of claim 6 or 8, wherein said specific probe is selected from the group consisting of 5'-ACACGTGTATCCCTG-3' (SEQ ID NO: 5) or 5'-CACGTGTGTCCCTG-3' (SEQ ID NO: 6).

**10.** The use of claim 6, 8 or 9, wherein said primer is selected from the group consisting of 5'-ACCCAGCCCCTGTGTCT-3' (SEQ ID NO: 7) (forward primer) or 5'-CGCGCCTCCGTTGTTC-3' (SEQ ID NO: 8) (reverse primer).

**11.** Use of a kit comprising specific probes and/or primers for diagnosing and/or predicting erosive joint destruction in rheumatoid arthritis independent of HLA-DR shared epitope (SE), said probes and/or primers being capable of detecting at least one nucleic acid sequence selected from the group consisting of

(a) a nucleic acid sequence encoding an IL-4 receptor (IL-4R) which contains a mutation in position 465 of the nucleotide sequence of the wild-type IL4R as shown in SEQ ID NO: 1, whereby at said position the nucleotide A is replaced by the nucleotide G;
(b) a nucleic acid sequence encoding an IL-4 receptor (IL-4R), said IL-4R comprising at position 75 as shown in SEQ ID NO: 2 a valine;
(c) a nucleic acid sequence comprising at least 15 nucleotides of the nucleic acid sequence of (a) or (b) and comprising the mutation as defined in (a) or (b);
(d) a nucleic acid sequence comprising a nucleotide sequence as shown in SEQ ID NO: 3 or a fragment of the sequence as shown in SEQ ID NO: 3, said fragment comprising the nucleotide sequence "gtc"/"guc" as shown in position 5758 to 5760 of SEQ ID NO: 3 and said fragment comprising at least 15 nucleotides;
(e) a nucleic acid sequence encoding a polypeptide comprising the amino acid sequence as shown in SEQ ID NO: 4; and
(f) a nucleic acid sequence comprising at least 12 nucleotides which hybridizes under stringent conditions to a nucleotide sequence defined in any one of (a) to (e) and having a mutation as defined in (a) or (b).

**12.** Use of a kit comprising specific antibody molecules or specific binding molecules for diagnosing and/or predicting erosive joint destruction in rheumatoid arthritis independent of HLA-DR shared epitope (SE), wherein said specific antibody molecule or specific binding molecule is capable to specifically detect and/or bind to an epitope which is encoded by nucleic acid sequence selected from the group consisting of

(a) a nucleic acid sequence encoding an IL-4 receptor (IL-4R) which contains a mutation in position 465 of the nucleotide sequence of the wild-type IL4R as shown in SEQ ID NO: 1, whereby at said position the nucleotide A is replaced by the nucleotide G;
(b) a nucleic acid sequence encoding an IL-4 receptor (IL-4R), said IL-4R comprising at position 75 as shown in SEQ ID NO: 2 a valine;
(c) a nucleic acid sequence comprising at least 15 nucleotides of the nucleic acid sequence of (a) or (b) and comprising the mutation as defined in (a) or (b);
(d) a nucleic acid sequence comprising a nucleotide sequence as shown in SEQ ID NO: 3 or a fragment of the sequence as shown in SEQ ID NO: 3, said fragment comprising the nucleotide sequence "gtc"/"guc" as shown in position 5758 to 5760 of SEQ ID NO: 3 and said fragment comprising at least 15 nucleotides;
(e) a nucleic acid sequence encoding a polypeptide comprising the amino acid sequence as shown in SEQ ID NO: 4; and
(f) a nucleic acid sequence comprising at least 12 nucleotides which hybridizes under stringent conditions to a nucleotide sequence defined in any one of (a) to (e) and having a mutation as defined in (a) or (b),

wherein said antibody or binding molecule detects an epitope comprising the amino acid stretch SEAHTCVPENN.

**Patentansprüche**

**1.** Verfahren zum Diagnostizieren und/oder Prognostizieren erosiver Gelenkzerstörung bei rheumatoider Arthritis, umfassend, unabhängig von der Bestimmung des HLA-DR-Shared-Epitope (SE), das Bestimmen in einer Probe, die von einem Individuum erhalten wurde, des Vorliegens mindestens einer Nucleinsäuresequenz, die ausgewählt ist aus der Gruppe bestehend aus:

(a) einer Nucleinsäuresequenz, die einen IL-4-Rezeptor (IL-4R) codiert, der eine Mutation an Position 465 der Nucleotidsequenz des wie in SEQ ID NO:1 gezeigten Wildtyp-IL4R enthält, wodurch an dieser Position das Nucleotid A durch das Nucleotid G ersetzt ist;
(b) einer Nucleinsäuresequenz, die einen IL-4-Rezeptor (IL-4R) codiert, wobei der IL-4R an der wie in SEQ ID NO:2 gezeigten Position 75 ein Valin umfasst;
(c) einer Nucleinsäuresequenz, die mindestens 15 Nucleotide der Nucleinsäuresequenz von (a) oder (b) umfasst und die wie in (a) oder (b) definierte Mutation umfasst;
(d) einer Nucleinsäuresequenz, die eine wie in SEQ ID NO:3 gezeigte Nucleotidsequenz oder ein Fragment der wie in SEQ ID NO:3 gezeigten Sequenz umfasst, wobei das Fragment die wie an den Positionen 5758 bis 5760 der SEQ ID NO:3 gezeigte Nucleotidsequenz "gtc"/"guc" umfasst und das Fragment mindestens 15 Nucleotide umfasst;
(e) einer Nucleotidsequenz, die ein Polypeptid codiert, das die in SEQ ID NO: 4 gezeigte Aminosäuresequenz umfasst; und
(f) einer Nucleinsäuresequenz, die mindestens 12 Nucleotide umfasst, die unter stringenten Bedingungen an eine wie in einem von (a) bis (e) definierte Nucleotidsequenz hybridisiert und eine wie in (a) oder (b) definierte Mutation aufweist.

**2.** Verfahren zum Diagnostizieren und/oder Prognostizieren erosiver Gelenkzerstörung bei rheumatoider Arthritis, umfassend, unabhängig von der Bestimmung des HLA-DR-Shared-Epitope (SE), das Bestimmen in einer Probe, die von einem Individuum erhalten wurde, des Vorliegens mindestens eines codierten IL-4-Rezeptors (IL-4R), der an der homologen Position 75 des wie in SEQ ID NO:2 gezeigten IL-4-Rezeptors eine Mutation umfasst, wobei die Mutation den Austausch eines Isoleucins zu einem Valin umfasst.

**3.** Verfahren nach Anspruch 1, wobei die Mutation in homozygoten Allelen/Homozygotie nachgewiesen wird.

**4.** Verfahren nach einem der Ansprüche 1 bis 3, wobei die Gelenkzerstörung mit Erosion(en) und/oder einem höheren Schweregrad beim Fortschreiten der rheumatoiden Arthritis (RA) in Zusammenhang steht.

**5.** Verfahren nach einem der Ansprüche 1 bis 4, wobei das Verfahren PCR-Technik, Ligasekettenreaktion, NASBA, Restriktionsverdau, direkte Sequenzierung, Nucleinsäureamplifikationstechniken, MALDI, MALDI-TOF, Hybridisierungstechniken und/oder Immunassays umfasst.

**6.** Verwendung spezifischer Sonden und/oder Primer bei der *in vitro*-Diagnose und/oder Prognose erosiver Gelenkzerstörung bei rheumatoider Arthritis unabhängig von der Bestimmung des HLA-DR-Shared-Epitope (SE), wobei die Sonden und/oder Primer in der Lage sind, mindestens eine Nucleinsäuresequenz nachzuweisen, die ausgewählt ist aus der Gruppe bestehend aus

(a) einer Nucleinsäuresequenz, die einen IL-4-Rezeptor (IL-4R) codiert, der eine Mutation an Position 465 der Nucleotidsequenz des wie in SEQ ID NO:1 gezeigten Wildtyp-IL4R enthält, wodurch an dieser Position das Nucleotid A durch das Nucleotid G ersetzt ist;
(b) einer Nucleinsäuresequenz, die einen IL-4-Rezeptor (IL-4R) codiert, wobei der IL-4R an der wie in SEQ ID NO:2 gezeigten Position 75 ein Valin umfasst;
(c) einer Nucleinsäuresequenz, die mindestens 15 Nucleotide der Nucleinsäuresequenz von (a) oder (b) umfasst und die wie in (a) oder (b) definierte Mutation umfasst;
(d) einer Nucleinsäuresequenz, die eine wie in SEQ ID NO:3 gezeigte Nucleotidsequenz oder ein Fragment der wie in SEQ ID NO:3 gezeigten Sequenz umfasst, wobei das Fragment die wie an den Positionen 5758 bis 5760 der SEQ ID NO:3 gezeigte Nucleotidsequenz "gtc"/"guc" umfasst und das Fragment mindestens 15 Nucleotide umfasst;
(e) einer Nucleinsäuresequenz, die ein Polypeptid codiert, das die wie in SEQ ID NO:4 gezeigte Aminosäuresequenz umfasst; und
(f) einer Nucleinsäuresequenz, die mindestens 12 Nucleotide umfasst, die unter stringenten Bedingungen an eine wie in einem von (a) bis (e) definierte Nucleotidsequenz hybridisiert und eine wie in (a) und (b) definierte Mutation aufweist.

**7.** Verwendung spezifischer Antikörpermoleküle oder spezifischer Bindemoleküle bei der *in vitro*-Diagnose und/oder Prognose erosiver Gelenkzerstörung bei rheumatoider Arthritis unabhängig von der Bestimmung des HLA-DR-Shared-Epitope (SE), wobei das spezifische Antikörpermolekül oder das spezifische Bindemolekül in der Lage ist, ein Epitop spezifisch zu erkennen und/oder spezifisch an ein Epitop zu binden, das von einer Nucleinsäuresequenz codiert wird, die ausgewählt ist aus der Gruppe bestehend aus

(a) einer Nucleinsäuresequenz, die einen IL-4-Rezeptor (IL-4R) codiert, der eine Mutation an Position 465 der Nucleotidsequenz des wie in SEQ ID NO:1 gezeigten Wildtyp-IL4R enthält, wodurch an dieser Position das Nucleotid A durch das Nucleotid B ersetzt ist;
(b) einer Nucleinsäuresequenz, die einen IL-4-Rezeptor (IL-4R) codiert, wobei der IL-4R an der wie in SEQ ID NO:2 gezeigten Position 75 ein Valin umfasst;
(c) einer Nucleinsäuresequenz, die mindestens 15 Nucleotide der Nucleinsäuresequenz von (a) oder (b) umfasst und eine wie in (a) oder (b) definierte Mutation umfasst;
(d) einer Nucleinsäuresequenz, die eine wie in SEQ ID NO:3 gezeigte Nucleotidsequenz oder ein Fragment der wie in SEQ ID NO:3 gezeigten Sequenz umfasst, wobei das Fragment die wie an den Positionen 5758 bis 5760 der SEQ ID NO:3 gezeigte Nucleotidsequenz "gtc"/"guc" umfasst und wobei das Fragment mindestens 15 Nucleotide umfasst;
(e) einer Nucleinsäuresequenz, die ein Polypeptid codiert, das die wie in SEQ ID NO:4 gezeigte Aminosäuresequenz umfasst; und
(f) einer Nucleinsäuresequenz, die mindestens 12 Nucleotide umfasst, die unter stringenten Bedingungen an eine wie in einem von (a) bis (e) definierte Nucleotidsequenz hybridisiert und eine wie in (a) oder (b) definierte Mutation aufweist,

wobei der Antikörper oder das Bindemolekül ein Epitop erkennt, das den Aminosäureabschnitt SEAHTCVPENN umfasst.

**8.** Verwendung nach Anspruch 6 oder 7, wobei die Gelenkzerstörung mit früher Erosion/frühen Erosionen und/oder einem höheren Schweregrad beim Fortschreiten der rheumatoiden Arthritis (RA) in Zusammenhang steht.

**9.** Verwendung nach Anspruch 6 oder 8, wobei die spezifische Sonde aus der Gruppe bestehend aus 5'-ACACGTGTATCCCTG-3' (SEQ ID NO:5) oder 5'-CACGTGTGTCCCTG-3' (SEQ ID NO:6) ausgewählt ist.

**10.** Verwendung nach Anspruch 6, 8 oder 9, wobei der Primer aus der Gruppe bestehend aus 5'-ACCCAGCCCCTGTGTCT-3' (SEQ ID NO:7) (Vorwärts-Primer) oder 5'-CGCGCCTCCGTTGTTC-3' (SEQ ID NO:8) (Rückwärts-Primer) ausgewählt ist.

**11.** Verwendung eines Kits, der spezifische Sonden und/oder Primer umfasst, für das Diagnostizieren und/oder Prognostizieren erosiver Gelenkzerstörung bei rheumatoider Arthritis unabhängig von der Bestimmung des HLA-DR-Shared-Epitope (SE), wobei die Sonden und/oder Primer in der Lage sind, mindestens eine Nucleinsäuresequenz nachzuweisen, die ausgewählt ist aus der Gruppe bestehend aus

(a) einer Nucleinsäuresequenz, die einen IL-4-Rezeptor (IL-4R) codiert, der eine Mutation an Position 465 der Nucleotidsequenz des wie in SEQ ID NO:1 gezeigten Wildtyp-IL4R enthält, wodurch an dieser Position das Nucleotid A durch das Nucleotid G ersetzt ist;
(b) einer Nucleinsäuresequenz, die einen IL-4-Rezeptor (IL-4R) codiert, wobei der IL-4R an der wie in SEQ ID NO:2 gezeigten Position 75 ein Valin umfasst;
(c) einer Nucleinsäuresequenz, die mindestens 15 Nucleotide der Nucleinsäuresequenz von (a) oder (b) umfasst und die wie in (a) oder (b) definierte Mutation umfasst;
(d) einer Nucleinsäuresequenz, die eine wie in SEQ ID NO:3 gezeigte Nucleotidsequenz oder ein Fragment der wie in SEQ ID NO:3 gezeigten Sequenz umfasst, wobei das Fragment die wie an den Positionen 5758 bis 5760 der SEQ ID NO:3 gezeigte Nucleotidsequenz "gtc"/"guc" umfasst und das Fragment mindestens 15 Nucleotide umfasst;
(e) einer Nucleinsäuresequenz, die ein Polypeptid codiert, das die wie in SEQ ID NO:4 gezeigte Aminosäuresequenz umfasst; und
(f) einer Nucleinsäuresequenz, die mindestens 12 Nucleotide umfasst, die unter stringenten Bedingungen an eine wie in einem von (a) bis (e) definierte Nucleotidsequenz hybridisiert und eine wie in (a) oder (b) definierte Mutation aufweist.

**12.** Verwendung eines Kits, das spezifische Antikörpermoleküle oder spezifische Bindemoleküle umfasst, zum Diagnostizieren und/oder Prognostizieren erosiver Gelenkzerstörung bei rheumatoider Arthritis unabhängig von der Bestimmung des HLA-DR-Shared-Epitope (SE), wobei das spezifische Antikörpermolekül oder das spezifische Bindemolekül in der Lage ist, ein Epitop spezifisch zu erkennen und/oder spezifisch an ein Epitop zu binden, das von einer Nucleinsäuresequenz codiert wird, die ausgewählt ist aus der Gruppe bestehend aus

(a) einer Nucleinsäuresequenz, die einen IL-4-Rezeptor (IL-4R) codiert, der eine Mutation an Position 465 der Nucleotidsequenz des wie in SEQ ID NO:1 gezeigten Wildtyp-IL4R enthält, wodurch an dieser Position das Nucleotid A durch das Nucleotid G ersetzt ist;
(b) einer Nucleinsäuresequenz, die einen IL-4-Rezeptor (IL-4R) codiert, wobei der IL-4R an der wie in SEQ ID NO:2 gezeigten Position 75 ein Valin umfasst;
(c) einer Nucleinsäuresequenz, die mindestens 15 Nucleotide der Nucleinsäuresequenz von (a) oder (b) umfasst und die wie in (a) oder (b) definierte Mutation umfasst;
(d) einer Nucleinsäuresequenz, die eine wie in SEQ ID NO:3 gezeigte Nucleotidsequenz oder ein Fragment der wie in SEQ ID NO:3 gezeigten Sequenz umfasst, wobei das Fragment die wie an den Positionen 5758 bis 5760 der SEQ ID NO:3 gezeigte Nucleotidsequenz "gtc"/"guc" umfasst und das Fragment mindestens 15 Nucleotide umfasst;
(e) einer Nucleinsäuresequenz, die ein Polypeptid codiert, das die wie in SEQ ID NO:4 gezeigte Aminosäuresequenz umfasst; und
(f) einer Nucleinsäuresequenz, die mindestens 12 Nucleotide umfasst, die unter stringenten Bedingungen an eine wie in einem von (a) bis (e) definierte Nucleotidsequenz hybridisiert und eine wie in (a) oder (b) definierte Mutation aufweist,

wobei der Antikörper oder das Bindemolekül ein Epitop detektiert, das den Aminosäureabschnitt SEAHTCVPENN umfasst.

## Revendications

**1.** Procédé de diagnostic et/ou de prédiction d'une destruction érosive des articulations dans la polyarthrite rhumatoïde comprenant la détermination dans un échantillon obtenu d'un individu indépendante de l'épitope partagé (EP) HLA-DR de la présence d'au moins une séquence d'acide nucléique choisie dans le groupe constitué de :

(a) une séquence d'acide nucléique codant pour un récepteur de l'IL-4 (IL-4R) qui contient une mutation en position 465 de la séquence nucléotidique de l'IL4R de type sauvage comme il est montré par SEQ ID NO : 1, par laquelle au niveau de ladite position le nucléotide A est remplacé par le nucléotide G ;
(b) une séquence d'acide nucléique codant pour un récepteur de l'IL-4 (IL-4R), ledit IL-4R comprenant au niveau de la position 75 comme il est montré par SEQ ID NO : 2 une valine ;
(c) une séquence d'acide nucléique comprenant au moins 15 nucléotides de la séquence d'acide nucléique de (a) ou (b) et comprenant la mutation telle que définie en (a) ou (b) ;
(d) une séquence d'acide nucléique comprenant une séquence nucléotidique comme il est montré par SEQ ID NO : 3 ou un fragment de la séquence comme il est montré par SEQ ID NO : 3, ledit fragment comprenant la séquence nucléotidique « gtc »/« guc » comme il est montré en position 5758 à 5760 de SEQ ID NO : 3 et ledit fragment comprenant au moins 15 nucléotides ;
(e) une séquence d'acide nucléique codant pour un polypeptide comprenant la séquence d'acides aminés comme il est montré par SEQ ID NO : 4 ; et
(f) une séquence d'acide nucléique comprenant au moins 12 nucléotides qui s'hybride dans des conditions stringentes à une séquence nucléotidique définie dans l'un quelconque de (a) à (e) et comportant une mutation telle que définie en (a) ou (b).

**2.** Procédé de diagnostic et/ou de prédiction d'une destruction érosive des articulations dans la polyarthrite rhumatoïde comprenant la détermination dans un échantillon obtenu d'un individu indépendante de l'épitope partagé (EP) HLA-DR de la présence d'au moins un récepteur de l'IL-4 (IL-4R) codé qui comprend au niveau de la position homologue 75 du récepteur de l'IL-4 comme il est illustré par SEQ ID NO : 2 une mutation, ladite mutation comprenant l'échange d'une isoleucine en une valine.

**3.** Procédé selon la revendication 1, dans lequel ladite mutation est détectée dans des allèles homozygotes/l'homozygosité.

**4.** Procédé selon l'une quelconque des revendications 1 à 3, dans lequel ladite destruction des articulations est associée à une ou plusieurs érosions et/ou une gravité supérieure de la progression de la polyarthrite rhumatoïde (PA).

**5.** Procédé selon l'une quelconque des revendications 1 à 4, ledit procédé comprenant la technologie de la PCR, la réaction en chaîne de la ligase, NASBA, la digestion par restriction, le séquençage direct, des techniques d'amplification d'acide nucléique, MALDI, MALDI-TOF, des techniques d'hybridation et/ou des tests immunologiques.

**6.** Utilisation de sondes et/ou d'amorces spécifiques dans le diagnostic et/ou la prédiction *in vitro* d'une destruction érosive des articulations dans la polyarthrite rhumatoïde indépendante de l'épitope partagé (EP) HLA-DR, où lesdites sondes et/ou amorces sont capables de détecter au moins une séquence d'acide nucléique choisie dans le groupe constitué de

(a) une séquence d'acide nucléique codant pour un récepteur de l'IL-4 (IL-4R) qui contient une mutation en position 465 de la séquence nucléotidique de l'IL4R de type sauvage comme il est montré par SEQ ID NO : 1, par laquelle au niveau de ladite position le nucléotide A est remplacé par le nucléotide G ;
(b) une séquence d'acide nucléique codant pour un récepteur de l'IL-4 (IL-4R), ledit IL-4R comprenant au niveau de la position 75 comme il est montré par SEQ ID NO : 2 une valine ;
(c) une séquence d'acide nucléique comprenant au moins 15 nucléotides de la séquence d'acide nucléique de (a) ou (b) et comprenant la mutation telle que définie en (a) ou (b) ;
(d) une séquence d'acide nucléique comprenant une séquence nucléotidique comme il est montré par SEQ ID NO : 3 ou un fragment de la séquence comme il est montré par SEQ ID NO : 3, ledit fragment comprenant la séquence nucléotidique « gtc »/« guc » comme il est montré en position 5758 à 5760 de SEQ ID NO : 3 et ledit fragment comprenant au moins 15 nucléotides ;
(e) une séquence d'acide nucléique codant pour un polypeptide comprenant la séquence d'acides aminés comme il est montré par SEQ ID NO : 4 ; et
(f) une séquence d'acide nucléique comprenant au moins 12 nucléotides qui s'hybride dans des conditions stringentes à une séquence nucléotidique définie dans l'un quelconque de (a) à (e) et comportant une mutation telle que définie en (a) ou (b).

**7.** Utilisation de molécules d'anticorps spécifiques ou de molécules de liaison spécifique dans le diagnostic et/ou la prédiction *in vitro* d'une destruction érosive des articulations dans la polyarthrite rhumatoïde indépendante de l'épitope partagé (EP) HLA-DR, où ladite molécule d'anticorps spécifique ou molécule de liaison spécifique est capable de détecter et/ou de se lier spécifiquement à un épitope qui est codé par une séquence d'acide nucléique choisie dans le groupe constitué de

(a) une séquence d'acide nucléique codant pour un récepteur de l'IL-4 (IL-4R) qui contient une mutation en position 465 de la séquence nucléotidique de l'IL4R de type sauvage comme il est montré par SEQ ID NO : 1, par laquelle au niveau de ladite position le nucléotide A est remplacé par le nucléotide G ;
(b) une séquence d'acide nucléique codant pour un récepteur de l'IL-4 (IL-4R), ledit IL-4R comprenant au niveau de la position 75 comme il est montré par SEQ ID NO : 2 une valine ;
(c) une séquence d'acide nucléique comprenant au moins 15 nucléotides de la séquence d'acide nucléique de (a) ou (b) et comprenant la mutation telle que définie en (a) ou (b) ;
(d) une séquence d'acide nucléique comprenant une séquence nucléotidique comme il est montré par SEQ ID NO : 3 ou un fragment de la séquence comme il est montré par SEQ ID NO : 3, ledit fragment comprenant la séquence nucléotidique « gtc »/« guc » comme il est montré en position 5758 à 5760 de SEQ ID NO : 3 et ledit fragment comprenant au moins 15 nucléotides ;
(e) une séquence d'acide nucléique codant pour un polypeptide comprenant la séquence d'acides aminés comme il est montré par SEQ ID NO : 4 ; et
(f) une séquence d'acide nucléique comprenant au moins 12 nucléotides qui s'hybride dans des conditions stringentes à une séquence nucléotidique définie dans l'un quelconque de (a) à (e) et comportant une mutation telle que définie en (a) ou (b),

où ledit anticorps ou molécule de liaison détecte un épitope comprenant la séquence d'acides aminés SEAHTCVPENN.

**8.** Utilisation selon la revendication 6 ou 7, où ladite destruction des articulations est associée à une ou plusieurs érosions précoces et/ou une gravité supérieure de la progression de la polyarthrite rhumatoïde (PA).

**9.** Utilisation selon la revendication 6 ou 8, où ladite sonde spécifique est choisie dans le groupe constitué de 5'-ACACGTGTATCCCTG-3' (SEQ ID NO : 5) ou 5'-CACGTGTGTCCCTG-3' (SEQ ID NO : 6).

**10.** Utilisation selon la revendication 6, 8 ou 9, où ladite amorce est choisie dans le groupe constitué de 5'-ACCCAGCCCCTGTGTCT-3' (SEQ ID NO : 7) (amorce directe) ou 5'-CGCGCCTCCGTTGTTC-3' (SEQ ID NO : 8) (amorce inverse).

**11.** Utilisation d'un kit comprenant des sondes et/ou des amorces spécifiques pour le diagnostic et/ou la prédiction d'une destruction érosive des articulations dans la polyarthrite rhumatoïde indépendante de l'épitope partagé (EP) HLA-DR, lesdites sondes et/ou amorces étant capables de détecter au moins une séquence d'acide nucléique choisie dans le groupe constitué de

(a) une séquence d'acide nucléique codant pour un récepteur de l'IL-4 (IL-4R) qui contient une mutation en position 465 de la séquence nucléotidique de l'IL4R de type sauvage comme il est montré par SEQ ID NO : 1, par laquelle au niveau de ladite position le nucléotide A est remplacé par le nucléotide G ;
(b) une séquence d'acide nucléique codant pour un récepteur de l'IL-4 (IL-4R), ledit IL-4R comprenant au niveau de la position 75 comme il est montré par SEQ ID NO : 2 une valine ;
(c) une séquence d'acide nucléique comprenant au moins 15 nucléotides de la séquence d'acide nucléique de (a) ou (b) et comprenant la mutation telle que définie en (a) ou (b) ;
(d) une séquence d'acide nucléique comprenant une séquence nucléotidique comme il est montré par SEQ ID NO : 3 ou un fragment de la séquence comme il est montré par SEQ ID NO : 3, ledit fragment comprenant la séquence nucléotidique « gtc »/« guc » comme il est montré en position 5758 à 5760 de SEQ ID NO : 3 et ledit fragment comprenant au moins 15 nucléotides ;
(e) une séquence d'acide nucléique codant pour un polypeptide comprenant la séquence d'acides aminés comme il est montré par SEQ ID NO : 4 ; et
(f) une séquence d'acide nucléique comprenant au moins 12 nucléotides qui s'hybride dans des conditions stringentes à une séquence nucléotidique définie dans l'un quelconque de (a) à (e) et comportant une mutation telle que définie en (a) ou (b).

**12.** Utilisation d'un kit comprenant des molécules d'anticorps spécifiques ou des molécules de liaison spécifique pour le diagnostic et/ou la prédiction d'une destruction érosive des articulations dans la polyarthrite rhumatoïde indépendante de l'épitope partagé (EP) HLA-DR, où ladite molécule d'anticorps spécifique ou molécule de liaison spécifique est capable de détecter et/ou de se lier spécifiquement à un épitope qui est codé par une séquence d'acide nucléique choisie dans le groupe constitué de

(a) une séquence d'acide nucléique codant pour un récepteur de l'IL-4 (IL-4R) qui contient une mutation en position 465 de la séquence nucléotidique de l'IL4R de type sauvage comme il est montré par SEQ ID NO : 1, par laquelle au niveau de ladite position le nucléotide A est remplacé par le nucléotide G ;
(b) une séquence d'acide nucléique codant pour un récepteur de l'IL-4 (IL-4R), ledit IL-4R comprenant au niveau de la position 75 comme il est montré par SEQ ID NO : 2 une valine ;
(c) une séquence d'acide nucléique comprenant au moins 15 nucléotides de la séquence d'acide nucléique de (a) ou (b) et comprenant la mutation telle que définie en (a) ou (b) ;
(d) une séquence d'acide nucléique comprenant une séquence nucléotidique comme il est montré par SEQ ID NO : 3 ou un fragment de la séquence comme il est montré

# Figure 1a

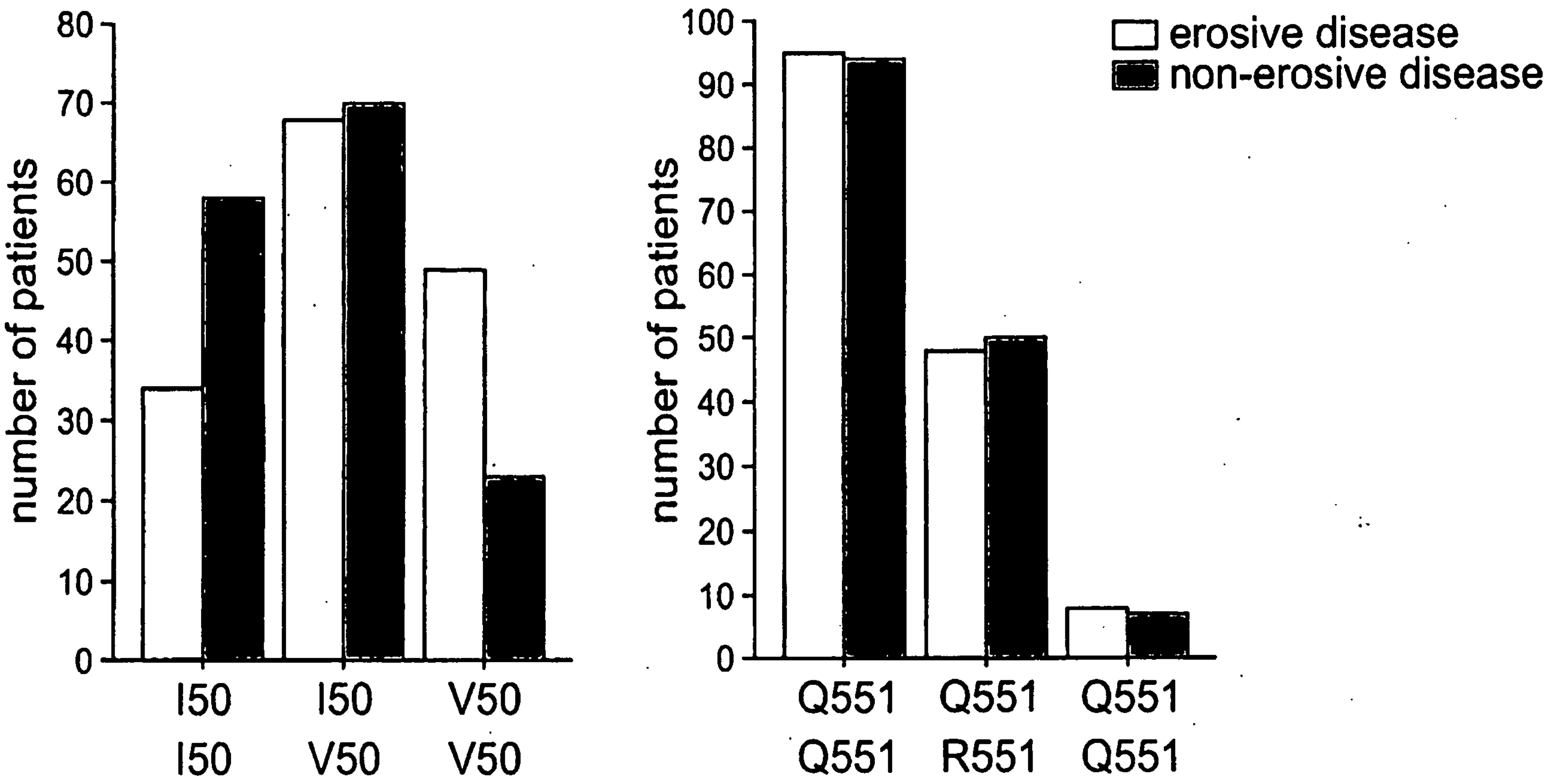
number of patients
80
70
60
50
40
30
20
10
0
I50 I50
I50 V50
V50 V50
number of patients
100
90
80
70
60
50
40
30
20
10
0
Q551 Q551
Q551 R551
Q551 Q551
erosive disease
non-erosive disease

Figure 1b

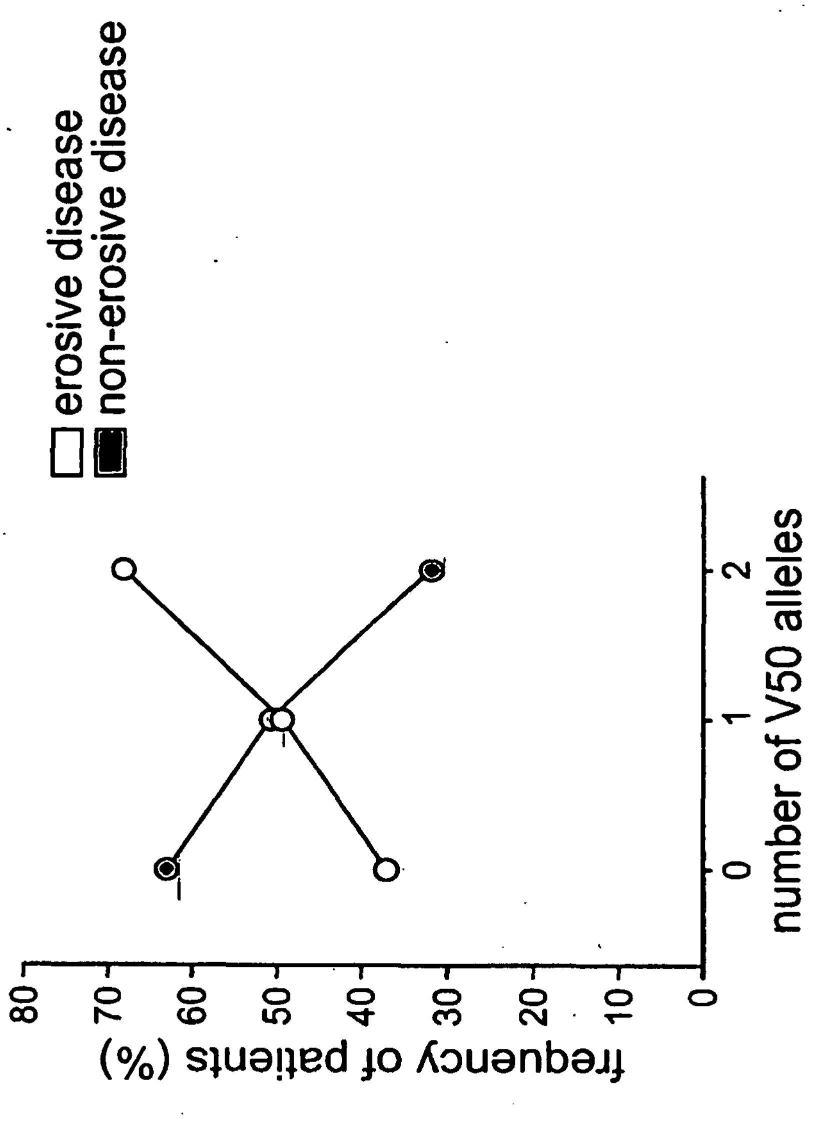
erosive disease
non-erosive disease
frequency of patients (%)
80
70
60
50
40
30
20
10
0
0
1
2
number of V50 alleles

Figure 2a

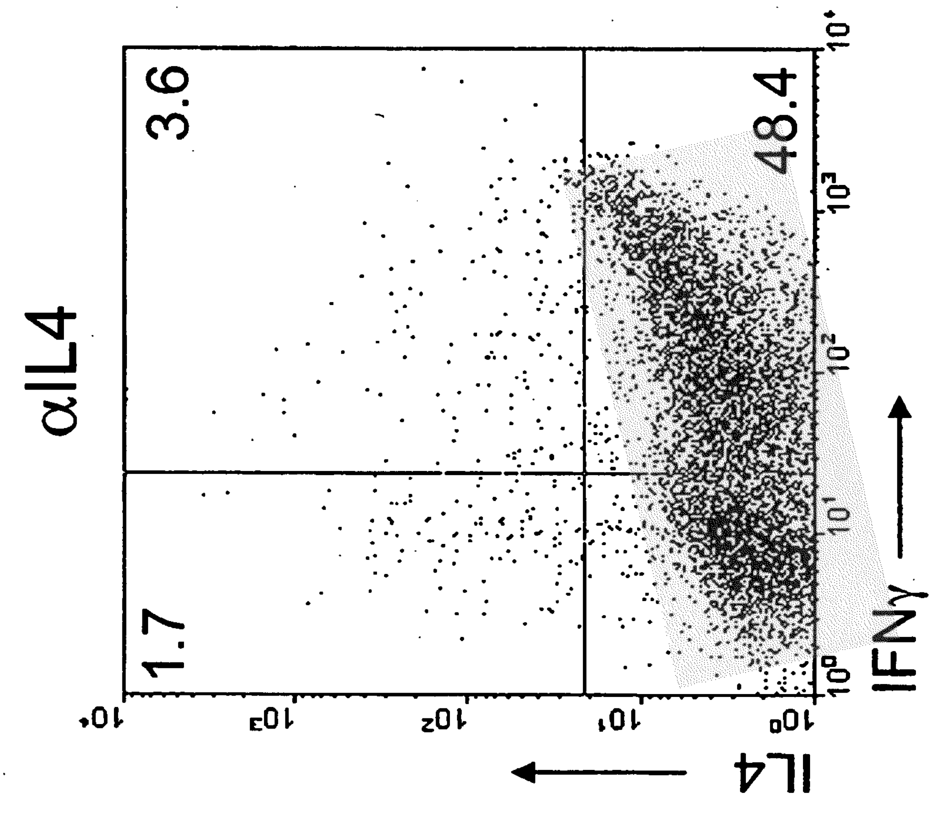

# Figure 2b

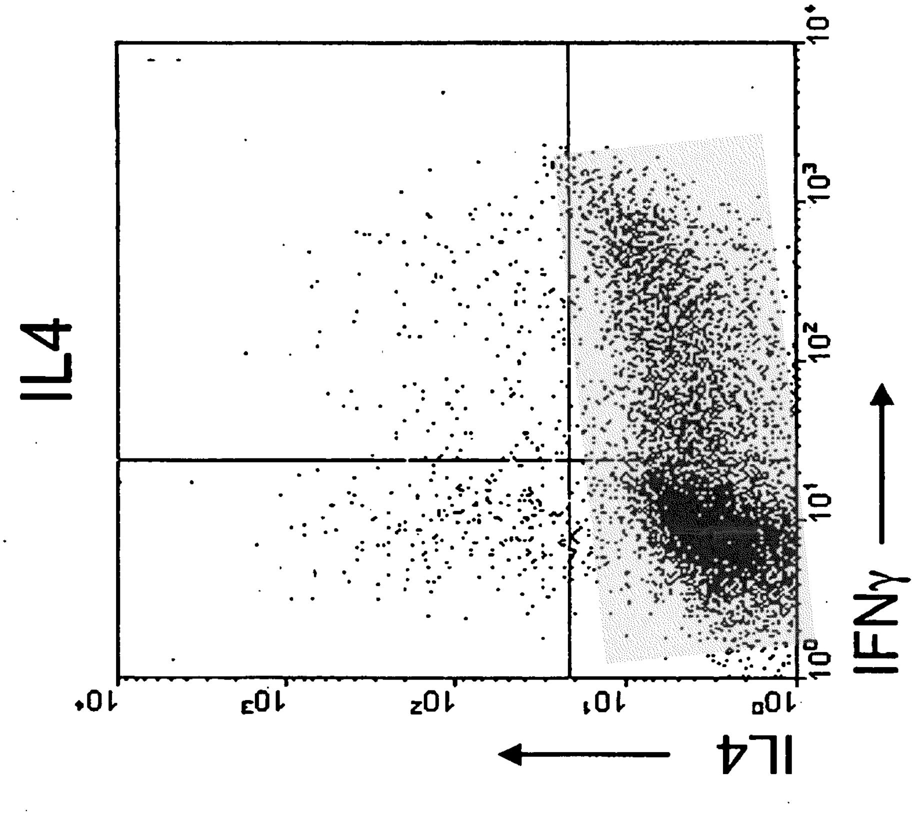

Figure 2c

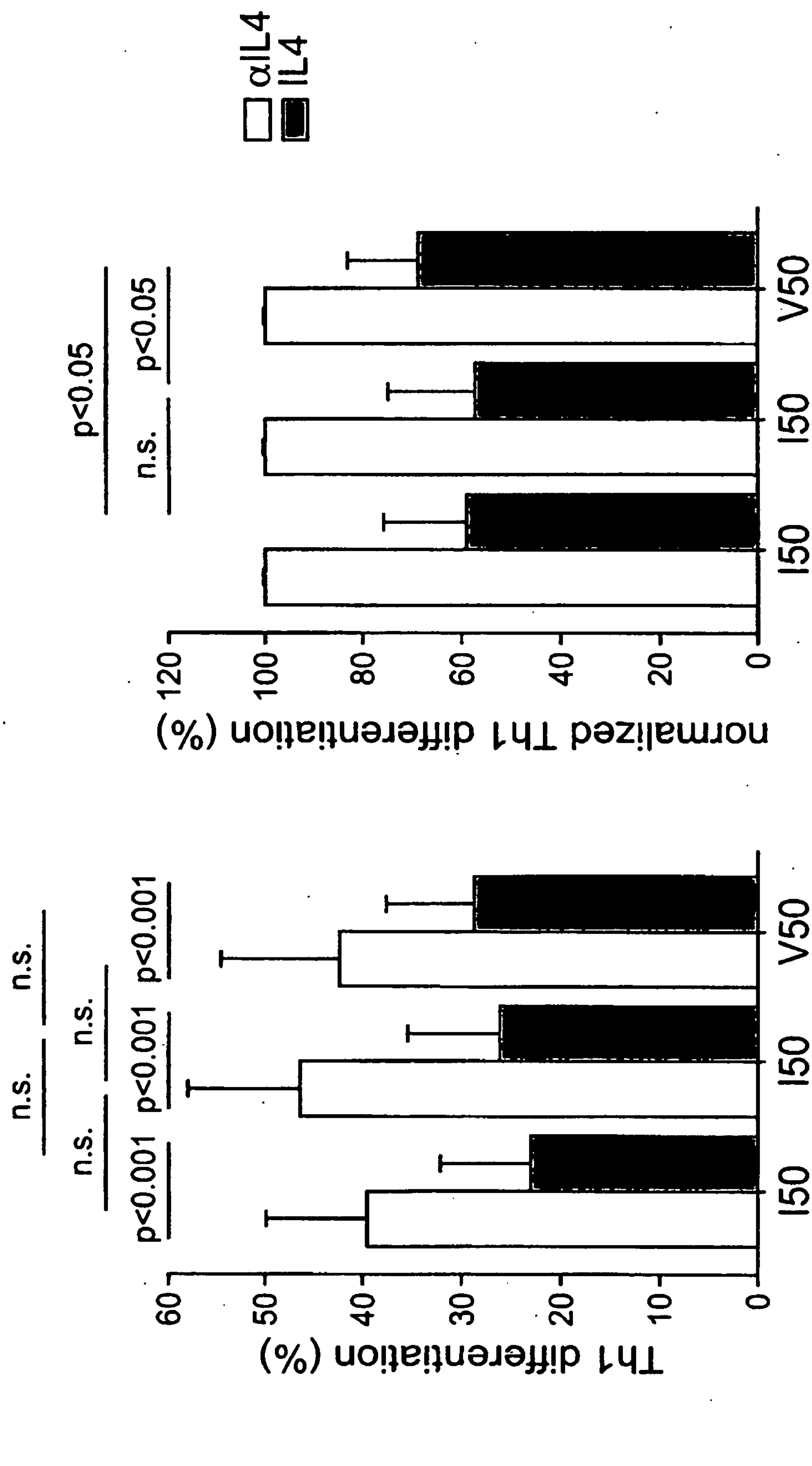
Th1 differentiation (%)
0
10
20
30
40
50
60
I50 I50
I50 V50
V50 V50
p<0.001
p<0.001
p<0.001
n.s.
n.s.
n.s.
n.s.
normalized Th1 differentiation (%)
0
20
40
60
80
100
120
p<0.05
n.s.
p<0.05
αIL4
IL4

Figure 3a

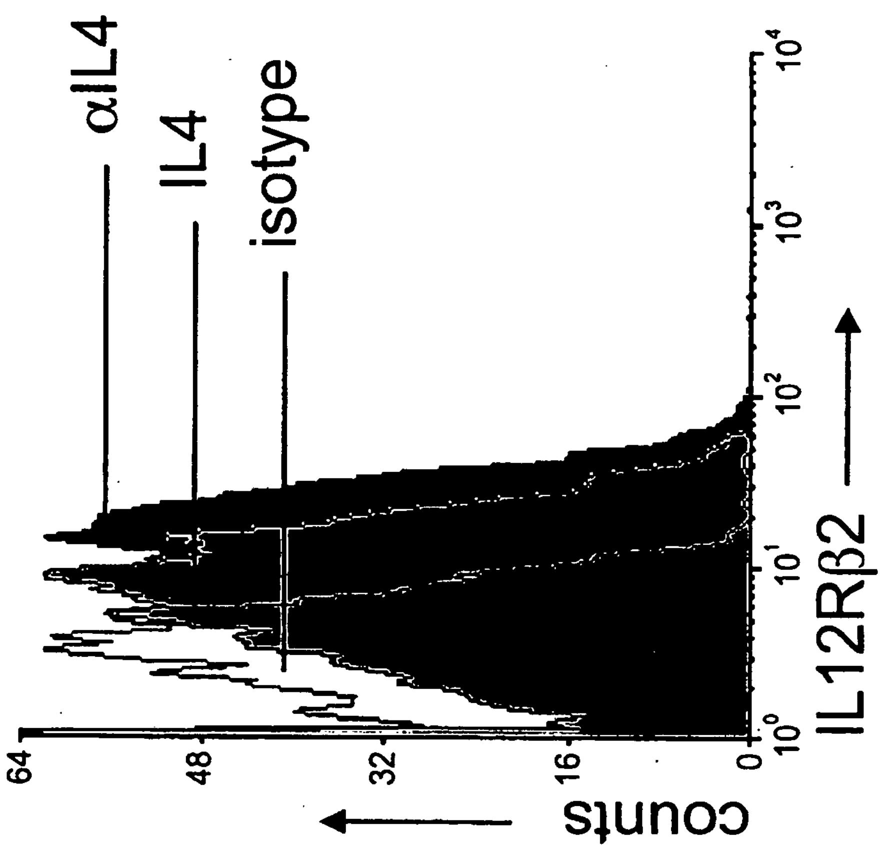
αIL4
IL4
isotype
counts
64
48
32
16
0
$10^0$
$10^1$
$10^2$
$10^3$
$10^4$
IL12Rβ2

Figure 3b

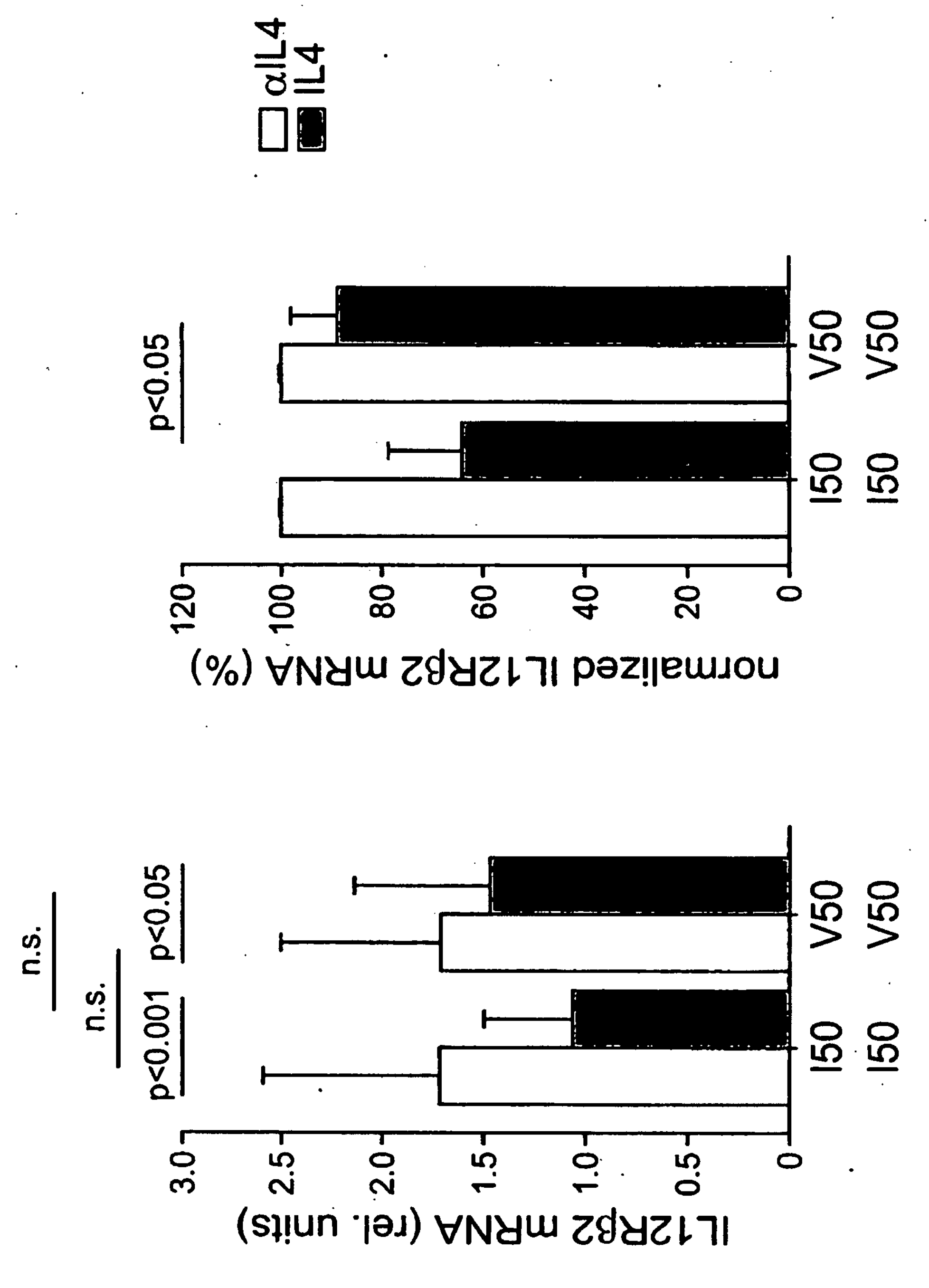
IL12Rβ2 mRNA (rel. units)
3.0
2.5
2.0
1.5
1.0
0.5
0
p<0.001
p<0.05
n.s.
n.s.
I50
V50
I50
V50
normalized IL12Rβ2 mRNA (%)
120
100
80
60
40
20
0
p<0.05
I50
V50
I50
V50
αIL4
IL4

# Figure 3c

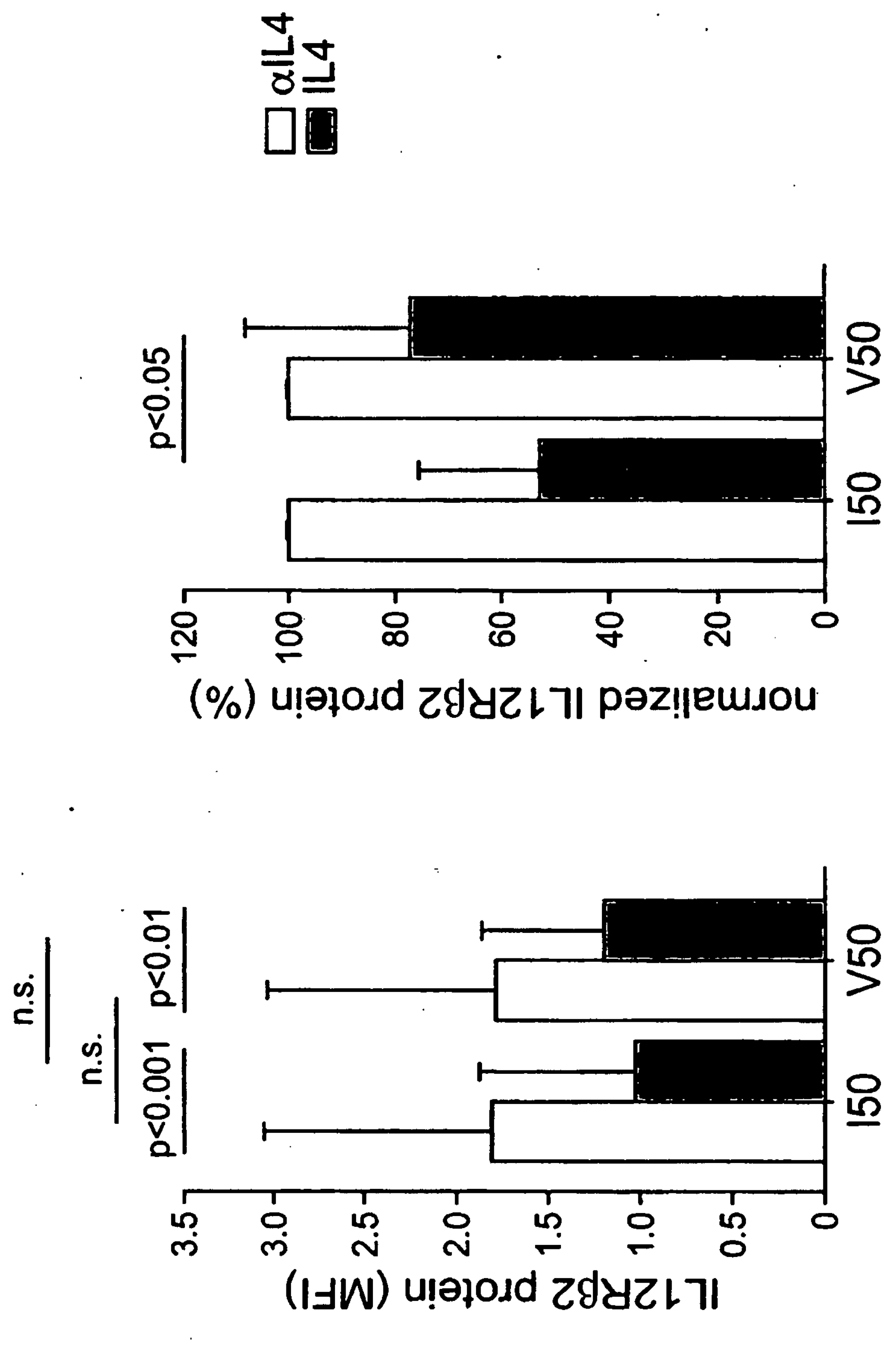

αIL4
IL4
IL12Rβ2 protein (MFI)
3.5
3.0
2.5
2.0
1.5
1.0
0.5
0
n.s.
n.s.
p<0.001
p<0.01
I50 I50
V50 V50
normalized IL12Rβ2 protein (%)
120
100
80
60
40
20
0
p<0.05
I50 I50
V50 V50

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **FIRESTEIN.** *Nature,* 2003, vol. 423 (6937), 356-361
- **AHO.** *J Rheumatol,* 1986, vol. 13 (5), 899-902
- **SILMAN.** *Br J Rheumatol,* 1993, vol. 32 (10), 903-907
- **DEIGHTON.** *Ann Rheum Dis,* 1991, vol. 50 (1), 62-65
- **MACGREGOR.** *Arthritis Rheum,* 2000, vol. 43 (1), 30-37
- **SELDIN.** *Arthritis Rheum,* 1999, vol. 42 (6), 1071-1079
- **DEIGHTON.** *Clin Genet,* 1989, vol. 36 (3), 178-182
- **COMELIS.** *Proc Natl Acad Sci U S A,* 1998, vol. 95 (18), 10746-10750
- **JAWAHEER.** *Am J Hum Genet,* 2001, vol. 68 (4), 927-936
- **MACKAY.** *Arthritis Rheum,* 2002, vol. 46 (3), 632-639
- **SHIOZAWA.** *Int Immunol,* 1998, vol. 10 (12), 1891-1895
- **SKAPENKO.** *J Immunol,* 1999, vol. 163, 491-499
- **SIMON.** *Proc Natl Acad Sci USA,* 1994, vol. 91, 8562-8566
- **DEICHMANN.** *Biochem Biophys Res Commun,* 1997, vol. 231 (3), 696-697
- **KRUSE.** *Immunology,* 1999, vol. 96 (3), 365-371
- **HERSHEY.** *N Engl J Med,* 1997, vol. 337 (24), 1720-1725
- **MITSUYASU.** *Nat Genet,* 1998, vol. 19 (2), 119-120
- **MITSUYASU.** *J Immunol,* 1999, vol. 162 (3), 1227-1231
- **RISMA.** *J Immunol,* 2002, vol. 169 (3), 1604-1610
- **STEPHENSON.** *J Immunol,* 2004, vol. 173 (7), 4523-4528
- **WANG.** *J Immunol,* 1999, vol. 162 (8), 4385-4389
- **KR.** *Immunology,* 1999, vol. 96 (3), 365-371
- **MIREL.** *Diabetes,* 2002, vol. 51 (11), 3336-3341
- **BUGAWAN.** *Am J Hum Genet,* 2003, vol. 72 (6), 1505-1514
- **KANEMITSU.** *Arthritis Rheum,* 1999, vol. 42 (6), 1298-1300
- **LEE.** *Lancet,* 2001, vol. 358 (9285), 903-911
- **O'DELL.** *Arthritis Rheum,* 2002, vol. 46 (2), 283-285
- **DE RYCKE.** *Ann Rheum Dis,* 2004, vol. 63 (12), 1587-1593
- **THOMSON.** *Arthritis Rheum,* 1999, vol. 42 (4), 757-762
- **GORMAN.** *Arthritis Rheum,* 2004, vol. 50 (2), 400-412
- **KANIK.** *J Rheumatol,* 1998, vol. 25 (1), 16-22
- **VAN DER GRAAFF.** *Lancet,* 1998, vol. 351 (9120), 1931
- **ARNETT.** *Arthritis Rheum,* 1988, vol. 31, 315-324
- **MACHOLD.** *Rheumatology,* 09 August 2006
- **RODAK.** Haematology: Clinical Principles & Applications. WB Saunders Co, 2002
- **BRUNZEL.** Fundamentals of Urine and Body Fluids Analysis. WB Saunders Co, 1994
- Cerebrospinal Fluid. Kluwer Academic Pub, 1989
- **SAMBROOK et al.** Molecular Cloning: A Laboratory Manual. Cold Spring Harbor Laboratory
- Protocols for Oligonucleotides and Analogs: Synthesis and Properties. Methods in Molecular Biology, 20. Humana Press, 1993
- PCR Applications: Protocols for Functional Genomics. Academic Press, 1999
- PCR Cloning Protocols: From Molecular Cloning to Genetic. Humana Press, 2002
- Automated DNA Sequencing and Analysis. Academic Press, 1994
- **ALPHEY.** DNA Sequencing: From Experimental Methods to Bioinformatics. Springer Verlag Publishing, 1997
- **NOLLAU et al.** *Clin. Chem.,* 1997, vol. 43, 1114-1128
- Polymorphism Detection & Analysis Techniques. Eaton Pub Co, 2000
- Mutation Detection: A Practical Approach. Irl Press, 1998
- Laboratory Methods for the Detection of Mutations and Polymorphisms in DNA. CRC Press, 1997
- **BOTSTEIN.** *Am J Hum Genet,* 1980, vol. 32, 314-31
- **ORITA.** *PNAS,* 1989, vol. 86, 2766-2770
- **SAIKI.** *PNAS,* 1989, vol. 86, 6230-6234
- **VERPY.** *PNAS,* 1994, vol. 91, 1873-1877
- **BONNET.** *PNAS,* 1999, vol. 96, 6171-6176
- **MARRAS.** *Genet Anal,* 1999, vol. 14, 151-156

- **LIVAK.** *Genet Anal,* 1999, vol. 14, 143-149
- **GAYLORD.** *PNAS,* 2005, vol. 102, 34-39
- **WANG.** *Science,* 1998, vol. 280, 1077-1082
- **TARANENKO.** *Genet Anal,* 1996, vol. 13, 87-94
- **BALL.** *Protein Sci,* 1998, vol. 7, 758-764
- **HARLOW ; LANE.** Antibodies: A Laboratory Manual. Cold Spring Harbor Laboratory Press, 1988
- **ALTSCHUL.** *Nucl. Acids Res.,* 1997, vol. 25, 3389-3402
- **ALTSCHUL.** *J. Mol. Evol.,* 1993, vol. 36, 290-300
- **ALTSCHUL.** *J. Mol. Biol.,* 1990, vol. 215, 403-410
- **THOMPSON.** *Nucl. Acids Res.,* 1994, vol. 2, 4673-4680
- *Brutlag Comp. App. Biosci.,* 1990, vol. 6, 237-245
- **SAMBROOK ; RUSSELL.** Molecular Cloning, A Laboratory Manual. Cold Spring Harbor Laboratory, 2001
- **AUSUBEL.** Current Protocols in Molecular Biology. Green Publishing Associates and Wiley Interscience, 1989
- Nucleic acid hybridization, a practical approach. IRL Press, 1985
- **ALTSCHUL.** *Nucl. Acids Res.,* 1977, vol. 25, 3389-3402
- **HENIKOFF.** *Proc. Natl. Acad. Sci., USA,* 1989, vol. 89, 10915
- **TIJSSEN.** Practice and theory of enzyme immunoassays. 1985, vol. 15
- Basic methods in molecular biology. Elsevier, 1990
- Immunochemical methods in cell and molecular biology. Academic Press, 1987
- Methods in Enzymology. Academic Press, Inc,
- **FRASER, C G.** Interpretation of Clinical Chemistry Laboratory Data. Blackwell, 1986
- **MAYNE, P D.** Clinical Chemistry in Diagnosis and Treatment. 1994
- **MARSHALL, W J.** Clinical Chemistry. Gower, 1996
- **PRICE, C P.** Principles and Practice of Immunoassay. Stockton Press, 1990
- **ROITT, I M.** Essential Immunology. Blackwell, 1993
- **TEITZ, N W.** *Clinical Guide to Laboratory Tests. Saunders,* 1996
- **WALMSLEY, R N ; WATKINSON, L R ; KOAG, E S C.** Cases in Chemical Pathology - a Diagnostic Approach. P. G. Publishing, 1989
- **WHITBY, L G ; SMITH, A F ; BECKETT, G J.** Cases in Clinical Biochemistry. Blackwell, 1988
- **BURTIS ; ASHWOOD.** Tietz Textbook of Clinical Chemistry. SAUNDERS, 1999
- **BISHOP.** Clinical Chemistry: Principles, Procedures, Correlations. Lippincott Williams & Wilkins, 2005
- **MCPHERSON ; MOLLER.** *PCR (The Basics),* 2006
- **INNIS et al.** PCR Protocols: A Guide to Methods and Applications. Academic Press, 1989
- **KIRWAN.** *J Rheumatol,* 2001, vol. 28 (4), 881-886
- **SOKKA.** *J Rheumatol,* 2001, vol. 28 (7), 1718-1722
- **CLARKE.** *J Rheumatol,* 2001, vol. 28 (11), 2416-2424
- **AMETT.** *Arthritis Rheum,* 1988, vol. 31, 315-324
- **EXCOFFIER.** *Mol Biol Evol,* 1995, vol. 12 (5), 921-927
- **SKAPENKO.** *J Immunol,* 2001, vol. 166 (7), 4283-4292
- **SCHULZE-KOOPS.** *Eur J Immunol,* 1998, vol. 28, 2517-2529
- **HACKSTEIN.** *Hum Immunol,* 1999, vol. 60 (11), 1119-1127
- **HOWARD.** *Am J Hum Genet,* 2002, vol. 70 (1), 230-6
- **SZABO.** *J Exp Med,* 1997, vol. 185, 817-824
- **MEYER.** *J Rheumatol,* 1999, vol. 26 (5), 1024-1034
- **COMBE.** *Br J Rheumatol,* 1995, vol. 34 (6), 529-534
- **BATHON.** *N Engl J Med,* 2000, vol. 343 (22), 1586-1593
- **LIPSKY.** *N Engl J Med,* 2000, vol. 343 (22), 1594-1602
- **MEYER.** *Ann Rheum Dis,* 2003, vol. 62 (2), 120-126
- **OBER.** *Am J Hum Genet,* 2000, vol. 66 (2), 517-526
- **HOWARD.** *Am J Hum Genet,* 2002, vol. 70 (1), 230-236
- **NELMS.** *Annu Rev Immunol,* 1999, vol. 17, 701-738
- **ROMAGNANI.** *Immunol Today,* 1997, vol. 18, 263-6
- **SCHLAAK.** *J Invest Dermatol.,* 1994, vol. 102, 145-9
- **RITCHLIN.** *J Rheumatol.,* 1998, vol. 25, 1544-52
- **PARTSCH.** *Ann Rheum Dis.,* 1998, vol. 57, 691-3
- **HUFFMEIER.** *J Invest Dermatol.,* 2005, vol. 125, 906-12
- **LASCORZ.** *Ann Rheum Dis.,* 2005, vol. 64, 951-4
- **MOLL ; WRIGHT.** *Semin Arthritis Rheum,* 1973, vol. 3, 55-78

SEQUENCE LISTING

<110> Friedrich-Alexander-Universität Erlangen-Nürnberg

<120> Means and methods for the prediction of joint destruction

<130> L2585 PCT S3

<160> 15

<170> PatentIn version 3.3

<210> 1
<211> 3678
<212> DNA
<213> Homo sapiens

<400> 1

```
cccccgcgcgg cgcgggccag ggaagggcca cccaggggtc ccccacttcc cgcttgggcg     60
cccggacggc gaatggagca ggggcgcgca gataattaaa gatttacaca cagctggaag    120
aaatcataga gaagccgggc gtggtggctc atgcctataa tcccagcact tttggaggct    180
gaggcgggca gatcacttga gatcaggagt tcgagaccag cctggtgcct tggcatctcc    240
caatggggtg gctttgctct gggctcctgt tccctgtgag ctgcctggtc ctgctgcagg    300
tggcaagctc tgggaacatg aaggtcttgc aggagcccac ctgcgtctcc gactacatga    360

gcatctctac ttgcgagtgg aagatgaatg gtcccaccaa ttgcagcacc gagctccgcc    420
tgttgtacca gctggttttt ctgctctccg aagcccacac gtgtatccct gagaacaacg    480
gaggcgcggg gtgcgtgtgc cacctgctca tggatgacgt ggtcagtgcg gataactata    540
cactggacct gtgggctggg cagcagctgc tgtggaaggg ctccttcaag cccagcgagc    600
atgtgaaacc cagggcccca ggaaacctga cagttcacac caatgtctcc gacactctgc    660
tgctgacctg gagcaacccg tatccccctg acaattacct gtataatcat ctcacctatg    720
cagtcaacat ttggagtgaa aacgacccgg cagatttcag aatctataac gtgacctacc    780
tagaaccctc cctccgcatc gcagccagca ccctgaagtc tgggatttcc tacagggcac    840
gggtgagggc ctgggctcag tgctataaca ccacctggag tgagtggagc cccagcacca    900
agtggcacaa ctcctacagg gagcccttcg agcagcacct cctgctgggc gtcagcgttt    960
cctgcattgt catcctggcc gtctgcctgt tgtgctatgt cagcatcacc aagattaaga   1020
aagaatggtg ggatcagatt cccaacccag cccgcagccg cctcgtggct ataataatcc   1080
aggatgctca ggggtcacag tgggagaagc ggtcccgagg ccaggaacca gccaagtgcc   1140
cacactggaa gaattgtctt accaagctct tgccctgttt tctggagcac aacatgaaaa   1200
```

gggatgaaga tcctcacaag gctgccaaag agatgccttt ccagggctct ggaaaatcag 1260

catggtgccc agtggagatc agcaagacag tcctctggcc agagagcatc agcgtggtgc 1320

gatgtgtgga gttgtttgag gccccggtgg agtgtgagga ggaggaggag gtagaggaag 1380

aaaaagggag cttctgtgca tcgcctgaga gcagcaggga tgacttccag gagggaaggg 1440

agggcattgt ggcccggcta acagagagcc tgttcctgga cctgctcgga gaggagaatg 1500

ggggcttttg ccagcaggac atgggggagt catgccttct tccaccttcg ggaagtacga 1560

gtgctcacat gccctgggat gagttcccaa gtgcagggcc caaggaggca cctccctggg 1620

gcaaggagca gcctctccac ctggagccaa gtcctcctgc cagcccgacc cagagtccag 1680

acaacctgac ttgcacagag acgcccctcg tcatcgcagg caaccctgct taccgcagct 1740

tcagcaactc cctgagccag tcaccgtgtc ccagagagct gggtccagac ccactgctgg 1800

ccagacacct ggaggaagta gaacccgaga tgccctgtgt cccccagctc tctgagccaa 1860

ccactgtgcc ccaacctgag ccagaaacct gggagcagat cctccgccga aatgtcctcc 1920

agcatggggc agctgcagcc cccgtctcgg cccccaccag tggctatcag gagtttgtac 1980

atgcggtgga gcagggtggc acccaggcca gtgcggtggt gggcttgggt cccccaggag 2040

aggctggtta caaggccttc tcaagcctgc ttgccagcag tgctgtgtcc ccagagaaat 2100

gtgggtttgg ggctagcagt ggggaagagg ggtataagcc tttccaagac ctcattcctg 2160

gctgccctgg ggaccctgcc ccagtccctg tccccttgtt cacctttgga ctggacaggg 2220

agccacctcg cagtccgcag agctcacatc tcccaagcag ctccccagag cacctgggtc 2280

tggagccggg ggaaaaggta gaggacatgc caaagccccc acttccccag gagcaggcca 2340

cagacccccct tgtggacagc ctgggcagtg gcattgtcta ctcagccctt acctgccacc 2400

tgtgcggcca cctgaaacag tgtcatggcc aggaggatgg tggccagacc cctgtcatgg 2460

ccagtccttg ctgtggctgc tgctgtggag acaggtcctc gccccctaca accccccctga 2520

gggccccaga cccctctcca ggtggggttc cactggaggc cagtctgtgt ccggcctccc 2580

tggcaccctc gggcatctca gagaagagta aatcctcatc atccttccat cctgcccctg 2640

gcaatgctca gagctcaagc cagaccccca aaatcgtgaa ctttgtctcc gtgggaccca 2700

catacatgag ggtctcttag gtgcatgtcc tcttgttgct gagtctgcag atgaggacta 2760

gggcttatcc atgcctggga aatgccacct cctggaaggc agccaggctg gcagatttcc 2820

aaaagacttg aagaaccatg gtatgaaggt gattggcccc actgacgttg gcctaacact 2880

gggctgcaga gactggaccc cgcccagcat tgggctgggc tcgccacatc ccatgagagt 2940

agagggcact gggtcgccgt gccccacggc aggcccctgc aggaaaactg aggcccttgg 3000

```
gcacctcgac ttgtgaacga gttgttggct gctccctcca cagcttctgc agcagactgt   3060
ccctgttgta actgcccaag gcatgttttg cccaccagat catggcccac gtggaggccc   3120
acctgcctct gtctcactga actagaagcc gagcctagaa actaacacag ccatcaaggg   3180
aatgacttgg gcggccttgg gaaatcgatg agaaattgaa cttcagggag ggtggtcatt   3240
gcctagaggt gctcattcat ttaacagagc ttccttaggt tgatgctgga ggcagaatcc   3300
cggctgtcaa ggggtgttca gttaagggga gcaacagagg acatgaaaaa ttgctatgac   3360
taaagcaggg acaatttgct gccaaacacc catgcccagc tgtatggctg gggctcctc    3420
gtatgcatgg aacccccaga ataaatatgc tcagccaccc tgtgggccgg gcaatccaga   3480
cagcaggcat aaggcaccag ttaccctgca tgttggccca gacctcaggt gctagggaag   3540
gcgggaacct tgggttgagt aatgctcgtc tgtgtgtttt agtttcatca cctgttatct   3600
gtgtttgctg aggagagtgg aacagaaggg gtggagtttt gtataaataa agtttctttg   3660
tctctttaaa aaaaaaaa                                                 3678
```

<210> 2
<211> 825
<212> PRT
<213> Homo sapiens

<400> 2

```
Met Gly Trp Leu Cys Ser Gly Leu Leu Phe Pro Val Ser Cys Leu Val
1               5                   10                  15

Leu Leu Gln Val Ala Ser Ser Gly Asn Met Lys Val Leu Gln Glu Pro
            20                  25                  30

Thr Cys Val Ser Asp Tyr Met Ser Ile Ser Thr Cys Glu Trp Lys Met
        35                  40                  45

Asn Gly Pro Thr Asn Cys Ser Thr Glu Leu Arg Leu Leu Tyr Gln Leu
    50                  55                  60

Val Phe Leu Leu Ser Glu Ala His Thr Cys Ile Pro Glu Asn Asn Gly
65                  70                  75                  80

Gly Ala Gly Cys Val Cys His Leu Leu Met Asp Asp Val Val Ser Ala
                85                  90                  95

Asp Asn Tyr Thr Leu Asp Leu Trp Ala Gly Gln Gln Leu Leu Trp Lys
            100                 105                 110
```

```
Gly Ser Phe Lys Pro Ser Glu His Val Lys Pro Arg Ala Pro Gly Asn
        115                 120                 125

Leu Thr Val His Thr Asn Val Ser Asp Thr Leu Leu Leu Thr Trp Ser
    130                 135                 140

Asn Pro Tyr Pro Pro Asp Asn Tyr Leu Tyr Asn His Leu Thr Tyr Ala
145                 150                 155                 160

Val Asn Ile Trp Ser Glu Asn Asp Pro Ala Asp Phe Arg Ile Tyr Asn
                165                 170                 175

Val Thr Tyr Leu Glu Pro Ser Leu Arg Ile Ala Ala Ser Thr Leu Lys
            180                 185                 190

Ser Gly Ile Ser Tyr Arg Ala Arg Val Arg Ala Trp Ala Gln Cys Tyr
        195                 200                 205

Asn Thr Thr Trp Ser Glu Trp Ser Pro Ser Thr Lys Trp His Asn Ser
    210                 215                 220

Tyr Arg Glu Pro Phe Glu Gln His Leu Leu Leu Gly Val Ser Val Ser
225                 230                 235                 240

Cys Ile Val Ile Leu Ala Val Cys Leu Leu Cys Tyr Val Ser Ile Thr
                245                 250                 255

Lys Ile Lys Lys Glu Trp Trp Asp Gln Ile Pro Asn Pro Ala Arg Ser
            260                 265                 270

Arg Leu Val Ala Ile Ile Ile Gln Asp Ala Gln Gly Ser Gln Trp Glu
        275                 280                 285

Lys Arg Ser Arg Gly Gln Glu Pro Ala Lys Cys Pro His Trp Lys Asn
    290                 295                 300

Cys Leu Thr Lys Leu Leu Pro Cys Phe Leu Glu His Asn Met Lys Arg
305                 310                 315                 320

Asp Glu Asp Pro His Lys Ala Ala Lys Glu Met Pro Phe Gln Gly Ser
                325                 330                 335

Gly Lys Ser Ala Trp Cys Pro Val Glu Ile Ser Lys Thr Val Leu Trp
```

340 345 350

Pro Glu Ser Ile Ser Val Val Arg Cys Val Glu Leu Phe Glu Ala Pro
355 360 365

Val Glu Cys Glu Glu Glu Glu Glu Val Glu Glu Glu Lys Gly Ser Phe
370 375 380

Cys Ala Ser Pro Glu Ser Ser Arg Asp Asp Phe Gln Glu Gly Arg Glu
385 390 395 400

Gly Ile Val Ala Arg Leu Thr Glu Ser Leu Phe Leu Asp Leu Leu Gly
405 410 415

Glu Glu Asn Gly Gly Phe Cys Gln Gln Asp Met Gly Glu Ser Cys Leu
420 425 430

Leu Pro Pro Ser Gly Ser Thr Ser Ala His Met Pro Trp Asp Glu Phe
435 440 445

Pro Ser Ala Gly Pro Lys Glu Ala Pro Pro Trp Gly Lys Glu Gln Pro
450 455 460

Leu His Leu Glu Pro Ser Pro Pro Ala Ser Pro Thr Gln Ser Pro Asp
465 470 475 480

Asn Leu Thr Cys Thr Glu Thr Pro Leu Val Ile Ala Gly Asn Pro Ala
485 490 495

Tyr Arg Ser Phe Ser Asn Ser Leu Ser Gln Ser Pro Cys Pro Arg Glu
500 505 510

Leu Gly Pro Asp Pro Leu Leu Ala Arg His Leu Glu Glu Val Glu Pro
515 520 525

Glu Met Pro Cys Val Pro Gln Leu Ser Glu Pro Thr Thr Val Pro Gln
530 535 540

Pro Glu Pro Glu Thr Trp Glu Gln Ile Leu Arg Arg Asn Val Leu Gln
545 550 555 560

His Gly Ala Ala Ala Ala Pro Val Ser Ala Pro Thr Ser Gly Tyr Gln
565 570 575

```
Glu Phe Val His Ala Val Glu Gln Gly Gly Thr Gln Ala Ser Ala Val
        580                 585                 590

Val Gly Leu Gly Pro Pro Gly Glu Ala Gly Tyr Lys Ala Phe Ser Ser
    595                 600                 605

Leu Leu Ala Ser Ser Ala Val Ser Pro Glu Lys Cys Gly Phe Gly Ala
    610                 615                 620

Ser Ser Gly Glu Glu Gly Tyr Lys Pro Phe Gln Asp Leu Ile Pro Gly
625                 630                 635                 640

Cys Pro Gly Asp Pro Ala Pro Val Pro Val Pro Leu Phe Thr Phe Gly
                645                 650                 655

Leu Asp Arg Glu Pro Pro Arg Ser Pro Gln Ser Ser His Leu Pro Ser
            660                 665                 670

Ser Ser Pro Glu His Leu Gly Leu Glu Pro Gly Glu Lys Val Glu Asp
        675                 680                 685

Met Pro Lys Pro Pro Leu Pro Gln Glu Gln Ala Thr Asp Pro Leu Val
    690                 695                 700

Asp Ser Leu Gly Ser Gly Ile Val Tyr Ser Ala Leu Thr Cys His Leu
705                 710                 715                 720

Cys Gly His Leu Lys Gln Cys His Gly Gln Glu Asp Gly Gly Gln Thr
                725                 730                 735

Pro Val Met Ala Ser Pro Cys Cys Gly Cys Cys Cys Gly Asp Arg Ser
            740                 745                 750

Ser Pro Pro Thr Thr Pro Leu Arg Ala Pro Asp Pro Ser Pro Gly Gly
        755                 760                 765

Val Pro Leu Glu Ala Ser Leu Cys Pro Ala Ser Leu Ala Pro Ser Gly
    770                 775                 780

Ile Ser Glu Lys Ser Lys Ser Ser Ser Ser Phe His Pro Ala Pro Gly
785                 790                 795                 800

Asn Ala Gln Ser Ser Ser Gln Thr Pro Lys Ile Val Asn Phe Val Ser
                805                 810                 815

Val Gly Pro Thr Tyr Met Arg Val Ser
            820                 825
```

<210> 3
<211> 26550
<212> DNA
<213> Homo sapiens

<400> 3

```
tacaggtgtg agctactgtg tctggcctga ataataaaat ttaaaacaat ttttcaaaaa      60
ttcaccatga ggtctcacta tattccctag gctggtctca aacccctgga ctccaagtga     120
tccaccccac cttcccgagt agctgggact agagatgcac accattgcac ccaatagagc     180
aatacgtttc tgttctttgt aaattacctg ctctaaggta tttttgttat agcagcctgt     240
atggactaag ctgacttgta acgttacttg agactttaaa gtgttccggt cactgttgga     300
gggctctgtc tgtgttagct catttaatcc ccacaacacc tcaatcagat ggggctattc     360
ttagtcccac tttatagata aggaaactga ggcatggaag cacagcttgc tcaaggttca     420
catctagtca gtgacagagc aggtatttaa acctcaggaa ataatcagag aaacatgtgt     480
agagggttgt ccaaggaagg ccacatccag aagcatctcc caggacagtt gttgtgtagc     540
tcaccctctg gactttgtgg gtctgggtgt tgtttcatga ttatagagag agctctgtga     600
acgtggagga cctgttgtcg gcagagacac aaatggccag ggcatggctg ggcagccgca     660
gtggctcagg cctgtaatcc cagcacttcg agaagaccag aggggcagat catgaggtca     720
gaagttcaag accagcctgg ccaacatggt gaaaccccgt ctctactaaa aatacaaaaa     780
ttagccaggt gtggtggtgg gcacctgtaa tcccagctac tcgggaggct gaggcagaag     840
aatcgcttga acccgggagg tggaggttgc agtgagctga gattgcaccg ctgcactcca     900
gccttggaga cagagcgaga ctctgtctcg gaaaaacaaa caaacaagca aacaaacaaa     960
caaataaatg gccagggcag gggagggttg catattgaat aagatgagct ctgctggaag    1020
cacaggtcag cactaacctg cttcctctct ctctgcaggt gccttggcat ctcccaatgg    1080
ggtggctttg ctctgggctc ctgttccctg tgagctgcct ggtcctgctg caggtggcaa    1140
gctctggtaa gtcaccactt ctcaatcatt catttgttgg ctattaatgg cgtgccaggg    1200
tcctgcagta tgtcacctgg ccttatggag attacactgc agtgggaggg gacagccaat    1260
gacaagtggc cctgattatc agtaaattct aaagattgtt agaaagtgat gggagccggg    1320
tgcagtggct cacacctgta atcccagcac ttcaggaggc cgaggcagga ggatcgcttg    1380
agcccaggag ttcgaggtca gcttgggcaa catagggaga ccttgtctct acaaataata    1440
```

aaatattagc caggtgtggc agtgcacgcc tgtagcccca gctactcagg aggccgaggt 1500

gggaggatcc cttgaactca ggaggtcaag gctgcagtga actgtgatcg cgccactcca 1560
ctccagcctg cgtgagaaag tgagaccctg tcaaaaaaaa agagaaggtg atggggaaag 1620
aacacagaac agcataagag ggggttgggg aagctgggtg gagtgggggg gattgcagtt 1680
gaaagtaggg aagtcaggga aggcctcatt gagctgactt ggaggaagcg ggaaccgtgc 1740
agatgtctgg ggaaggctca ttcttggcag agaggccctg cactgagcct ggcgggaggg 1800
ttgagcacag gagggaatgt ggtggaggag agtgagcagc aggagggagc agtgaaggtc 1860
agcaaggtga cagagtggct gaatcaaaaa agaccttgca gtgtttgagc agaggatcca 1920
tatcatccat tatgttccaa aggactcttc aggatgccgt gtggagaaag gaagagggtg 1980
gaagccagga ggtctggagg gaggtctgga gtggaggaga tgagaggctc cggatccctc 2040
tgggaggtag atttgaggac agattggaat tgaggtgaaa gacagagaaa gagaagtggc 2100
caggatgact ccaagatttc tgacctaaac tactgggaag gacgcggttg tcatttctga 2160
aatgcagaag gatgccagaa gagaaggtac tttggggagg ggcgggaatc aggagttagt 2220
tttggacatg agataagctt ggaatattta tttgctatct aagacagctc cttaacatgg 2280
taagccctta tgcaagttgt tgtcagctga gatgggcgtg gcactgagca tgggagcatg 2340
gaggcgcctg agtggtctca tgctcaggtg gtttagcaaa ctcagtgtac atcctgccaa 2400
ttccagtcct gccatggcca ctgacaagct aggagggcgc tgaaaggaga aggaccccga 2460
tgtctcctcc agcccatcca tctcctctct cccattggcc aaacccaacc ggaaactaaa 2520
ggccaagggt acccggtgat gaagactgtg gtatcagcct cctgagcaca gagagggcag 2580
aaaggggtgg agacaaagag gggcgcagat agtgggcaaa tggggaagtg gcacttcccc 2640
tagctcgagg gcagaggctt ggtgtgatgg aatggcactc cttaaactgc tacatatttt 2700
ccctttaatt tggccaagaa caagttgtca agtttgtgtg agataaaggt gcacttggtt 2760
cgttcttgtc taatggcccc cgcacccatg ggtatttctt cagcttccac agtcatcccg 2820
acactagctg ggaagctcca gcagccctgg tcctggcccc agctctgtgg gcgctggccc 2880
tcaactttgc ctgcactgtg cttttgtgct attccccttg gtcctgtttg ggtgcaagtc 2940
cccctcacgc attgagttcc tgggccgctc aggctgctcc tgtgtctccc cagggaacat 3000
gaaggtcttg caggagccca cctgcgtctc cgactacatg agcatctcta cttgcgagtg 3060
gaagatgaat ggtcccacca attgcagcac cgagctccgc ctgttgtacc agctggtttt 3120
tctgctctcc gagtaagcct gcgctggagc tggaggtttg gggaggttgt gcccaaaggg 3180

tttgccccaa gagtgagctg ggtccaggtg gtgcgctgga gtgcaggatg ctgagtatgg 3240

tttgctgctg tttatatggt gttagagggg aggtcccatc tccagggaca tgttatgtaa 3300

gatacagtgg agcgcatggt gggagtgttg gtccacgtgg cacatggata cggctggaat 3360

actggactag accagcagtt ctcacacttt ttggtctcag gaccctttt cacacttaaa 3420

aatgagtgag gacccaaagg gctttggtgt aggtaacaca tcattctatg tttacctaat 3480

tagaacttgc aatgaagaaa tggtgtaatt tttaaaaaat taaaacaatt aaaaattttt 3540

tttcttactg aaatggaggt ctcactgtgt tgcccaggct gctctcaaac tcctgggctc 3600

cagtgatcct cctgcctccg cctcccaaag tgctgggatt acaagcgtga gccgctgtat 3660

ccggcccaaa atggagaaat tttaagtccc aacaacatgc aagcccgcat tcaacaaatc 3720

ttcagatcaa ttacatgatc acaggtcatg tagcctctag aaaattccac tgtacgccag 3780

tgagagagag tgaaaaggca aataacgtcc ctgtattatg atgaaaagag ttttacctgg 3840

tgggcccaga ccacactttg agaaccactg gactagaccc ttgattgagg agtacggtgt 3900

tgagagtgga gtcctctgtg atggtggatg gaccaggaca catggcatag gagtcaggtg 3960

gttccctggg ctactccatg gtgcacagga tgcttcgtta cactggtgcc caggacataa 4020

tcacgtacac aagacacaca gttacggggc agactgggga tatacggcac accagcatgc 4080

agggttcacc agtaaaggtg gtattccatg attattctaa ggtagatggg ctgtgctttg 4140

tttccattgg cttagtccag ggattggcaa actatggccc gtgagccaaa tccggcccac 4200

tgcttgtttt tgtaaataaa gttttattgg aacacactgg ctgctgtagt tgtaacagaa 4260

actgcatggc cctcctttat gtttttgtt tgtttgtttg tttgtttgtt tgttttcttt 4320

gagacagagt ttcgctcttg ttgcccaggc tggagtgcag tggcacaatc tcggctcact 4380

gcaacctctg cctcccgggt tcaagcgatt ctcctgtctc agcctcccga gtagttggga 4440

ttaatggtgc ctgccaccac acccggctaa tttttcgtat ttttagtaga gaccggtttt 4500

catcatgttg gccaagctgg tctcgaactc ctgaactcag gtgatccacc cgcctcagcc 4560

tcccaaagtg ctgggattac aggcatgagc cactgagccc ggcctcctcc tttatcttaa 4620

ttgaaataat tcagaaatgg aaagtcaaat actgcatgtt ctcacttata agtaagagtt 4680

aaataatgtg tacacatggg cattattcca tgtaccatgg aataacagac attgaagact 4740

tgggagggtg ggagaggggt gaaggaagag aagttactta atgggcatag tgtacaccat 4800

ttgggtgacg gacccaccag aaccccagac ttcaccacta ggcagcatat ccagtgagaa 4860

cagatctgag gcttgccatc aaaattgcac ttgtaaggcc gggcactgtg gtggctcgcg 4920

gctgtaatcc cagccctttg ggaggccgag gtgggcagat cacttgaggt caggagttcg 4980

```
agaccggcct ggccaacatg gtgaagctcc atctctacta aaaatacaac aattaactgg    5040
gtgtagtggc gcacacctgt aatcccagct actagggagg ctgaggcggg agaattgctt    5100
gagcccagga ggtggaggtt gcagtgagcc gagatcacat cactgtactc tagcctgggt    5160
gacagtgaga ctttgtctca ggaaaaaaaa acaaaaacaa aaaacaaaaa actcgtaccc    5220
cctaaattta tacaaataac caaaaaaaaa aaaaaaaaag gaaattgtgt ggctttgaag    5280
tccaaaatat taactatctg gcctgttaca gaaaaagttt gcagacccct ggcctagccc    5340

gtgagatgtg ggttggctgt taaggtggaa cattggaatt atcttacgat ggccaaactg    5400
tgcgatgcag agcttatgtt gttctaaatt aattagtgcc accggttctt ccctttcatg    5460
ggctttcagg aacaagctaa gtcccaggac cagggccggc agctaggcag gtgtgaggag    5520
catccttggt gcatgtggta agaggctgtg gccagcaaga gaggcaaccc tagtcggctg    5580
ccccagcaca ccctggccgc tcccaagccc ccagatctgt cctcacatcc gtgatcggga    5640
agctggaaga gtctgatgcg gttcctggag gcatgtcccg gacacagctg tggggcccag    5700
ccagcctaca ggtgaccagc ctaacccagc ccctgtgtct gcagagccca cacgtgtgtc    5760
cctgagaaca acggaggcgc ggggtgcgtg tgccacctgc tcatggatga cgtggtcagt    5820
gcggataact atacactgga cctgtgggct gggcagcagc tgctgtggaa gggctccttc    5880
aagcccagcg agcatggtga gcagggcgga gtgcggcagg ggtggctggg tgtgttccca    5940
cagctgcctg ggctgagggt ggggtgggca ggggaggagg tggggtcata gcaacagcag    6000
gaggaagccg cctgtatttt cccaaatctg atgggattcc tgcccctgcc tgggcctcag    6060
tcctcccacc tttgaaacgg agctggtcgc agtagaccac caagccccct tcagcccagc    6120
tgtttccacc cctgaactta agtgcccagg aaggcgtatt gagatgaggt gtgcttgctg    6180
gaaggcatgc ctgctgctga ttgaaaaccg aactgggaac attccttcca ttctgtgtcc    6240
actggtcagc tgctgcggct ttggatggtc ttgaccgtgg aaggctgacc ttcttctggt    6300
acccggagtc cctgcaggaa tccccccttga gcttgctggg ctgtggtgac aggagtttaa    6360
aacatgcgtt gtattccagt gatgcatgat atgacatgca tcacaggaat aaaaacctga    6420
ggtctcatgg atatgattgc ttcaaaggag accaagtttt aaaacagatg aatcaaaata    6480
aagaaaaata ctcagtaaat catcataaag tacagagatg tggccaaagg tgtgaaggat    6540
gcagctgtaa aagctgaagt ttgaggccgg gtgtggtggt tcatgcctat aatcccagca    6600
ctttgggagg ccgagcccag cggatcaccg gaggtcagga gttcgagacc agcctggaca    6660
acatggtaaa accccgtctc tactaaaaat acaaaaaatt agtctggcat ggtggcaggc    6720
```

gcctgtaatc ccagctactt gggaggctga ggtaggagaa tggcttgaac ccaggagaag 6780
gaggttgcag tgagcttaga tcatgctact gccctccagc ctgggcgaca gagtgagatt 6840
acgtctcaaa aaaataaaaa taaataaaaa taaaaagatt ttttaaaagg ctgaagtttg 6900
ggttactttg gctcatacac tttgccttca ctgtagaaag gtggttagta aagaccaggc 6960
gcggtggctc atgcctggaa tcccagcact ttgggagccc agcgcaggca gatcacttga 7020
gccctgggct attgaggctg cagtgagctg ggattgtgcc actgcactcc agcctgggca 7080
acagagtggg accctgtctc aaaaaagaag aaaaaaaggg taattaataa acactaaagt 7140
tctatgtaga attttagcaa cattattgtt attataatct tctttgctat ggctctgaat 7200
ctgtgtggtg ctccagaagt atgctatgga ggttttgtcg accaaaaatc tgggtggtgg 7260
ctgtggtttg taggccgggg ctgggctggg tgatgggga gtcactgcat agatcctcac 7320
atagaggccg cttctcccgc agtgaaaccc agggccccag gaaacctgac agttcacacc 7380
aatgtctccg acactctgct gctgacctgg agcaacccgt atccccctga caattacctg 7440
tataatcatc tcacctatgc agtcaacatt tggagtgaaa acgacccggc agatgtgagt 7500
gggcatgctt tgacgttttt ctgtgacctc tggggaacag ggtgggtgac cagcagaggc 7560
ccagtccctg gagccaggag cctgggaggc aagccctggg gctggatagc aaatcccagg 7620
agctagagac ctggcttctc acctggctct gccctaggca agtccctttg cttcctggcc 7680
ccccacccct cacatcagag aaggggagtt atctctgcat gccgctcctc ctctgtaaag 7740
gtagggctgt gggccacatc tgtgtttccc agtttggggg acacaagtga tcgtaggtgg 7800
cacattgaca gctcacttga ataaccctat tattgaagag aataatactg actcaagaga 7860
cagtgacccg tgtcagttcc cttttgaggc caacgggtta aggaggaagt ccccatacag 7920
ctgactcgtt tactaattcc tcttaatgaa gagagcagag gccacacccc aggcttagac 7980
tttcccaaga aaacaagatc agtttgttgg ttgttcccca tggaagctgg tcctgacatt 8040
cccttcacag tagtgttggt ggagtttttg ttgttgtttg ttttgagaca gagtctcact 8100
ctgtcaccca gggtggaaca cagtggcgtg atcttggctc actgcaacct ccgcctcctg 8160
ggttctagcg attctcctgc ctcagcctcc tgagcagccg ggactacagg cacctgccac 8220
cgtgcccagc taatttttgt atatttagta gagatggggt ttcactgcgt tggccaggct 8280
ggtctcaaac tcctgacctc agatgatcca ctcgcctcgg cctcccaaag tgctgggatt 8340
acaggtgtga gccaccgcac ctggccagtg gagttccttc ttaagtacat gtattgacat 8400
ctttaaaaag ggcgagagga tttacaggaa actatcaggt cagtaatggc aggggccgtc 8460

cacagtgggt ggctgagtcc ccctattttt ctgctggtgt gcggggaggt catttcctgc 8520

cacccatgtt tccccaccct gaatccacct tcctcacatt cccattggag ggacactctc 8580

tggacatatg ggacctgggg tcccacaggg ctgcattcca atgcctgctg tgccactcgc 8640

cagctgtgtg atgttgggca tatcccataa cctctttgtg cctcagtttc ctcatctgta 8700

acacaggagt gacaagagca cccgcccaca gggctatgac agtacaaggt gtgtgataca 8760

gatgagctcc cctgtttggc ccacatgtgt cctaaaagcc atgtgccctt tctcttgagt 8820

gccccaggcc acagagatcc ccatctgccc gctgtcccac acactggtct gtcatttgtt 8880

ccttgaggtt tgtgagggcc ggctctgtgc atcccagggg cccaggctgg gcctggttgg 8940

ctctcaggga gcaggcaccc gccaccttaa gctcccatgc tggtgtctgt cactgcttcc 9000

tctcaatctg gccaagccag ggtgtcgat ttatatctct caggtctggt ttcccctttg 9060

gcactgggcc aggtatgggg aaagagcagg aatggggcag ttggctcaca cagcagaggc 9120

tcagaaagcg gggggcatgg ggggaaggag tgcacagatg ctagagagtg ggcaagttt 9180

tgtttggtca ataaatctcc ttctcatgcc ccaggcctgt gcaagaccta cagagagtcc 9240

caaggatggg ctgggggaa gagaaaggta ccaccttcag agtccaaaga tatgttattt 9300

aatattttca tatttctaga tctgccttca ggcatggctg gatccagctt ctaggaacct 9360

gtccagctct gcgccctgct ttattctgta ctggcttcgt ttttaggcag gctcttccct 9420

catgtagtgg cagatatgcc tactagttgc tccaggccta catcccaaag ccacagtggg 9480

aaaagggttt tttttcttga cggttctaat aagagtccta aggctgctgc tcagtggcct 9540

ggcttcgatg ctgtgccagc ctctgaacca atcactggct gtgggtggag agagggtgct 9600

ggtggagggc cctgcttgtc cagggaggag tcacatacct gcctctaggg ctgcaggtgg 9660

gctcagctcc atccaaacca gatgaactga aaataaggca ggagtggctt ccccagggga 9720

aactggggaa gaggaagcag gactgtgctg gctaaaatgc cagccaggtt taagacgtgg 9780

caccagatgc cagtcatggg attggattgg tcagcatgcc tgggctatgg cttaggggta 9840

tgttggtgct cagggatgcc acaggcctcc agataccagg tctgaggcag aagaatgaag 9900

tccagcttct cttgtgggtg gaacagtggc aactgagata ccccatctct cccttcccaa 9960

gaacagagct gaacataaag aatttagtga ttggccagag cttggccaca tgctcccctc 10020

tgatgaatga taggccaggt gatgggattg gcacaattgg cttagactaa tgagggttgg 10080

ccctggagtt gcaggcagtg gagttctgtc ctaagcagtg ggcacctaaa cccgatggca 10140

taaaagctgg gcgggtgtcc acctgcatct gccacagcac tgcaggcacc aactgtggct 10200

catactgagt gggataaatt ccagaaagaa acattaggaa cttactatag aattttgggg 10260
ctagagctac tcattcattc ccctagataa tttctaggca aggttccata gtggaggggg 10320
agttttggct tgggcattga aggatgcata ggagttttct agatggggaa agaagggaac 10380
ggtagaccag gcagagggaa ctgcatgata aaaggtttat gggtgtgaaa attcatggaa 10440
tgtttgagga ttatggggtt gggggatgtg ggaatatgtg tagcgataaa gcaccaaaca 10500
aagccaaaag tttagttaga gccctgaatg cctgcctcat aatggtttcc atattttata 10560
tgcctactat gtgccaggca cattgctcag ggtcacacag ctggaaatgg cagggctgag 10620
tttttgttgt tgttgttgtt gttgttgaga cagagtctca ctctatcacc caggctggaa 10680
tgcaggggcg tgatcatggc tcactgcatc cttgacttcc tgggatcagg tgattctccc 10740
acctctgcct cccaggtagc tgggactaca ggcacaggcc accacgccag gctaattttt 10800
tgtattttta gtagcgacag ggtctcgcca tgttgtccgg gctggtctgg atctcctggc 10860
ttcaagtgat ccccctggct cagcctccca aggtgctggg attacaggct tgagccaccg 10920
catccagccc agatctgaga tttgcaccca gtatttgaac tcccaagcct gtgctctttt 10980
tcctcccatg gacatttctc tcagagatgg tctcccaaac acctgtcctt cttgttaaaa 11040
aacagacaaa ccgcaagtag ttctttggaa gctcagattt ctcttttgtt tcttagtaaa 11100
acatttccca gttcccagct cccttccagg gtgtaagatt tcttcggtaa cttacatcta 11160
gctgttgctt cttgtttgct catgtttaga aagaaagaca aaagagagtg agaatttttct 11220
ctcccttccc cagtctcccc acaactcaca ccccaccctc agctccctct gtaataggaa 11280
aatctctgaa ctctctgtag ttgctccagc aatcttttgg aactttgctt ctttcttgtg 11340
aaaaaacctc cccttggctc actttgcacc aggtttcccc aaatgtgctt ccaaccacaa 11400
gcagaaatgg agctgccagt aaccaggaag aaactgccgg gggctgagga agaggagagg 11460
gaggtgcata gccctggatc tcgcagggag aggggtgaca ggatgagaac tcaggttgct 11520
cacttgccat cagggtcagt catgaatata gcgttcatgt atcacttttt aaagcttttt 11580
tggagggtaa aagtaatagt tacacaaaat aaaaatacaa atggtacaaa aggacttaga 11640
atggaaacat gtttctctcc cgactccagc ctcctgtttt tcttcccaga gactgaccac 11700
tgctgtctgt ctcttgccag aagggaaagg gaggcaaggt tagggcaggc agagggcatg 11760
tgcatccttt agagagagct tatgtctata caagcaaatg tgtgtgttca gtcatcgctg 11820
tcttagtttt ctattgctgc ataataatgg tactaccagc ttcacagctt taaacaacac 11880
ccatttatta tctcatagtt tctgtggttg ggagtctgga catagcttag ccaggttctc 11940
tgctttagag tctcgtgagg ctataatcaa ggtgtgggat ggggctgcag tttcatctga 12000

ggctcaattg gggaagggtc acttctaagc tcatacaata ttggtgacat tcagtccctg 12060
gcaggctgtt gaactgagag cctcagtttc gtgctggctg ttggttgtag ttaaccctga 12120
attccttccc atgtgccctt tgcaaagcca tcaaggcaga gagacttgcc tagcaagtag 12180
gatattacag tcttctgtaa tataatcaca tccatgaaat cctctatata tcccatcacc 12240
tttaccatat tctgtgggtt agaaacaagt agcaggtcct gcccacactc gagaagacca 12300
gatgacacaa agatgtgatt caaagtgggg atcatcgggg ccatcttagg tttgtctgca 12360
gtgatcactg tgccatctct ctctctctct ctcttttttt tttttttccg agacgaagtc 12420
gtcactctgt cacccaggct ggagtgcagt ggcatgatct cagcttacca caatctctgc 12480
ctcccaggtt caaatgattc ttctgcctca gcctcctgag tagctgggat tacaggtgcc 12540
cgccaccaca cccagctaat ttttgtattt ttagtagaga cagagtttca ccatgttggc 12600
caggctggtc ttgaactcct cacctcaagt gatccaccca cttcggcctc ccaaagtgct 12660
gggattacag gcatgagcca ccatgcccag ccccatctct ctttaaaaaa caaacaaaca 12720
aacaaaaaac ataaaaagaa gcagagaaca catacacatc tgcatcttcc cttgtttact 12780
taacaataga tcttggaagt cacttctcag tagaggctag gttgggcaga gcattggatt 12840
ctaggccagt gagtttggac ttgaccatgg agacactagg aagcccatga aggacagaga 12900
gagatgcctc gaccctgcca gtcctttaga aagatcaccc agtgcttttt gtataccaaa 12960
ccctatttga aatacttacg tatattaacc catttcctta tcaccacaac cctgcgggaa 13020

gggagatagg cacttttatt atcttcattt tgcagatgag gacattgagg tccagagagg 13080
ttatgtcact tacttaaggt cacacagcca ggaagtggta gtagggactc ttacccttgt 13140
tttacagatg agattgaatt atctcacgaa aactcagaaa ggttaaacaa cttgcctaag 13200
taacatacag ctaattagtc gaggagcctg acgcatgttg ctctagcctg gtcacagtta 13260
cagaggtggc aagcaatggc ctgaacagga cgaacaacca aatacccagg ctggtggctc 13320
ttaaacatgg tggggtcagc taacgacagc aaccagggtg ggcactggtg cccctcgccc 13380
ccggctggtg ccctaacatc tccctttttct ctaccagttc agaatctata acgtgaccta 13440
cctagaaccc tccctccgca tcgcagccag caccctgaag tctgggattt cctacagggc 13500
acgggtgagg gcctgggctc agtgctataa caccacctgg agtgagtgga gccccagcac 13560
caagtggcac aactgtgagt atcaagaggc ctaagcaatg gtaatctcca ctctccattc 13620
ttcccctgtg gccagacact tcccctggct gagtctctgg gcttttatat cataggatgc 13680
ctctaatggc aatcctgcca ttagatacac ctgccgtggt gtatctgcca ggtaggcagg 13740

ctaggctgca gtaacacaca agcccacaat ttccatggct taacactata ggaatatatt 13800
tcttgctcac gtaacaagct aacgtgaatg ttgctggttt gtaggtggtt tccctccctg 13860
tagaaatctg gggagtgagg ttctttccat cttgtggtgc catcattctc caggacaaag 13920
attcttacct acttttgtgt cctggtttcc tttggcagcc tggtgaagcc tatggacctc 13980
atttcagaat atttttaaat acataaaatc ccagcctggg caatatagtg aaaccccat 14040
ctgtacaaaa attagccagg catggtggca tgcacctgta gtcccaggta ctgggaaggc 14100
tgaggtggga ggatcacttg agcccaggag tttgaggctg cagtgagccg tgatcgtacc 14160
actttactcc cacctgggtg acagagcaag agcccatctc taaaaataaa taaatacaat 14220
gaaataaaat aaaataaaat aaatagaact acagaggaaa ctaattgtat tgaaatgcag 14280
ttataaaaca tttaaacaca tttttaatct agagatatat gtgcttcttt attaagatct 14340
ataaataata agttctaggg gtagctcgca taaatactgt aatttcaaag tagataagca 14400
taaataatac tttatgatac tgaaattgtg atgtgatatg agaatagctg tgagttttgt 14460
tttgctgggg acaggatcac tgatgctgtc attactgggg tctcttccct ccattctttt 14520
tttaaaattg tattttattt tatttttaaa attttaaaat aaatagagac agggtatcac 14580
tatgttgccc aggctgcttt tgacctcctg ggctccagtg atcttcccat cttggcttcc 14640
caaagtgctg ggattacaag tgggagccag tgttcctggc cccttcctcc attcttaatg 14700
gaaggagatg ctaggtgtga gaggttaggg aaagtaaaga tgtaatttct ttcccatcca 14760
agttctcaga cccctgaatt ctacctgcag ccatgttggt ccatcaaccc caagtgaaga 14820
atccctgctc tagggcccca ccattgtctg tatccagcca gcagaagagg cgtgattatg 14880
gagatcacat ctgcttcttg aaagcagaca gcccggaagt gggccgcatc acttcctctc 14940
aaattctatt ggtgaaaatg gtcacatgac tacacatagc cacaaaggag gctgggaact 15000
ttctcacttg gaacctacat cccagaaaca actcttttca gtgaggtatc ccacaggtct 15060
ttcgcagtag aaatattgat tatctcacat aaaatgaagt cttacaaatg gacctactgg 15120
gttttgtaca gcagccaagt gatatctctt cccttctgct gtcttccctt ctgccatcct 15180
tcacatggtg gcattgtatc cttagacttg ccacccatgc cctcaggttg gccgttgcac 15240
actgtcttac ataaagcagg aaggaaagga aaggctgcta cgagagagtg taccttgtgc 15300
atctcttttt taatcaggaa gcaaacatct ttctagaagc ttccctagca aaattcccct 15360
tacatctcat tggccaagac tgttacatgt tacatggtta ctgttattac ttgctcattg 15420
caaggaagac tgggaactca aatgcctgga aaaaggaaca ggataatcgt gattggctca 15480

agccttaggg tgggcatggc tccctgacaa gggagagagg aaaaagctgt tgagtgaaga 15540
agactgcttc agtttcccca tctgtataat gggaggagta agggctgtcg tgaaaactca 15600
atgaaagaag attcttcaac gtggtaggtg cagtggcagc tggcagtacc ctgaccctgc 15660
caccgcacag ccctctcagc attgctcatc ctgcactgtg gatatcagtt gagccacgtg 15720
tctcctgccc tgggctgtga gctccatagg cagggtctcc atggctgtat ctccagaacc 15780
cagcacagaa ccaggtgctt gggaaagttt tgaattgatt ctcatctgcc attggcatgg 15840
ggaagggaac tagcttgtat gaaacagata acaatgtatg ggaccctcat tcattatttc 15900
agcaaatatt tgctgagttc ctcctacatg gctagccctg tgctagacac tggggaatcg 15960
gcgatgaaca aagcagatag aaatccccac tcttgtggag ctgacattct ggagggagag 16020
acaaaaagca aacatataaa gaaagaaaga aatcacatgg atctggatga cagtgagtgc 16080
tgggaagaaa ataaaagcag aggaagggga tggagcgatg ggcagggggc aacggtaggg 16140
agggtgtcgg ggaaaacttt ttggagaatg tgacgatgaa agtgaacaag gagaagtcaa 16200
ccgtgttgag atgatggcag ctaatgatgt ggacaggcca ctctgttctg agtgcattat 16260
ctattgattc atcatgtcat cctcgcaaca gccctgcacg atcaattctg tcattaaccc 16320
catagtacag atgaggatgc ggaggcacag agaagataag ggacttgtcc tgtgtcacac 16380
agcaaggagc catccggctc ctaagttggt gcatttgact tctgtgcttc cggaaagaaa 16440
gagcagcaag tttaagatct ggaggtggca ctgagctttg gaggagcagg gggcaatgag 16500
gtggccggtg tgacgaggac tcaatgtgca agagggagag tggtggggag atgaggtgga 16560
ggggtggtcg gcggtcagat cgtggagggt ctcggacgag ggtcctgacc ctgggtctcc 16620
agtcctggga agtggagccc aggctgtacc atggctgacc tcagctcatg gcttcccctc 16680
ccacttccag cctacaggga gcccttcgag cagcacctcc tgctgggcgt cagcgtttcc 16740
tgcattgtca tcctggccgt ctgcctgttg tgctatgtca gcatcaccaa gtgagtcctg 16800
ggcccagtgc tgccgagcag tccctctgga gtgcagggtg gcagggactt gcccctctag 16860

tctgcccctt tgcagtcctc tcagtcaata atacgtattt actgagcagc tactacacac 16920
cttgagagta gagctgagaa catatcgaca aggaccccac tttttctttt tttttttttt 16980
tttttttttt tgagacggag tctcactctg tcacccaggc tggagtatag tggcacaatc 17040
ttgcctaaca gtaacctccg cctcccgggt tcaagcaatt cttctgcctc agcctccaga 17100
gtagctggga ttacaggcgc atgccactat gcccggctaa tttttgtat ttttggtaga 17160
gatggggttt caccatgttg gtcaggctgg tctcgaactc ctgacctcat gatctgcctg 17220

cctcagcctc ccaaagtgct gggattacag gtgtgagcca ctgcacccaa ccaggactcc 17280
acatttctaa aaccggcatc ctactgggga gactgaaaat acatatcaat cacaaacagg 17340
tggttttcca tagtgaccca ctctctgaat gcactagacc agggtggagg ccagagatct 17400
tctggggtgc tttttgcaag gggaccagg ataaggctct ccaaggaggg aaaatttgag 17460
gggggccctg actggggaga atgagctggc cagggataag caagatggag tcatcccaca 17520
tccccttaca acactgggtg cctgggcaac tgggggcatt tgggggcatg tggtaggagc 17580
cagaggaatt tgcgacgatt gccctgatgg agtcaggaga cctgggtttg aatcctggcc 17640
ttggagcttg gtagctggcg gccgacaagt tgctgaaacc cctgagcctg gggttcctgc 17700
tttgcagagt gacagtgatg gtgagaacat atttcatcag ccagaagagg ccaaatcaca 17760
gtaaaggctg agggaggaga tgagtggcga gtggctggga ggtggtggaa ggagcctcgt 17820
ttccagagag ctcttgccag cccttggaat catggtgtct cagagcctca gtcctcccat 17880
ctctgaaatg ggactagcaa gctcaacctc actaagtcag gattagaggt ggctaaggat 17940
tattaacatg attgatgaaa gtgcccactc ttggcccagc acacactagg taggcaggga 18000
atgcaaattc ccctccatat cttgtcactg atgcctccga gcaaccttgg actgatcgcc 18060
ttgctctgag cctcagtttc cccatcacct gtacctcttc ccactcccca tcactatatc 18120
ccagcatgcc agcctctttg ctgttctttg tctttggttt cttgttttgt tctgtttttt 18180
agacagggtc tcactctgtt agccaggctg aagtgcagtg gcgcggttac ggctcactgc 18240
agcctccaat tcctgggcta aagagatcct cccatttcaa cttccagagc agctgggaca 18300
acaggcgctt gccaccacac ctggctaatt ttcttatttt aatttaattt tattttattt 18360
tttgggacag agtggagtct caaaaaccaa gctagagtgc agtggtgcga tctcgactca 18420
ctgcaatctc tgcctcccgg gttcaagcga ttctcctgcc ttagcctccc gactagctgg 18480
gattacaggc gtgtgccacg acacccagct aatttttgta tttttagtag agatggggtt 18540
tcaccatgtt ggccaggatg gtcttgaact cctgacctca agtgatccac ccacctcgtt 18600
ctcccaaggt gctgggtaca ggcatgagcc actgtgcctg gccaattttc ttacattttg 18660
tagagactgg ctgtcactta tgtagcccag gctgatcttg aacttctacc cctttatctt 18720
tattcatggc acttattacc atgaatgaat gacctcatat aagcatttct ttcgtttttt 18780
tttttttttc tttgagatgg agtctcatgt tgtcccccag gctggagtgc agtggcgcga 18840
tctcagctca ctgcaacctc cgccttccgg gttcaagcga ttctcctgcc tcagcctcct 18900
gagtagctgg gattgcaggc gcctgccacc atgcctggct aagttttgca tttttagtag 18960
agacggtgtt tcaccatatt ggccaggctg gtctcgaact tctgacctca ggtgatacac 19020

ctgccttggc ctcccaaagt gctgggatta caggcgtgag ccgccatgcc tggcctcata 19080

taagcatttc tgtctccatt tatcatccat ctttccctct tgaaggtcag tttcaccaag 19140

gcaggcatct ttgtctcgtt cactgttgtg gcctcagggc caggcacagt gagtcaaaca 19200

tagaaggtgc tcaataaata tgtgtttatt tattgaaacc atgggcagag gctaattcag 19260

aagcggtctg aggaccttac ctcccagtga tgatgcacca tggccccagg caggccagga 19320

agagagaagg gttgtgtttc tccgtaggtc ccccagcttc ccaggccatc ccaggccatt 19380

ccctggtcat ttgccctcag ctgctctgaa aaagggattg ttgaggggaa cctagaatcc 19440

tctctctgca gtttgagtct ttcctaatcc cctggggtct cattcccact gaggacatag 19500

gtggcctcct caggaactct gtgctgggta acagaatgcg ggagtgtgaa cctggctctg 19560

ccacctacca gctgtcactc cacctccttg ggcctcactc tcctcatctg tagaataggg 19620

ttagcaatag aatccatgtc accaggttag aatgatgagt cagtggtttg acctccagaa 19680

actaatcagc ctgatctctg atgccaaata agtattggtg ataacgacca cttttatggg 19740

aggagcgttc acctgtcaat aattcagaga tcaacacctt ttccttttgt ttttcaggat 19800

taagaaagaa tggtgggatc agattcccaa cccagcccgc agccgcctcg tggctataat 19860

aatccaggat gctcaggtag gagtaggcgt ggatgaggac atgtgggact gtgtacatga 19920

agaagtgtgg ttcagaacac ctgggctgtt aaggaccttc actggcttct ggaatggcaa 19980

atagacagtc aggagggttg caggggagac agaggcagaa gccgaatgag gtcattagca 20040

gaccagaggc tttcccgccc ttccccttgg caatcccagc ctggggtggg cttctctggg 20100

gttggtttcc tgtttttttc cctccccttg ggagaatgac ccttgggtca tcatcactgt 20160

gtcattccct ggggaggtgc cagtaccagg gctagaggcc agaaggagtg gaggaaggag 20220

agggtgacag gctttctgtg tcttcttctt aagcatagga aactgccccc gaagcactag 20280

caaatccctt ccgggttctc attggcctga aatgtatccc accccctaagc caggggtgga 20340

gtcagcttcc ccaaggcgat ggtcctgtgg gtgagtgggt ggggtttgcc tgagcaagat 20400

gagagttctc taggtaggag aaagggggat tataggtcct gtctagaaga gaaggtctga 20460

gggtccttgc ttttccaggg actctggaat ctagtggtgt tggctttgaa tcctgactct 20520

gccactcact ggcagtgtgg acttgagcaa gttgcttaat tctctgagcc tcagtttcct 20580

cttgtgggtt ataacagtgt ttacctggta ggacagatat tggaatttat tgagacaata 20640

catataaagt gcatattcca gcctcttgca aataccaagt gccatttatg tatcagttag 20700

tgtttgctgt gtaacaaatg accccgaaat gtagagggtt acaacaactt tatttagctt 20760

atgcttctgc aggctggcat ttggggctgg gctcagcagt gagggtggcg ggggaggctg 20820

ggctgggctg ggctgggcag atctgaattg agctgacccg tccccgtagc ctccctccgt 20880

gtctgacagt tggctttttt tttttttttc tttttctgag acggagtttt gctcttattg 20940

cccaggagtg caatggcgtg atcttggctc actgcaacct ctgcctcctg ggttcaagca 21000

attttcttgc ctcagcctcc caagtagctg ggattacagg catgtgccac cacgccaggc 21060

taattttgta tttttaatag agatggggtt tcttcatgtt ggtcaggctg gtctggaact 21120

cctaatatca gatgatccac ccacctcagc ctcccaaagt gctgggatta caggcgtgag 21180

ccactgcacc cagcctagtt ggctgacttt tacctgggac agtgcaggtg cctgagccat 21240

gtgcctctca ctctccagca ggccggccca ggcttgttta cagagtggct cagttttcaa 21300

gggtgggaag tcccaaggct tcttgaggcc taggcgcagc actggcatga tatcacttcc 21360

atcacattct atgggcccaa gcaagtccca gggccagtgt agattcaagg gatgggagga 21420

gattcagagc actcctctgt ggccactttt gccatcgacc acagtccctg taaatattag 21480

gacaatgtaa ttaattccca ggaatctgag gctcagaaag cgtaagtgac ctgttggact 21540

tctgatctgt gtgatgtcga ggcttgtacc ccttcctgag cattgccgta ctccaggccg 21600

ggctgcaagg ccactctgct ctttcattgg ctgtctctgt attttagggg tcacagtggg 21660

agaagcggtc ccgaggccag gaaccagcca agtgcccgta tgtatctgaa cttaggtcac 21720

agcctgcatg cattgggaag gtgatagaat tggagaggca agcccctagc tccatgtctg 21780

ccttctcttc cctgcattcg gtaattgccc tgtgacatta gccttcaagg gacggcagga 21840

ggaggggtgt tctggaaacg tggactgctg gccaagcccc ctgagtttca ctggtgtgtc 21900

aggtacatgg tgatacccct tgggagtgct gttatagtta acaaccagag cagccgtgcc 21960

tgttgttaaa atcttgacct aattgtatac ttgtcggcaa atagccacta tcctgaacac 22020

tcccctcctt ttttttaata tacaggatct cactctgtgg cccaggctgg tgtgcagtgg 22080

tgcgatcata gctcactgca ccttcaaact cctgagctca agtgatcctc ccatcttagc 22140

ctcccgagta gctgatacta cagatgtgca ttaccacgcc tggctatttt aaaaggtttt 22200

tgcctgtaat tccagctact caggaggctg aggcatgaga atcacttgaa cccgggaggc 22260

agaggttgca gtgagcgcag attgtgccac tgcactccag cctgggcgac agagtgagac 22320

tcttgtctca aaaaaaataa taccaaaaaa agtttttgta aagacaagct ctcgctgtgt 22380

tgccccgcca ctgtggcctc cttagcttct tccctggggc ctgctggacc tttccatact 22440

ccagaaacta aagggggtcc aggaccctgc ttcaacccta ggatcccgca tctttttttt 22500

tttttttttt ttggacgcag ggtcttgctg tgtccctcag gctggagtgc agtgattcac 22560

tgcagcctca aactcgtggg ctcaagtgat tctctagcct cagccttcta agtagctggg 22620

actacagtca tacaccaaca tgcccagcta attttccttt tttttaattc ttgtagagat 22680

gtttgagacg gcttgggctc tgttgcccag gctgttctca aactcctgag ctcaagcgat 22740

cctccctcct cagcctccta aagtgctggg attacaggcg tgagccaccg cacccggctt 22800

ccatatcctt tctaattggt catggcttgg gataatggtg ttgcttttaa ttatcatcat 22860

ccataaagac tttttcttac tcaacagatc tgagcttgta tttggtgccc aggacatgtg 22920

ctgggttccc gaaatcccaa agacacagac cctaccctca gggatttctc attctagcaa 22980

catagactga tcaattactg attataacgt tagaaggcat gtctgaagta gacagccatc 23040

aggacatggt gatttcaggc tgggctttga agaatgaata ggagtttttc aagtgtcgaa 23100

actgaaccct gaccaacctt tgcttttgca gacactggaa gaattgtctt accaagctct 23160

tgccctgttt tctggagcac aacatgaaaa gggatgaaga tcctcacaag gctgccaaag 23220

agatgccttt ccagggctct ggaaaatcag catggtgccc agtggagatc agcaagacag 23280

tcctctggcc agagagcatc agcgtggtgc gatgtgtgga gttgtttgag gccccggtgg 23340

agtgtgagga ggaggaggag gtagaggaag aaaaagggag cttctgtgca tcgcctgaga 23400

gcagcaggga tgacttccag gagggaaggg agggcattgt ggcccggcta acagagagcc 23460

tgttcctgga cctgctcgga gaggagaatg ggggcttttg ccagcaggac atgggggagt 23520

catgccttct tccaccttcg ggaagtacga gtgctcacat gccctgggat gagttcccaa 23580

gtgcagggcc caaggaggca cctccctggg gcaaggagca gcctctccac ctggagccaa 23640

gtcctcctgc cagcccgacc cagagtccag acaacctgac ttgcacagag acgcccctcg 23700

tcatcgcagg caaccctgct taccgcagct tcagcaactc cctgagccag tcaccgtgtc 23760

ccagagagct gggtccagac ccactgctgg ccagacacct ggaggaagta gaacccgaga 23820

tgccctgtgt cccccagctc tctgagccaa ccactgtgcc ccaacctgag ccagaaacct 23880

gggagcagat cctccgccga aatgtcctcc agcatggggc agctgcagcc cccgtctcgg 23940

cccccaccag tggctatcag gagtttgtac atgcggtgga gcagggtggc acccaggcca 24000

gtgcggtggt gggcttgggt cccccaggag aggctggtta caaggccttc tcaagcctgc 24060

ttgccagcag tgctgtgtcc ccagagaaat gtgggtttgg ggctagcagt ggggaagagg 24120

ggtataagcc tttccaagac ctcattcctg gctgccctgg ggaccctgcc ccagtccctg 24180

tcccccttgtt cacctttgga ctggacaggg agccacctcg cagtccgcag agctcacatc 24240

tcccaagcag ctccccagag cacctgggtc tggagccggg ggaaaaggta gaggacatgc 24300

caaagccccc acttccccag gagcaggcca cagacccccct tgtggacagc ctgggcagtg 24360

gcattgtcta ctcagccctt acctgccacc tgtgcggcca cctgaaacag tgtcatggcc 24420

aggaggatgg tggccagacc cctgtcatgg ccagtccttg ctgtggctgc tgctgtggag 24480

acaggtcctc gccccctaca accccccctga gggccccaga cccctctcca ggtggggttc 24540

cactggaggc cagtctgtgt ccggcctccc tggcaccctc gggcatctca gagaagagta 24600

aatcctcatc atccttccat cctgcccctg gcaatgctca gagctcaagc cagaccccca 24660

aaatcgtgaa ctttgtctcc gtgggaccca catacatgag ggtctcttag gtgcatgtcc 24720

tcttgttgct gagtctgcag atgaggacta gggcttatcc atgcctggga aatgccacct 24780

cctggaaggc agccaggctg gcagatttcc aaaagacttg aagaaccatg gtatgaaggt 24840

gattggcccc actgacgttg gcctaacact gggctgcaga gactggaccc cgcccagcat 24900

tgggctgggc tcgccacatc ccatgagagt agagggcact gggtcgccgt gccccacggc 24960

aggcccctgc aggaaaactg aggcccttgg gcacctcgac ttgtgaacga gttgttggct 25020

gctccctcca cagcttctgc agcagactgt ccctgttgta actgcccaag gcatgttttg 25080

cccaccagat catggcccac gtggaggccc acctgcctct gtctcactga actagaagcc 25140

gagcctagaa actaacacag ccatcaaggg aatgacttgg gcggccttgg gaaatcgatg 25200

agaaattgaa cttcagggag ggtggtcatt gcctagaggt gctcattcat ttaacagagc 25260

ttccttaggt tgatgctgga ggcagaatcc cggctgtcaa ggggtgttca gttaagggga 25320

gcaacagagg acatgaaaaa ttgctatgac taaagcaggg acaatttgct gccaaacacc 25380

catgcccagc tgtatggctg gggctcctc gtatgcatgg aaccccccaga ataaatatgc 25440

tcagccaccc tgtgggccgg gcaatccaga cagcaggcat aaggcaccag ttaccctgca 25500

tgttggccca gacctcaggt gctagggaag gcgggaacct tgggttgagt aatgctcgtc 25560

tgtgtgtttt agtttcatca cctgttatct gtgtttgctg aggagagtgg aacagaaggg 25620

gtggagtttt gtataaataa agtttctttg tctctttatt ttttatgtat taaccaaaca 25680

tacctccaga cactgctgtg agtgctgtgt ctctgttaac tcctggaatt cacccatcca 25740

gaggaaccag gatgcaagag gttaagaaac ttgccatctg ggtttgggtt ccccatacaa 25800

ggattcaaat agttgattta ggaagtaatc ccgggaaacc ctgctaaggt agtggggaac 25860

tgaggcaggg aaggacacaa accaagaaag tgttacctga aaggggtcca gatgcagacc 25920

ccaaaagagg gttcttgaat ctcatgcaag aaagaattca gagcgagtcc atagagtcag 25980

tgaaagcaag ttaatgagga aagtaaagga ataaaagaat ggctactccg tagacagagc 26040

agccctgagg gttgctggct gcctattttt atggttattg attaattata ttccaaacaa 26100

ggggtggatt attatgcctc ccttttagac catatagggt aacttcctga tgttgccatg 26160

gcatttgtaa actgtcatgg cgctgttggg agtgtagcag tgaggacaac cagaggtcac 26220

tcttgttgcc atcttggttt tggtgggtta gagccatctt ctttactgca acctgtttta 26280

tcagcaaggt ctttatgact tgtatcggtg acgacctcct gtctcattct atgactaaga 26340

atgccctaac ctcccaggaa tgcagcccag taagtctcag cctcatttta cccagcccct 26400

cttcaaagct ccagtttaaa taaacctctg acaaaagggt gagttattca acagattacc 26460

agcatgagca actgatgctt acctgccggg gatctctgga agaccatgca tggcacatgc 26520

ccagttatgc ctgcaaagga gagggagctg 26550

<210> 4
<211> 825
<212> PRT
<213> Homo sapiens

<400> 4

Met Gly Trp Leu Cys Ser Gly Leu Leu Phe Pro Val Ser Cys Leu Val
1 5 10 15

Leu Leu Gln Val Ala Ser Ser Gly Asn Met Lys Val Leu Gln Glu Pro
20 25 30

Thr Cys Val Ser Asp Tyr Met Ser Ile Ser Thr Cys Glu Trp Lys Met
35 40 45

Asn Gly Pro Thr Asn Cys Ser Thr Glu Leu Arg Leu Leu Tyr Gln Leu
50 55 60

Val Phe Leu Leu Ser Glu Ala His Thr Cys Val Pro Glu Asn Asn Gly
65 70 75 80

Gly Ala Gly Cys Val Cys His Leu Leu Met Asp Asp Val Val Ser Ala
85 90 95

Asp Asn Tyr Thr Leu Asp Leu Trp Ala Gly Gln Gln Leu Leu Trp Lys
100 105 110

Gly Ser Phe Lys Pro Ser Glu His Val Lys Pro Arg Ala Pro Gly Asn
115 120 125

Leu Thr Val His Thr Asn Val Ser Asp Thr Leu Leu Leu Thr Trp Ser
130 135 140

Asn Pro Tyr Pro Pro Asp Asn Tyr Leu Tyr Asn His Leu Thr Tyr Ala
145 150 155 160

Val Asn Ile Trp Ser Glu Asn Asp Pro Ala Asp Phe Arg Ile Tyr Asn
165 170 175

Val Thr Tyr Leu Glu Pro Ser Leu Arg Ile Ala Ala Ser Thr Leu Lys
180 185 190

Ser Gly Ile Ser Tyr Arg Ala Arg Val Arg Ala Trp Ala Gln Cys Tyr
195 200 205

Asn Thr Thr Trp Ser Glu Trp Ser Pro Ser Thr Lys Trp His Asn Ser
210 215 220

Tyr Arg Glu Pro Phe Glu Gln His Leu Leu Leu Gly Val Ser Val Ser
225 230 235 240

Cys Ile Val Ile Leu Ala Val Cys Leu Leu Cys Tyr Val Ser Ile Thr
245 250 255

Lys Ile Lys Lys Glu Trp Trp Asp Gln Ile Pro Asn Pro Ala Arg Ser
260 265 270

Arg Leu Val Ala Ile Ile Ile Gln Asp Ala Gln Gly Ser Gln Trp Glu
275 280 285

Lys Arg Ser Arg Gly Gln Glu Pro Ala Lys Cys Pro His Trp Lys Asn
290 295 300

Cys Leu Thr Lys Leu Leu Pro Cys Phe Leu Glu His Asn Met Lys Arg
305 310 315 320

Asp Glu Asp Pro His Lys Ala Ala Lys Glu Met Pro Phe Gln Gly Ser
325 330 335

Gly Lys Ser Ala Trp Cys Pro Val Glu Ile Ser Lys Thr Val Leu Trp
340 345 350

Pro Glu Ser Ile Ser Val Val Arg Cys Val Glu Leu Phe Glu Ala Pro
355 360 365

Val Glu Cys Glu Glu Glu Glu Glu Val Glu Glu Glu Lys Gly Ser Phe
370 375 380

Cys Ala Ser Pro Glu Ser Ser Arg Asp Asp Phe Gln Glu Gly Arg Glu
385 390 395 400

Gly Ile Val Ala Arg Leu Thr Glu Ser Leu Phe Leu Asp Leu Leu Gly
405 410 415

Glu Glu Asn Gly Gly Phe Cys Gln Gln Asp Met Gly Glu Ser Cys Leu
420 425 430

Leu Pro Pro Ser Gly Ser Thr Ser Ala His Met Pro Trp Asp Glu Phe
435 440 445

Pro Ser Ala Gly Pro Lys Glu Ala Pro Pro Trp Gly Lys Glu Gln Pro
450 455 460

Leu His Leu Glu Pro Ser Pro Pro Ala Ser Pro Thr Gln Ser Pro Asp
465 470 475 480

Asn Leu Thr Cys Thr Glu Thr Pro Leu Val Ile Ala Gly Asn Pro Ala
485 490 495

Tyr Arg Ser Phe Ser Asn Ser Leu Ser Gln Ser Pro Cys Pro Arg Glu
500 505 510

Leu Gly Pro Asp Pro Leu Leu Ala Arg His Leu Glu Glu Val Glu Pro
515 520 525

Glu Met Pro Cys Val Pro Gln Leu Ser Glu Pro Thr Thr Val Pro Gln
530 535 540

Pro Glu Pro Glu Thr Trp Glu Gln Ile Leu Arg Arg Asn Val Leu Gln
545 550 555 560

His Gly Ala Ala Ala Ala Pro Val Ser Ala Pro Thr Ser Gly Tyr Gln
565 570 575

Glu Phe Val His Ala Val Glu Gln Gly Gly Thr Gln Ala Ser Ala Val
580 585 590

Val Gly Leu Gly Pro Pro Gly Glu Ala Gly Tyr Lys Ala Phe Ser Ser
595 600 605

```
Leu Leu Ala Ser Ser Ala Val Ser Pro Glu Lys Cys Gly Phe Gly Ala
    610                 615                 620

Ser Ser Gly Glu Glu Gly Tyr Lys Pro Phe Gln Asp Leu Ile Pro Gly
625                 630                 635                 640

Cys Pro Gly Asp Pro Ala Pro Val Pro Val Pro Leu Phe Thr Phe Gly
                645                 650                 655

Leu Asp Arg Glu Pro Pro Arg Ser Pro Gln Ser Ser His Leu Pro Ser
            660                 665                 670

Ser Ser Pro Glu His Leu Gly Leu Glu Pro Gly Glu Lys Val Glu Asp
        675                 680                 685

Met Pro Lys Pro Pro Leu Pro Gln Glu Gln Ala Thr Asp Pro Leu Val
    690                 695                 700

Asp Ser Leu Gly Ser Gly Ile Val Tyr Ser Ala Leu Thr Cys His Leu
705                 710                 715                 720

Cys Gly His Leu Lys Gln Cys His Gly Gln Glu Asp Gly Gly Gln Thr
                725                 730                 735

Pro Val Met Ala Ser Pro Cys Cys Gly Cys Cys Cys Gly Asp Arg Ser
            740                 745                 750

Ser Pro Pro Thr Thr Pro Leu Arg Ala Pro Asp Pro Ser Pro Gly Gly
        755                 760                 765

Val Pro Leu Glu Ala Ser Leu Cys Pro Ala Ser Leu Ala Pro Ser Gly
    770                 775                 780

Ile Ser Glu Lys Ser Lys Ser Ser Ser Ser Phe His Pro Ala Pro Gly
785                 790                 795                 800

Asn Ala Gln Ser Ser Ser Gln Thr Pro Lys Ile Val Asn Phe Val Ser
                805                 810                 815

Val Gly Pro Thr Tyr Met Arg Val Ser
            820                 825
```

<210> 5
<211> 15
<212> DNA
<213> artificial sequence

<220>
<223> primer

<400> 5
acacgtgtat ccctg 15

<210> 6
<211> 14
<212> DNA
<213> artificial sequence

<220>
<223> Primer

<400> 6
cacgtgtgtc cctg 14

<210> 7
<211> 17
<212> DNA
<213> artificial sequence

<220>
<223> Primer

<400> 7
acccagcccc tgtgtct 17

<210> 8
<211> 16
<212> DNA
<213> artificial sequence

<220>
<223> Primer

<400> 8
cgcgcctccg ttgttc 16

<210> 9
<211> 20
<212> DNA
<213> artificial sequence

<220>
<223> Primer

<400> 9
gccgaaatgt cctccagcat 20

<210> 10
<211> 19
<212> DNA
<213> artificial sequence

<220>
<223> Primer

<400> 10
tgctccaccg catgtacaa 19

<210> 11
<211> 17
<212> DNA
<213> artificial sequence

<220>
<223> Primer

<400> 11
cagtggctat caggagt 17

<210> 12
<211> 17
<212> DNA
<213> artificial sequence

<220>
<223> Primer

<400> 12
cagtggctat cgggagt 17

<210> 13
<211> 24
<212> DNA
<213> artificial sequence

<220>
<223> Primer

<400> 13
tccagatcca gcaaatagca cttg 24

<210> 14
<211> 22
<212> DNA
<213> artificial sequence

<220>
<223> Primer

<400> 14
agtctatcag gagcctgtcc aa 22

<210> 15
<211> 18
<212> DNA
<213> artificial sequence

<220>
<223> Primer

<400> 15
ttgcagagga gaagacac 18